# EUROPÄISCHE PATENTSCHRIFT

(11) **EP 2 135 516 B1**
(45) Veröffentlichungstag und Bekanntmachung des Hinweises auf die Patenterteilung: **15.08.2012**
(21) Anmeldenummer: 09162075.7
(22) Anmeldetag: 05.06.2009
(51) Int. Cl.: A23L 1/226, C07C 271/00, C07C 275/00, C07C 279/00, C07C 333/00, C07C 335/00

(54) **Neo-Menthylderivate als Geschmacksstoffe**
Neo-menthyl derivatives as flavourings
Néo-dérivés de menthyle en tant qu'arômes artificiels

(30) Priorität: 13.06.2008 US 61273 P
(43) Veröffentlichungstag der Anmeldung: 23.12.2009
(73) Patentinhaber: Symrise AG, 37603 Holzminden (DE)
(72) Erfinder: Ley, Jakob Peter, 37603, Holzminden (DE); Oertling, Heiko, 37603, Holzminden (DE); Backes, Michael, 37603, Holzminden (DE); Vössing, Tobias, 37688, Beverungen (DE)
(74) Vertreter: Eisenführ, Speiser & Partner

(56) Entgegenhaltungen:
- EP-A1- 1 803 357
- EP-A1- 1 989 944
- EP-A1- 2 064 959
- WO-A1-2004/000023
- DE-A1- 4 226 043
- READ J; ROEBUCK D: "The different reaction velocities of enantiomer with a commonoptically active reagent. part II. Reactions between stereoisomeric menthols and menthyl isocyatates" JOURNAL OF THE CHEMICAL SOCIETY, CHEMICAL SOCIETY, LETCHWORTH., GB, 1. Januar 1952 (1952-01-01), Seiten 812-816, XP009122846 ISSN: 0368-1769
- HOWSON PICKARD AND WILLIAM OSWALD LITTLEBURY R: "Alcohols of the hydroaromatic and terpene series. Part . The mentols corresponding with optically inactive menthone" JOURNAL OF THE CHEMICAL SOCIETY, CHEMICAL SOCIETY, LETCHWORTH., GB, Bd. 101, 1. Januar 1912 (1912-01-01), XP009122803 ISSN: 0368-1769
- BERNARD GASTAMBIDE: "Empêchement stérique et chromatographie" ANNALES DE CHIMIE, MASSON, PARIS, FR, 1. Januar 1954 (1954-01-01), Seiten 257-305, XP009122801 ISSN: 0151-9107
- MA D; CHENG K: "Enantioselective synthesis of functionalized alpha-amino acids via a chiral guanidine catalyzed Michael addition reaction" TETRAHEDRON ASYMMETRY, PERGAMON PRESS LTD, OXFORD, GB, Bd. 10, Nr. 4, 26. Februar 1999 (1999-02-26), Seiten 713-719, XP004222844 ISSN: 0957-4166
- WALLACH O; WERNER D F: "Zur Kenntniss der Terpene und der ätherischen Oele" JUSTUS LIEBIGS ANNALEN DER CHEMIE, VERLAG CHEMIE GMBH, WEINHEIM,; DE, 1. Januar 1898 (1898-01-01), Seiten 278-285, XP009122804 ISSN: 0075-4617

## Beschreibung

Die vorliegende Erfindung betrifft primär die Verwendung von bestimmten auf dem Neo-Menthylgerüst basierenden Verbindungen (insbesondere Derivaten des Harnstoffs, Thioharnstoffs sowie Carbamate, Thiocarbamate und Guanidine) der Formeln (I) und (*ent*-I) (siehe unten) und entsprechenden Mischungen untereinander bzw. mit weiteren Verbindungen als Geschmacksstoffe bzw. Geschmacksstoffmischungen. Die erfindungsgemäß zu verwendenden Verbindungen sind insbesondere zum Erzeugen, Vermitteln, Modifizieren oder Verstärken von würzigen Geschmacksnoten, insbesondere eines Umami-Geschmacks oder der Salzigkeit, geeignet. Die Erfindung betrifft des Weiteren bestimmte Zusammensetzungen, Zubereitungen und Halbfertigwaren, welche eine geschmacklich wirksame Menge der besagten Verbindungen der Formeln (I) und (*ent*-I) umfassen sowie bestimmte Verfahren zum Erzeugen, Vermitteln, Modifizieren und/oder Verstärken bestimmter Geschmackseindrücke, insbesondere Umami und Salzig. Schließlich betrifft die Erfindung auch neue Verbindungen der Formeln (I) und (*ent*-I), welche besondere Geschmackseindrücke vermitteln, und entsprechende Mischungen.

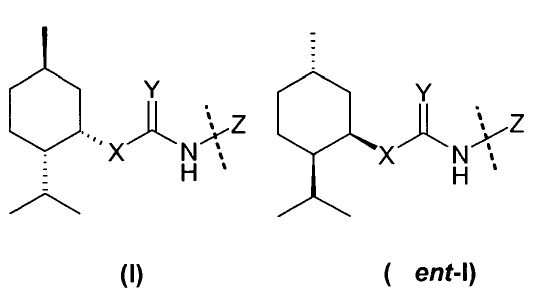

Weitere Aspekte ergeben sich aus der folgenden Beschreibung, den Ausführungsbeispielen, der Figur und den Ansprüchen.

Diverse *D*/*L*-Menthylcarbamate sind seit längerer Zeit als Kühlstoffe bekannt. So wird in DE4226043 die Verwendung folgender Derivate als Kühlwirkstoffe ohne genauere Beschreibung der Stereochemie beschrieben:

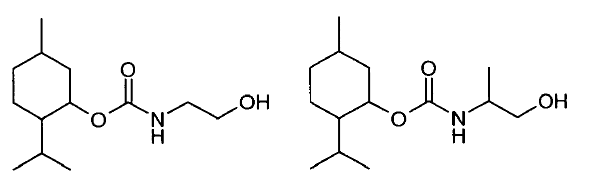

Die Geschmacklosigkeit der Verbindungen und der gemessene Drehwert weisen dabei auf die Verwendung von (*L*)-Menthol als Ausgangsmaterial hin.

In den Dokumenten WO 2006/127738 sowie WO 2006/127740 wird die Verwendung des *tert*-Butylderivats der *D*/*L*-Mentholreihe als Kühlstoff zur Verwendung in Kaugummi beschrieben.

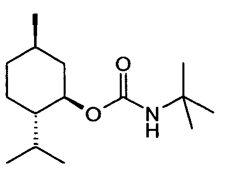

In EP1284145 werden aromatische Kohlensäurederivate der allgemeinen Formel 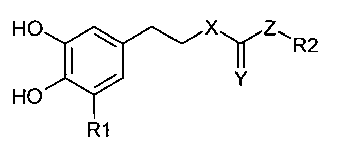 vorgestellt, wobei X,Y und Z unabhängig voneinander O, S und NH bedeuten. Diese Verbindungen können eine Verwendung als Radikalfänger und Antioxidantien finden, wobei R2 auch einen (*L*)-Menthyl-Rest beinhalten kann. Der Geschmack der Verbindungen wurde allerdings nicht weiter untersucht.

In der Veröffentlichung WO 2005/020897 werden verschiedene auf einem Menthylgerüst beruhenden Carbamate und Harnstoffe offenbart, welche als Trp-r8-Modulatoren eingesetzt werden können, sowie Zusammensetzungen, einschließlich pharmazeutischer Zusammensetzungen, welche eine dieser Verbindungen sowie pharmazeutisch einsetzbare Hilfsstoffe, Träger oder Verdünnungsmittel enthalten. Die Struktur einiger in WO 2005/020897 offenbarter Verbindungen entspricht den folgenden Formeln:

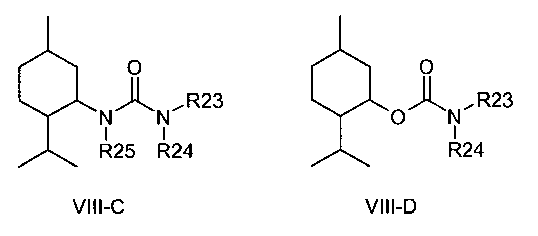

Für die Substituenten R23, R24 und R25 wird insoweit je eine umfangreiche Liste möglicher, voneinander unabhängiger Bedeutungen offenbart. Die gezeigten Beispiele enthalten jeweils ein (-)-Menthyl- bzw. (-)-Menthylamingrundgerüst. Der Geschmack der Verbindungen wird nicht beschrieben.

Des Weiteren ist in EP 1 803 357 beschrieben, dass Carbamate der allgemeinen Formel 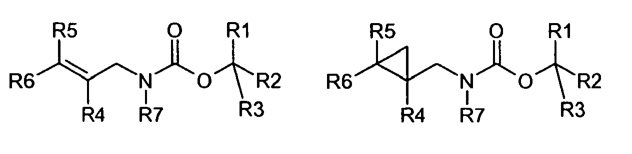 geschmacksmodifizierende Eigenschaften besitzen, die besonders zur Verstärkung eines Umami-Eindrucks sowie zur Verstärkung der Salzigkeit eingesetzt werden können. Es werden auch einige Beispiele vorgestellt, bei denen -CR1R2R3 = Menthyl (ohne genauere Definition der Stereochemie) ist.

In Anspruch 60 der Veröffentlichung US 2005/0084506 werden zahlreiche nichtnatürliche Verbindung der allgemeinen Strukturformel 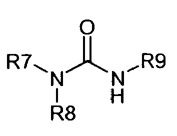 zur Modifizierung des Geschmacks, insbesondere der Eigenschaften süß und würzig, beschrieben. Für die Substituenten R7, R8 und R9 wird insoweit je eine umfangreiche Liste möglicher, voneinander unabhängiger Bedeutungen offenbart. Allerdings wird das Menthylgrundgerüst als möglicher Substituent nicht explizit beschrieben und der Schwerpunkt dieses Teil der Veröffentlichung liegt auf heteroaromatischen Substituenten.

In den US-Provisional Applications sowie 60/916,589 vom 08.05.2007 (Symrise) sowie 60/984,023 vom 31.10.2007 (Symrise) wurde dargelegt, dass die relative Stereochemie des Menthylgerüstes einen wesentlichen Einfluss auf die Geschmackseigenschaften der entsprechenden Verbindungen haben kann. Dabei ist die relative Neo-Stereochemie des Menthylgrundgerüsts wichtig für die Verstärkung eines würzigen und/oder salzigen Geschmackseindrucks dieser Verbindungen, von denen das racemische Neo-Menthylcyclopropancarbonsäureamid (FEMA 4558) bereits GRAS-Status besitzt.

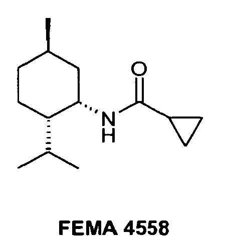

Es besteht ein ständiger Bedarf, neue Geschmacks- und Aromastoffe aufzufinden, d.h. geschmacksaktive Verbindungen oder Verbindungen, die einen würzigen Geschmackseindruck erzeugen, vermitteln, modifizieren oder verstärken können. Insbesondere besteht Bedarf an solchen Verbindungen, die den Geschmackseindruck "Umami" erzeugen, vermitteln, modifizieren oder verstärken können. Im Zuge des verstärkten Gesundheitsbewusstseins der Konsumenten werden zudem Verbindungen gesucht, die Salzigkeit erzeugen, vermitteln, modifizieren oder verstärken können. Für bevorzugte Anwendungen im würzigen Bereich sollten die anzugebenden Verbindungen idealerweise sowohl den Geschmackseindruck "Umami" als auch die Salzigkeit erzeugen, vermitteln, modifizieren oder verstärken.

Aufgabe der vorliegenden Erfindung war es daher, Verfahren und Verbindungen zur Verfügung zu stellen, mittels derer gewünschte Geschmacksnoten (vorzugsweise der Richtungen "Umami" und/oder salzig) erzeugt, vermittelt, modifiziert oder verstärkt werden können.

Diese Aufgabe wird erfindungsgemäß gelöst durch die Verwendung einer Verbindung oder einer Mischung bestehend aus zwei oder mehreren Verbindungen oder enthaltend eine oder mehrere Verbindungen ausgewählt aus der Gruppe bestehend aus Verbindungen der Formeln (I) und (*ent*-I) 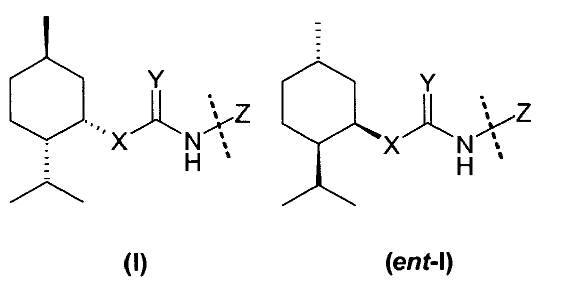 wobei die gestrichelte Linie jeweils die Bindung markiert, welche den organischen Rest Z mit dem in Formel (I) oder (*ent*-I) benachbarten Stickstoffatom verknüpft
und wobei in den Formeln (I) und (*ent*-I) unabhängig voneinander gilt:
X = NH oder O;
Y = O, S oder NR1, wobei R1 Wasserstoff, Methyl, Ethyl, Propyl oder Isopropyl bedeutet; und
Z = organischer Rest mit maximal 15 C-Atomen,
als Geschmacksstoff bzw. Geschmacksstoffmischung.

Bevorzugt ist die Verwendung einer Verbindung oder einer Mischung wie vorstehend definiert, wobei für die Verbindung der Formeln (I) und (*engt*-I) bzw. für eine oder mehrere oder sämtliche Verbindungen der Formeln (I) und (*ent*-I) in der Mischung gilt:
die Summe der Anzahl von Kohlenstoff- und Sauerstoffatomen im organischen Rest Z ist maximal 15 und die Zahl der Sauerstoffatome maximal 4, und
das Atom, mit dem der organische Rest Z an das in Formel (I) oder (*ent*-I) benachbarte Stickstoffatom gebunden ist, ist Kohlenstoff,
mit der Maßgabe dass
   der organische Rest Z keine C-C-Doppel- oder -Dreifachbindung enthält, höchstens einen aromatischen Ring enthält und keine Gruppe -C(=Y²)-R2 ist, in der Y² eine beliebige der oben für Y angegebenen Bedeutungen hat und R2 ein beliebiger organischer Rest ist,
als Geschmacksstoff bzw. Geschmacksstoffmischung.

Vorzugsweise, und das gilt für alle vorangehenden und nachfolgenden Ausgestaltungen, ist Z ein organischer Rest bestehend aus Kohlenstoff- und Wasserstoffatomen sowie gegebenenfalls Sauerstoffatomen.

Nicht bevorzugt ist die Verwendung von Verbindungen des folgenden Typs, in denen Z eine Gruppe -C(=Y²)-R2 ist, wobei R2 einen beliebigen organischen Rest darstellt und für X, Y und Y² das oben Gesagte gilt.

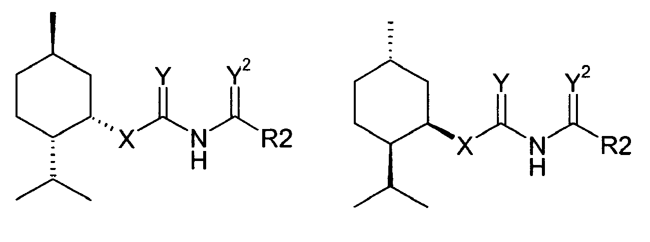

Bevorzugt ist insbesondere die Verwendung einer Verbindung oder einer Mischung wie vorstehend definiert, wobei (i) für die Verbindung der Formel (I) bzw. (*ent*-I) bzw. (ii) für eine oder mehrere oder sämtliche Verbindungen der Formeln (I) und (*ent*-I) in der Mischung gilt:
Y = O oder S und
die Summe der Anzahl von Kohlenstoff- und Sauerstoffatomen im organischen Rest Z ist maximal 11 und die Zahl der Sauerstoffatome maximal 2,
als Geschmacksstoff bzw. Geschmacksstoffmischung.

Besonders bevorzugt ist die Verwendung einer Verbindung oder einer Mischung wie vorstehend definiert, wobei (i) für die Verbindung der Formel (I) bzw. (*ent*-I) bzw. (ii) für eine oder mehrere oder sämtliche Verbindungen der Formeln (I) und (*ent*-I) in der Mischung gilt:
X = NH oder O.
Y = O.
Z = organischer Rest in dem die Summe der Anzahl von Kohlenstoff- und Sauerstoffatomen maximal 11 ist und die Zahl der Sauerstoffatome maximal 2, und das Atom, mit dem der organische Rest Z an das in Formel (I) oder (*ent*-I) benachbarte Stickstoffatom gebunden ist, ist Kohlenstoff,
mit der Maßgabe dass
der organische Rest Z keine C-C-Doppel- oder -Dreifachbindung enthält, höchstens einen aromatischen Ring enthält und keine Gruppe -C(=Y²)-R2 ist, in der Y² eine beliebige der oben für Y angegebenen Bedeutungen hat und R2 ein beliebiger organischer Rest ist,
als Geschmacksstoff bzw. Geschmacksstoffmischung.

Bevorzugt ist auch die Verwendung einer Verbindung oder einer Mischung wie vorstehend definiert, wobei für die Verbindung der Formeln (I) und (*ent-*I) bzw. für eine oder mehrere oder sämtliche Verbindungen der Formeln (I) und (*ent*-I) in der Mischung gilt:
Y = O; und
Z = organischer Rest, in dem die Summe der Anzahl von Kohlenstoff- und Sauerstoffatomen maximal 11 ist und die Zahl der Sauerstoffatome maximal 2, wobei Z ein Rest ausgewählt aus der Gruppe bestehend aus gegebenenfalls ein- oder mehrfach substituiertem Alkyl, C₃-C₇-Cycloalkyl, Phenyl und Phenylalkyl ist, wobei ein oder mehrere gegebenenfalls im Rest Z vorhandene Substituenten vorzugsweise ausgewählt sind aus der Gruppe bestehend aus OH, C₁-C₄-Alkoxy, C₁-C₄-Alkoxycarbonyl und cyclische Ether, wobei cyclische Gruppen im organischen Rest Z gegebenenfalls durch C₁-C₄-Alkylgruppen substituiert sind.

Insbesondere bevorzugt ist die Verwendung einer Verbindung oder einer Mischung wie vorstehend definiert, wobei (i) für die Verbindung der Formeln (I) bzw. (*ent*-I) bzw. (ii) für eine oder mehrere oder sämtliche Verbindungen der Formeln (I) und (*ent*-I) in der Mischung der organische Rest Z ausgewählt ist aus der Gruppe bestehend aus: 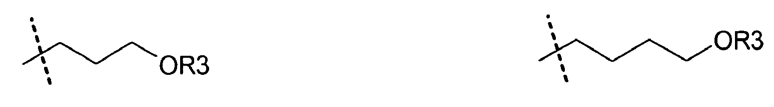 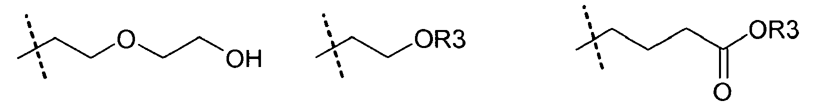 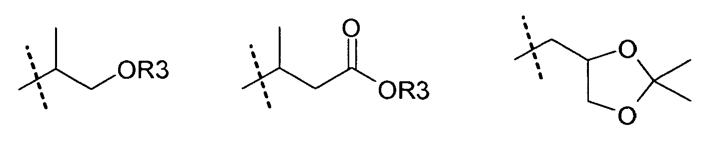 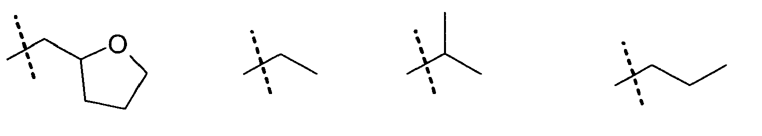 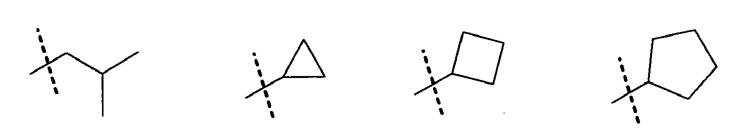 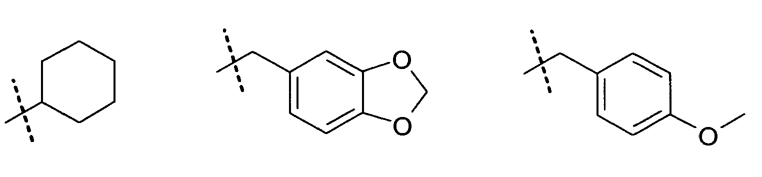 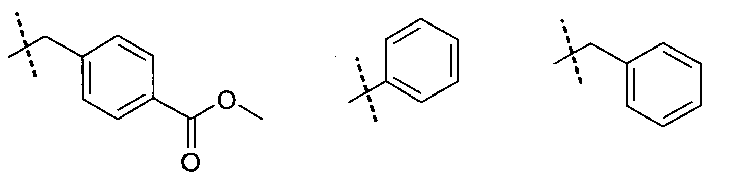 wobei gegebenenfalls vorhandene Reste R3 unabhängig voneinander Wasserstoff, Methyl, Ethyl, *n*-Propyl, *iso*-Propyl, *n*-Butyl, *iso*-Butyl bedeuten und die gestrichelte Linie die Bindung markiert, welche den organischen Rest Z mit dem in Formel (I) oder (*ent*-I) benachbarten Stickstoffatom verknüpft, als Geschmacksstoff bzw. Geschmacksstoffmischung.

Besonders bevorzugt ist auch die Verwendung einer Verbindung oder einer Mischung wie vorstehend definiert, wobei für die Verbindung der Formeln (I) und (*ent*-I) bzw. für eine oder mehrere oder sämtliche Verbindungen der Formeln (I) und (*ent*-I) in der Mischung gilt:
Y = O; und
Z = organischer Rest, in dem die Summe der Anzahl von Kohlenstoff- und Sauerstoffatomen maximal 11 ist und die Zahl der Sauerstoffatome maximal 2, wobei Z ein gegebenenfalls ein- oder mehrfach substituierter geradkettiger oder verzweigter C₁-C₆-Alkylrest ist, wobei ein oder mehrere gegebenenfalls vorhandene Substituenten vorzugsweise ausgewählt sind aus der Gruppe bestehend aus OH, C₁-C₄-Alkoxy und C₁-C₄-Alkoxycarbonyl.

Ganz besonders bevorzugt ist die Verwendung einer Verbindung oder einer Mischung wie vorstehend definiert als Geschmacksstoff bzw. Geschmacksstoffmischung, wobei (i) die Verbindung der Formel (I) bzw. (*ent*-I) bzw. (ii) eine, zwei, mehr als zwei oder sämtliche Verbindungen der Formeln (I) und (*ent*-I) in der Mischung ausgewählt sind aus der Gruppe bestehend aus: 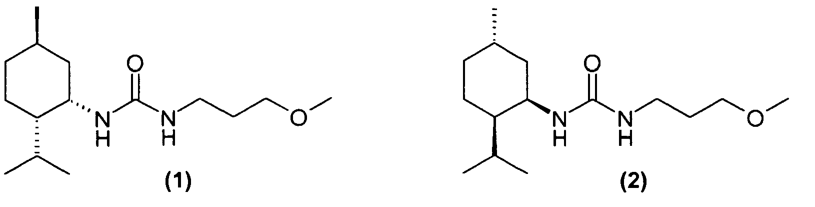 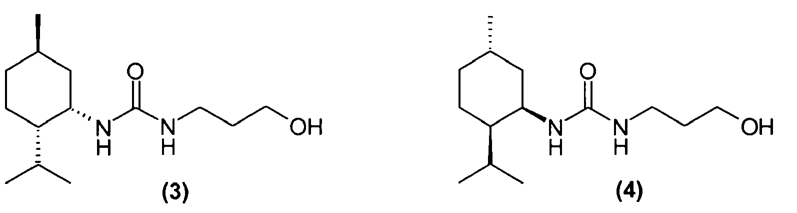 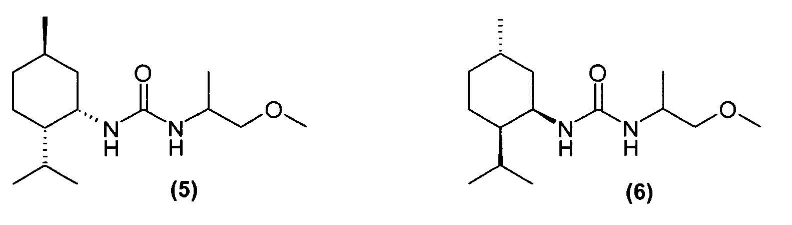 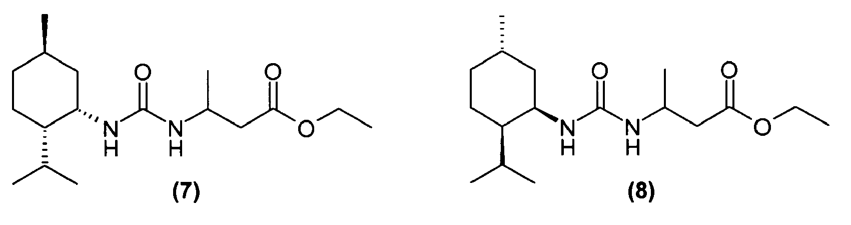 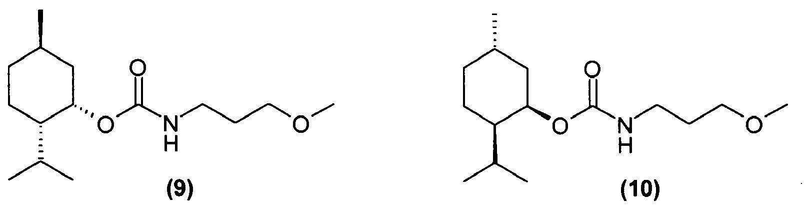 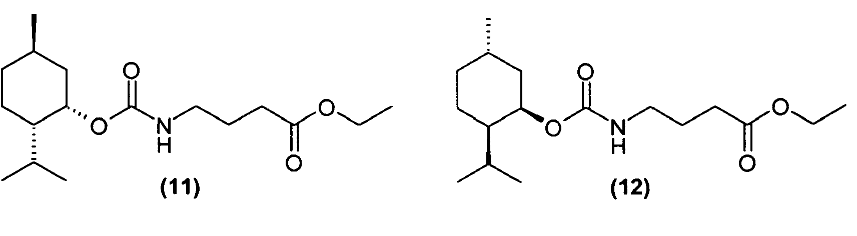
(1) 1-((1S,2S,5R)-2-Isopropyl-5-methyl-cyclohexyl)-3-(3-methoxy-propyl)-harnstoff
(2) 1-((1R,2R,5S)-2-Isopropyl-5-methyl-cyclohexyl)-3-(3-methoxy-propyl)-harnstoff
(3) 1-(3-Hydroxy-propyl)-3-((1S,2S,5R)-2-isopropyl-5-methyl-cyclohexyl)-harnstoff
(4) 1-(3-Hydroxy-propyl)-3-((1R,2R,5S)-2-isopropyl-5-methyl-cyclohexyl)-harnstoff
(5) 1-((1S,2S,5R)-2-Isopropyl-5-methyl-cyclohexyl)-3-(2-methoxy-1-methyl-ethyl)-harnstoff
(6) 1-((1R,2R,5S)-2-Isopropyl-5-methyl-cyclohexyl)-3-(2-methoxy-1-methyl-ethyl)-harnstoff
(7) 3-[3-((1S,2S,5R)-2-Isopropyl-5-methyl-cyclohexyl)-ureido]-buttersäureethylester
(8) 3-[3-((1R,2R,5S)-2-Isopropyl-5-methyl-cyclohexyl)-ureido]-buttersäureethylester
(9) (3-Methoxy-propyl)-carbaminsäure (1S,2S,5R)-2-isopropyl-5-methylcyclohexyl ester
(10) (3-Methoxy-propyl)-carbaminsäure (1R,2R,5S)-2-isopropyl-5-methylcyclohexyl ester
(11) 4-((1S,2S,5R)-2-Isopropyl-5-methyl-cyclohexyloxycarbonylamino)-buttersäureethylester
(12) 4-((1R,2R,5S)-2-Isopropyl-5-methyl-cyclohexyloxycarbonylamino)-buttersäureethylester

Bevorzugt enthalten die erfindungsgemäß zu verwendenden Mischungen zwei oder mehrere der o.g. besonders bevorzugten oder ganz besonders bevorzugten Verbindungen der Formeln (I) und (*ent*-I) bzw. bestehen aus zwei oder mehreren der o.g. besonders bevorzugten oder ganz besonders bevorzugten Verbindungen der Formeln (I) und (*ent*-I)*.*

Ganz besonders bevorzugt ist die Verwendung einer Mischung wie vorstehend definiert, wobei die Mischung ein oder mehrere Paare von Verbindungen der Formeln (I) und (*ent-*I) enthält oder aus ein oder mehreren solcher Paare besteht, wobei jedes Paar aus einer Verbindung der Formel (I) und einer Verbindung der Formel (*ent*-I) wie vorstehend definiert (und vorzugsweise als bevorzugt bezeichnet) besteht,
wobei
die Bedeutung von X und Y in der Verbindung der Formel (I) jeweils identisch ist mit der Bedeutung von X und Y in der Verbindung der Formel (*ent*-I) und
wobei
(a) die Bedeutung von Z in der Verbindung der Formel (I) identisch ist mit der Bedeutung von Z in der Verbindung der Formel (*ent*-I) oder
(b) Z in der Verbindung der Formel (I) sowie Z in der Verbindung der Formel (ent-I) ein oder mehrere chirale Zentren enthalten und sich ausschließlich durch die absolute Konfiguration an einem, mehreren oder sämtlichen dieser chirale Zentren unterscheiden,
wobei vorzugsweise jedes Paar ein Enantiomeren- oder Epimerenpaar ist.

In eigenen Untersuchungen hat sich gezeigt, dass die erfindungsgemäß zu verwendenden Verbindungen der Formeln (I) und (*ent*-I) bzw. die oben und nachfolgend beschriebenen Mischungen in stark Natriumglutamat-reduzierten, in Natriumglutamatfreien Lebensmitteln sowie in Lebensmitteln mit reduziertem Natriumchloridgehalt, so zum Beispiel in würzigen Lebensmitteln wie Tomatensuppe, Hühnersuppe, Knabbergebäck, Fertigpizza, Kartoffelchips und Popcorn einen Umami-Geschmack sowohl im Anfangsgeschmack (Impact) als auch in der längeranhaltenden Geschmackswahrnehmung besonders gut erzeugen vermitteln, modifizieren und/oder verstärken können. Die zusätzliche Fähigkeit - ähnlich dem Mononatriumglutamat - auch eine Wahrnehmung der Salzigkeit zu erzeugen und/oder zu verstärken, führt zu einem als angenehm empfunden Geschmackserlebnis, welches in vielen Fällen sogar als bevorzugt gegenüber Natriumglutamat bewertet wird. Einige Verbindungen der Formeln (I) und (*ent*-I) bzw. die oben beschriebenen erfindungsgemäß zu verwendenden Mischungen erzeugen, vermitteln, modifizieren und/oder verstärken den Geschmackseindruck "salzig" ohne einen starken Umami-Charakter aufzuweisen. Diese sind besonders geeignet für die Verwendung in Natriumchlorid-haltigen Lebensmitteln wie z.B. Salzlakritz, Salzstangen und Cracker, zur Erzeugung, Vermittelung, Modifizierung und/oder Verstärkung des reinen Salzgeschmacks bei gleichzeitiger Reduktion des Gehaltes an Natriumchlorid.

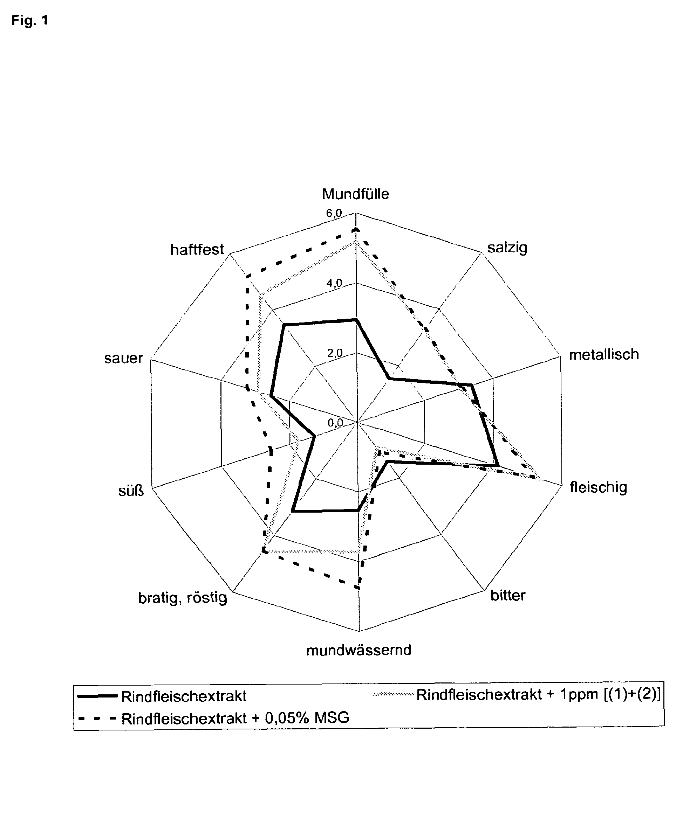

Im Vergleich zu anderen nach Umami schmeckenden Verbindungen zeichnet sich beispielsweise eine Mischung aus 1-((1S,2S,5R)-2-Isopropyl-5-methyl-cyclohexyl)-3-(3-methoxy-propyl)-harnstoff (**1**) und 1-((1R,2R,5S)-2-Isopropyl-5-methyl-cyclohexyl)-3-(3-methoxy-propyl)-harnstoff (**2**) durch einen klaren, dem Natriumglutamat (MSG) sehr nahe kommenden Umami-Geschmack aus, welcher auch die Salzigkeit der Base deutlich verstärkt, ohne dabei unangenehm süß zu schmecken. Dies zeigt auch das als Figur 1 beigefügte Spiderdiagramm, in welchem ein amerikanischer Rindfleischextrakt als Base mit (i) einer solchen Base mit einem Zusatz von 1 ppm einer 1:1 Mischung aus 1-((1S,2S,5R)-2-Isopropyl-5-methyl-cyclohexyl)-3-(3-methoxy-propyl)-harnstoff (**1**) und 1-((1R,2R,5S)-2-Isopropyl-5-methyl-cyclohexyl)-3-(3-methoxy-propyl)-harnstoff (**2**) und (ii) mit einer solchen Base mit einem Zusatz von 0,05 Gew.-% MSG (Natriumglutamat) verglichen wird. Die vorstehenden Beobachtungen gelten entsprechend für alle erfindungsgemäß zu verwendenden Mischungen.

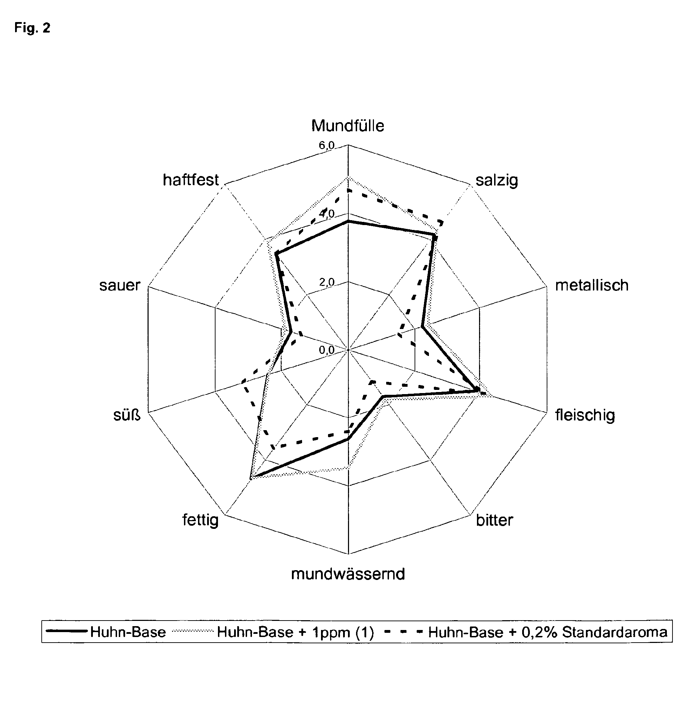

Der unverfälschte MSG-artige Charakter wird weiterhin durch das als Figur 2 beigefügte Spiderdiagramm belegt. Hier wird eine Base für ein Huhnaroma mit (i) einer solchen Base mit einem Zusatz von 1 ppm der enantiomerenreinen Verbindung 1-((1S,2S,5R)-2-Isopropyl-5-methyl-cyclohexyl)-3-(3-methoxy-propyl)-harnstoff (**1**) und (ii) mit einer solchen Base mit einem Zusatz von 0,2% eines Standardaromas (MSG-haltig) verglichen.

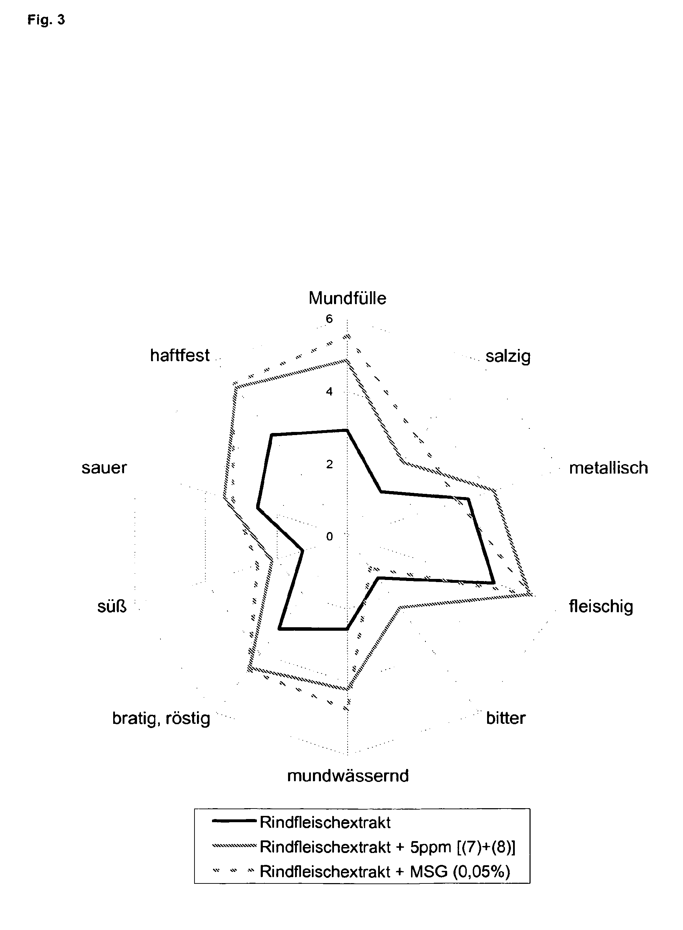

In dem als Figur 3 beigefügten Spiderdiagramm wird amerikanischer Rindfleischextrakt als Base mit (i) einer solchen Base mit einem Zusatz von 1 ppm einer 1:1 Mischung aus 3-[3-((1S,2S,5R)-2-Isopropyl-5-methyl-cyclohexyl)-ureido]-buttersäureethylester (**7**) und 3-[3-((1R,2R,5S)-2-Isopropyl-5-methyl-cyclohexyl)-ureido]-buttersäureethylester (**8**) und (ii) mit einer solchen Base mit einem Zusatz von 0,05 Gew.-% MSG (Natriumglutamat) verglichen.

In einem weiteren Test wurde die Salzigkeit einer Brühe mit einem Salzgehalt von 0,3% und dem Zusatz von 5 ppm einer äquimolaren Mischung aus 1-(3-Hydroxy-propyl)-3-((1S,2S,5R)-2-isopropyl-5-methyl-cyclohexyl)-harnstoff (3) und 1-(3-Hydroxy-propyl)-3-((1R,2R,5S)-2-isopropyl-5-methyl-cyclohexyl)-harnstoff (**4**) verkostet. Die Testpersonen bewerteten diese Brühe gegen eine Referenzreihe eben dieser Brühe mit einem Salzgehalt von 0,2%, 0,3%, 0,4% bzw. 0,5%. Die Salzigkeit der Brühe mit einem Salzgehalt von 0,3% und dem Zusatz von 5 ppm einer äquimolaren Mischung aus 1-(3-Hydroxy-propyl)-3-((1S,2S,5R)-2-isopropyl-5-methyl-cyclohexyl)-harnstoff (**3**) und 1-(3-Hydroxy-propyl)-3-((1R,2R,5S)-2-isopropyl-5-methyl-cyclohexyl)-harnstoff (**4**) wurde im Mittelwert mit einer Salzigkeit von 0,36% bewertet.

In einem weiteren Test wurde die Salzigkeit einer Brühe mit einem Salzgehalt von 0,3% und dem Zusatz von 3 ppm einer äquimolaren Mischung aus (3-Methoxy-propyl)-carbaminsäure (1S,2S,5R)-2-isopropyl-5-methyl-cyclohexyl ester (**9**) und (3-Methoxypropyl)-carbaminsäure (1R,2R,5S)-2-isopropyl-5-methyl-cyclohexyl ester (**10**) verkostet. Die Testpersonen bewerteten diese Brühe gegen eine Referenzreihe eben dieser Brühe mit einem Salzgehalt von 0,2%, 0,3%, 0,4% bzw. 0,5%. Die Salzigkeit der Brühe mit einem Salzgehalt von 0,3% und dem Zusatz von 3 ppm einer äquimolaren Mischung aus (3-Methoxy-propyl)-carbaminsäure (1S,2S,5R)-2-isopropyl-5-methyl-cyclohexyl ester (**9**) und (3-Methoxy-propyl)-carbaminsäure (1R,2R,5S)-2-isopropyl-5-methyl-cyclohexyl ester (**10**) wurde im Mittelwert mit einer Salzigkeit von 0,35% bewertet.

Ein weiterer Aspekt der Erfindung betrifft die Verwendung einer Mischung, welche aus folgenden Komponenten besteht oder diese enthält:
(a) eine Verbindung ausgewählt aus der Gruppe bestehend aus Verbindungen der Formeln (I) und (*ent*-I) wie oben definiert oder eine Mischung bestehend aus zwei oder mehreren Verbindungen oder enthaltend eine oder mehrere Verbindungen ausgewählt aus der Gruppe bestehend aus Verbindungen der Formeln (I) und (*ent*-I) wie oben definiert (vorzugsweise gemäß einer der vorstehend als bevorzugt bezeichneten Ausgestaltungen)
   sowie
(b) eine Verbindung ausgewählt aus der Gruppe bestehend aus Verbindungen der Formeln (II), (*ent*-II), (III), (ent-III), (IV), (*ent*-IV) oder eine Mischung bestehend aus zwei oder mehreren Verbindungen oder enthaltend eine oder mehrere Verbindungen ausgewählt aus der Gruppe bestehend aus Verbindungen der Formeln (II), (*ent*-II), (III), (*ent*-III), (IV), (*ent*-IV): 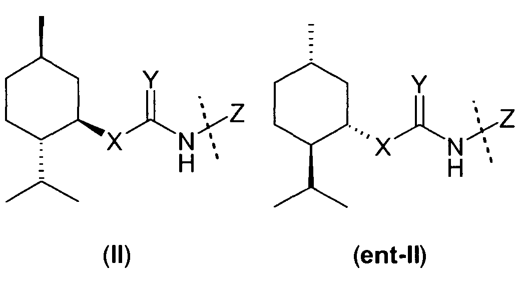 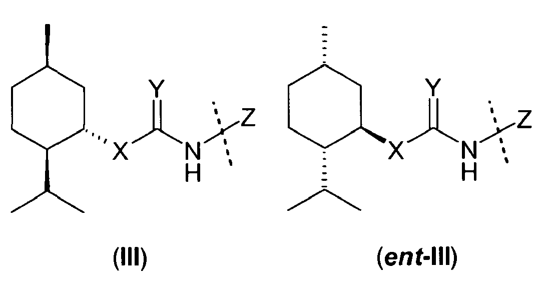 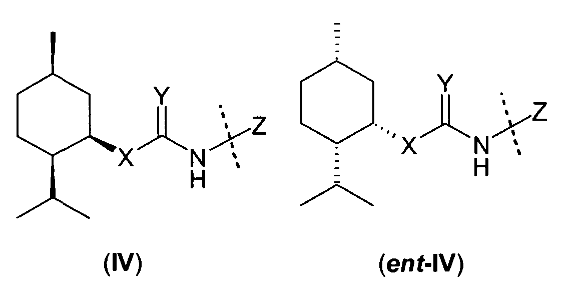
wobei in den Formeln (II), (*ent*-II), (III), (*ent*-III), (IV), (*ent*-IV) X, Y und Z jeweils unabhängig voneinander eine der oben für die Formeln (I) und (*ent*-I) angegebenen Bedeutungen besitzt, als Geschmacksstoffmischung.

In der vorstehend definierten Geschmacksstoffmischung beträgt das Gewichtsverhältnis von (a) der Gesamtheit von Verbindungen der Formeln (I) und (*ent*-I) zu (b) der Gesamtheit von Verbindungen der Formeln (II), (*ent*-II), (III), (*ent*-III), (IV) und (*ent*-IV) vorzugsweise (jedoch nicht notwendigerweise) mindestens 60:40, bevorzugt mindestens 90:10, insbesondere bevorzugt mindestens 95:5.

Dementsprechend betrifft die Erfindung auch die besonders bevorzugte Verwendung einer Verbindung der Formeln (I) und (*ent*-I) wie oben definiert oder einer Mischung bestehend aus zwei oder mehreren Verbindungen oder enthaltend eine oder mehrere Verbindungen der Formeln (I) und (*ent*-I) bzw. einer Mischung wie oben definiert umfassend (a) eine Verbindung der Formeln (I) und (*ent*-I) oder eine Mischung enthaltend oder bestehend aus Verbindungen der Formeln (I) und (*ent*-I) sowie (b) eine Verbindung der Formeln (II), (*ent*-II), (III), (*ent*-III), (IV), (*ent*-IV) wie oben definiert oder eine Mischung enthaltend oder bestehend aus Verbindungen der Formeln (II), (*ent*-II), (III), (*ent*-III), (IV), (*ent*-IV) zum Erzeugen, Vermitteln, Modifizieren oder Verstärken eines Umami-Geschmacks und/oder der Salzigkeit. Dabei gilt hinsichtlich bevorzugter Verbindungen und Mischungen das oben Gesagte entsprechend.

Gemäß einem weiteren Aspekt betrifft die vorliegende Erfindung ein Verfahren zum Erzeugen, Vermitteln, Modifizieren oder Verstärken eines Geschmackseindrucks, insbesondere eines Umami-Geschmacks und/oder der Salzigkeit. Entsprechend dem erfindungsgemäßen Verfahren wird einer Substanz oder Zusammensetzung eine Verbindung der Formeln (I) und (*ent*-I) wie oben definiert oder eine Mischung bestehend aus zwei oder mehreren Verbindungen oder enthaltend eine oder mehrere Verbindungen der Formeln (I) und (*ent*-I) bzw. eine Mischung wie oben definiert umfassend (a) eine Verbindung der Formeln (I) und (*ent*-I) oder eine Mischung enthaltend oder bestehend aus Verbindungen der Formeln (I) und (*ent*-I) sowie (b) eine Verbindung der Formeln (II), (*ent*-II), (III), (*ent*-III), (IV), (*ent*-IV) wie oben definiert oder eine Mischung enthaltend oder bestehend aus Verbindungen der Formeln (II), (*ent*-II), (III), (*ent*-III), (IV), (*ent*-IV) in einer geschmacklich wirksamen Menge zugesetzt. Dabei gilt hinsichtlich bevorzugter Verbindungen und Mischungen das oben Gesagte entsprechend.

Ein weiterer Aspekt der Erfindung betrifft neue Verbindungen der Formeln (I) und (*ent*-I)*,* und entsprechende Mischungen. Dementsprechend betrifft die Erfindung einzelne Verbindungen oder Mischungen bestehend aus zwei oder mehreren Verbindungen oder enthaltend eine oder mehrere Verbindungen ausgewählt aus der Gruppe bestehend aus Verbindungen der Formeln (I) und (*ent*-I) 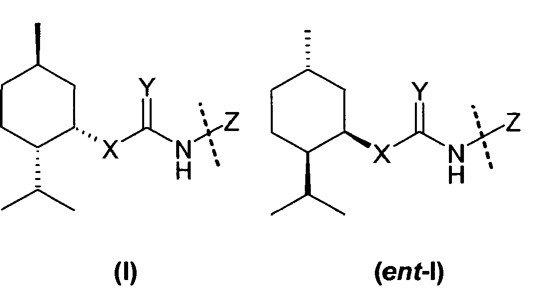 wobei die gestrichelte Linie jeweils die Bindung markiert, welche den organischen Rest Z mit dem in Formel (I) oder (*ent*-I) benachbarten Stickstoffatom verknüpft
und wobei in den Formeln (I) und (*ent*-I) unabhängig voneinander gilt:
X = NH oder O;
Y = O, S oder NR1, wobei R1 Wasserstoff, Methyl, Ethyl, Propyl oder Isopropyl bedeutet; und
Z = organischer Rest bestehend aus Kohlenstoff- und Wasserstoffatomen sowie gegebenenfalls Sauerstoffatomen, wobei
die Summe der Anzahl von Kohlenstoff- und Sauerstoffatomen maximal 15 ist und die Zahl der Sauerstoffatome maximal 4, und
das Atom, mit dem der organische Rest Z an das in Formel (I) oder (*ent*-I) benachbarte Stickstoffatom gebunden ist, Kohlenstoff ist,
mit der Maßgabe dass
der organische Rest Z keine C-C-Doppel- oder -Dreifachbindung enthält, höchstens einen aromatischen Ring enthält und keine Gruppe -C(=Y2)-R2 ist, in der Y2 eine beliebige der oben für Y angegebenen Bedeutungen hat und R2 ein beliebiger organischer Rest ist,
Z nicht -C(R5)(R6)-C(=O)OR4 ist, wobei R4 H, Methyl, Ethyl, Propyl, iso-Propyl, Butyl, iso-Butyl oder tert.-Butyl bedeutet und R5 und R6 unabhängig voneinander H oder einen organischen Rest bedeuten,
Z nicht 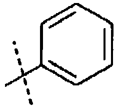 ist, wobei die gestrichelte Linie die Bindung markiert, welche den organischen Rest Z mit dem in Formel (I) oder (ent-I) benachbarten Stickstoffatom verknüpft,
wenn X = NH und Y = O ist, Z nicht Neomenthyl oder 2-Methylcyclohexyl ist und
wenn X = NH und Y = S ist, Z nicht Neomenthyl ist und
wenn X = O und Y = O ist, Z nicht Ethyl, Hydroxyethyl, 1-Phenylethyl, 2-Phenylethyl, 2-Hydroxy-1-methyl-2-phenylethyl, Isopropyl, 2-Hydroxy-1-methylethyl, Cyclohexyl, 4-Hydroxyphenyl, 2-Hydroxy-5-methylphenyl oder 2,4,7-Trioxa-3,3,8-trimethylbicyclo[3.3.0]octan-6-ylmethyl bedeutet,
und
wobei die Verbindungen der Formel (I) die folgende Verbindung nicht umfassen: 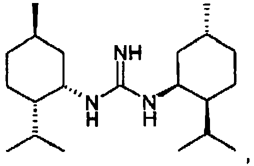 und wobei weiterhin ausgenommen sind
die einzelnen Verbindungen der Formeln (I) und (*ent*-I) in denen X = O, Y =O und Z = (-)-Menthyl bedeuten,
eine Mischung bestehend aus 50,2% der Verbindung der Formel (I), in der X = O, Y =O und Z = (-)-Menthyl bedeuten und 49,8% der Verbindung der Formel (*ent*-I) in der X = O, Y =O und Z = (-)-Menthyl bedeuten und
eine Mischung bestehend aus 30,7% der Verbindung der Formeln (I) in denen X = O, Y =O und Z = (-)-Menthyl bedeuten und 69,3% N-(-)-Menthylcarbaminsäure (-)-Menthylester.

Besonders bevorzugt sind Verbindungen oder Mischungen wie vorstehend definiert, wobei (i) für die Verbindung der Formel (I) bzw. (*ent*-I) bzw. (ii) für eine oder mehrere oder sämtliche Verbindungen der Formeln (I) und (*ent*-I) in der Mischung gilt:
X = NH oder O;
Y=O;
Z = organischer Rest, in dem die Summe der Anzahl von Kohlenstoff- und Sauerstoffatomen maximal 11 ist und die Zahl der Sauerstoffatome maximal 2, und das Atom, mit dem der organische Rest Z an das in Formel (I) oder (*ent*-I) benachbarte Stickstoffatom gebunden ist, ist Kohlenstoff,
mit der Maßgabe, dass
der organische Rest Z keine C-C-Doppel- oder -Dreifachbindung enthält, höchstens einen aromatischen Ring enthält und keine Gruppe -C(=Y²)-R2 ist, in der Y² eine beliebige der oben für Y angegebenen Bedeutungen hat und R2 ein beliebiger organischer Rest ist.

Bevorzugt sind auch einzelne Verbindungen oder Mischungen wie vorstehend definiert, wobei (i) für die Verbindungen der Formel (I) bzw. (*ent*-I) bzw. (ii) für eine oder mehrere oder sämtliche Verbindungen der Formeln (I) und (*ent*-I) in der Mischung gilt:
Y = O; und
Z = organischer Rest in dem die Summe der Anzahl von Kohlenstoff- und Sauerstoffatomen maximal 11 ist und die Zahl der Sauerstoffatome maximal 2, wobei Z ein Rest ausgewählt aus der Gruppe bestehend aus gegebenenfalls ein- oder mehrfach substituiertem Alkyl, C₃-C₇-Cycloalkyl und Phenylalkyl ist, wobei ein oder mehrere gegebenenfalls im Rest Z vorhandene Substituenten vorzugsweise ausgewählt sind aus der Gruppe bestehend aus OH, C₁-C₄-Alkoxy, C₁-C₄-Alkoxycarbonyl und cyclische Ether, wobei cyclische Gruppen im organischen Rest Z gegebenenfalls durch C₁-C₄-Alkylgruppen substituiert sind.

Besonders bevorzugt sind einzelne Verbindungen oder Mischungen wie vorstehend definiert, wobei (i) für die Verbindung der Formeln (I) bzw. (*ent*-I) bzw. (ii) für eine oder mehrere oder sämtliche Verbindungen der Formeln (I) und (*ent*-I) in der Mischung der organische Rest Z ausgewählt ist aus der Gruppe bestehend aus: 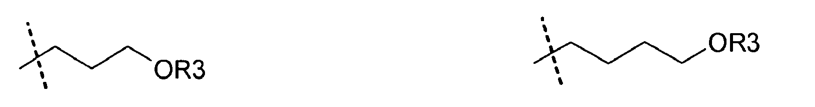 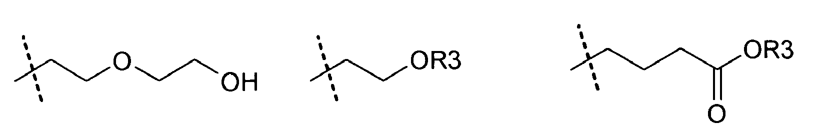 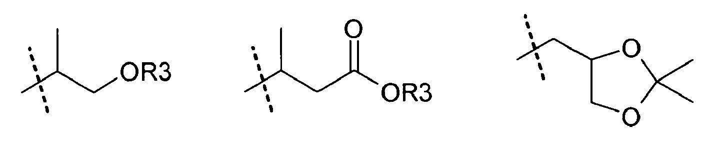 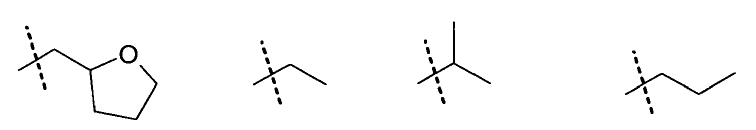 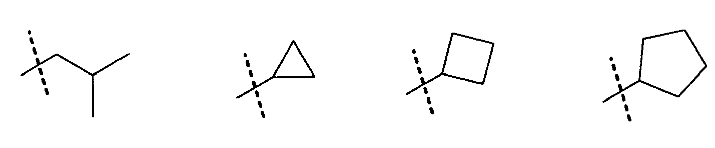 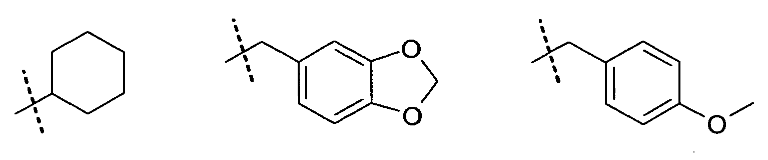 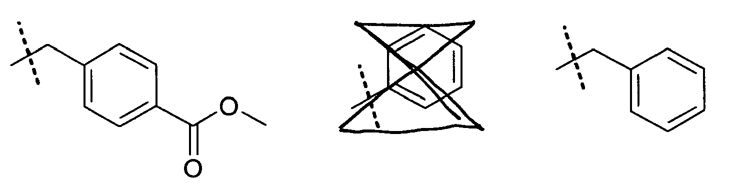 wobei gegebenenfalls vorhandene Reste R3 unabhängig voneinander Wasserstoff, Methyl, Ethyl, *n*-Propyl, *iso*-Propyl, *n*-Butyl oder *iso*-Butyl bedeuten und die gestrichelte Linie die Bindung markiert, welche den organischen Rest Z mit dem in Formel (I) oder (*ent*-I) benachbarten Stickstoffatom verknüpft.

Besonders bevorzugt sind auch einzelne Verbindungen oder Mischungen vorstehend definiert, wobei (i) für die Verbindung der Formel (I) bzw. (*ent*-I) bzw. (ii) für eine oder mehrere oder sämtliche Verbindungen der Formeln (I) und (*ent*-I) in der Mischung gilt:
Y = O; und
Z = organischer Rest, in dem die Summe der Anzahl von Kohlenstoff- und Sauerstoffatomen maximal 11 ist und die Zahl der Sauerstoffatome maximal 2, wobei Z ein gegebenenfalls ein- oder mehrfach substituierter geradkettiger oder verzweigter C₁-C₆-Alkylrest ist, wobei ein oder mehrere gegebenenfalls vorhandene Substituenten vorzugsweise ausgewählt sind aus der Gruppe bestehend aus OH, C₁-C₄-Alkoxy und C₁-C₄-Alkoxycarbonyl.

Besonders bevorzugt sind Verbindungen oder Mischungen wie vorstehend definiert, wobei (i) die Verbindung der Formel (I) bzw. (*ent*-I) bzw. (ii) eine, zwei, mehr als zwei oder sämtliche Verbindungen der Formeln (I) und (*ent*-I) in der Mischung ausgewählt sind aus der Gruppe bestehend aus: 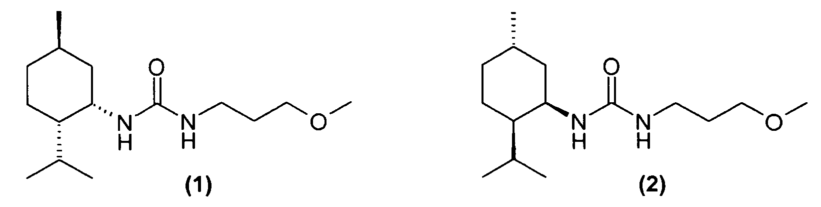 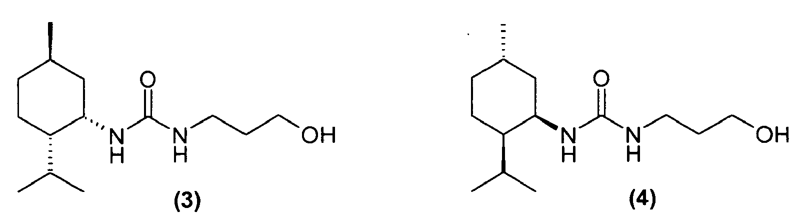 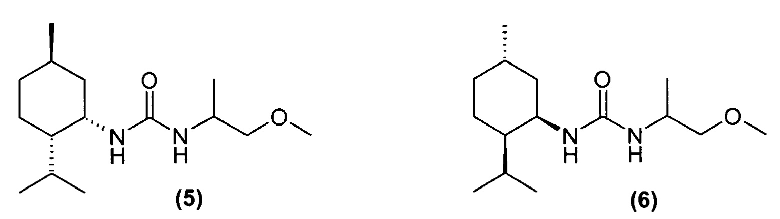 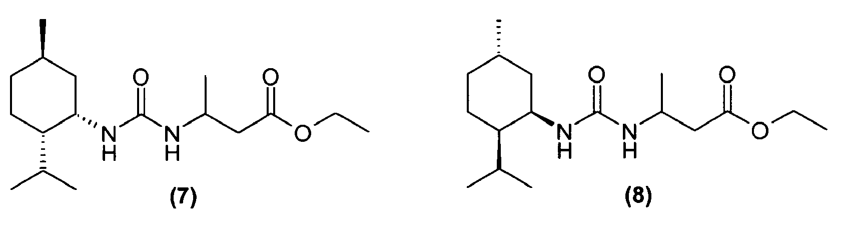 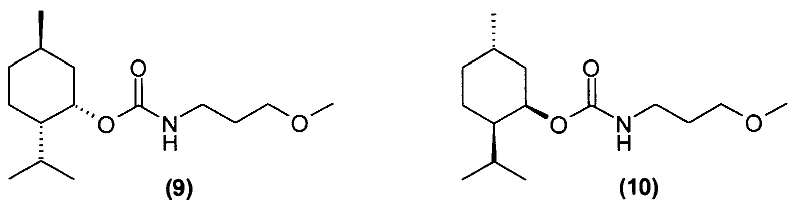 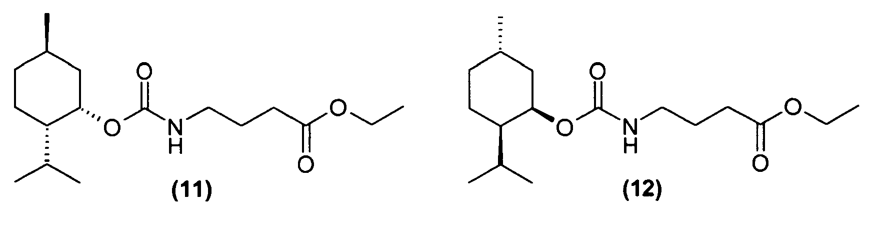
(1) 1-((1S,2S,5R)-2-Isopropyl-5-methyl-cyclohexyl)-3-(3-methoxy-propyl)-harnstoff
(2) 1-((1R,2R,5S)-2-Isopropyl-5-methyl-cyclohexyl)-3-(3-methoxy-propyl)-harnstoff
(3) 1-(3-Hydroxy-propyl)-3-((1S,2S,5R)-2-isopropyl-5-methyl-cyclohexyl)-harnstoff
(4) 1-(3-Hydroxy-propyl)-3-((1R,2R,5S)-2-isopropyl-5-methyl-cyclohexyl)-harnstoff
(5) 1-((1S,2S,5R)-2-Isopropyl-5-methyl-cyclohexyl)-3-(2-methoxy-1-methyl-ethyl)-harnstoff
(6) 1-((1R,2R,5S)-2-Isopropyl-5-methyl-cyclohexyl)-3-(2-methoxy-1-methyl-ethyl)-harnstoff
(7) 3-[3-((1S,2S,5R)-2-Isopropyl-5-methyl-cyclohexyl)-ureido]-buttersäureethylester
(8) 3-[3-((1R,2R,5S)-2-Isopropyl-5-methyl-cyclohexyl)-ureido]-buttersäureethylester
(9) (3-Methoxy-propyl)-carbaminsäure (1S,2S,5R)-2-isopropyl-5-methyl-cyclohexyl ester
(10) (3-Methoxy-propyl)-carbaminsäure (1R,2R,5S)-2-isopropyl-5-methyl-cyclohexyl ester
(11) 4-((1S,2S,5R)-2-Isopropyl-5-methyl-cyclohexyloxycarbonylamino)-buttersäureethylester
(12) 4-((1R,2R,5S)-2-Isopropyl-5-methyl-cyclohexyloxycarbonylamino)-buttersäureethylester.

Ebenfalls bevorzugt sind Mischungen wie vorstehend definiert, wobei die Mischungen ein oder mehrere Paare von Verbindungen der Formeln (I) und (*ent*-I) enthalten oder aus ein oder mehreren solcher Paare bestehen, wobei jedes Paar aus einer Verbindung der Formel (I) und einer Verbindung der Formel (*ent*-I) wie vorstehend definiert besteht,
wobei
die Bedeutung von X und Y in der Verbindung der Formel (I) jeweils identisch ist mit der Bedeutung von X und Y in der Verbindung der Formel (*ent*-I) und
wobei
(a) die Bedeutung von Z in der Verbindung der Formel (I) identisch ist mit der Bedeutung von Z in der Verbindung der Formel (*ent*-I) oder
(b) Z in der Verbindung der Formel (I) sowie Z in der Verbindung der Formel (*ent-*I) ein oder mehrere chirale Zentren enthalten und sich ausschließlich durch die absolute Konfiguration an einem oder sämtlichen dieser chirale Zentren unterscheiden,
wobei vorzugsweise jedes Paar ein Enantiomeren- oder Epimerenpaar ist.

Ebenfalls bevorzugt sind Mischungen, welche aus folgenden Komponenten besteht oder diese enthält:
(a) eine Verbindung ausgewählt aus der Gruppe bestehend aus Verbindungen der Formeln (I) und (*ent*-I) wie vorstehend definiert oder eine Mischung bestehend aus zwei oder mehreren Verbindungen oder enthaltend eine oder mehrere Verbindungen ausgewählt aus der Gruppe bestehend aus Verbindungen der Formeln (I) und (*ent*-I) wie vorstehend definiert oder eine Mischung bestehend aus oder enthaltend eine Mischung wie vorstehend definiert;
   sowie
(b) eine Verbindung ausgewählt aus der Gruppe bestehend aus Verbindungen der Formeln (II), (*ent*-II), (III), (ent-III), (IV), (*ent*-IV) oder eine Mischung bestehend aus zwei oder mehreren Verbindungen oder enthaltend eine oder mehrere Verbindungen ausgewählt aus der Gruppe bestehend aus Verbindungen der Formeln (II), (*ent*-II), (III), (*ent*-III), (IV), (*ent*-IV): 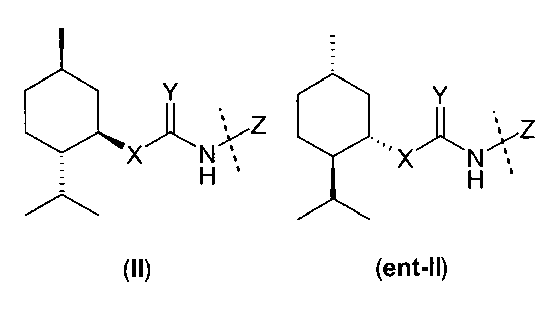 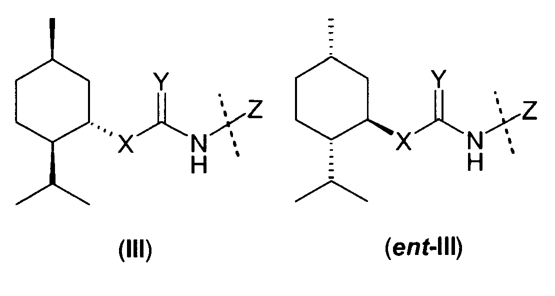 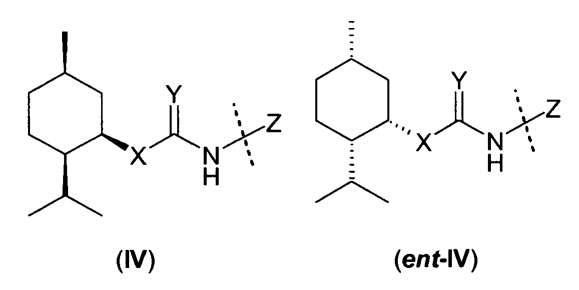
wobei in den Formeln (II), (*ent*-II), (III), (*ent*-III), (IV), (*ent*-IV) X, Y und Z jeweils unabhängig voneinander eine der in einem der vorhergehenden Ansprüche für die Formeln (I) und (*ent*-I) angegebenen Bedeutungen besitzt
und
wobei in der Mischung das Gewichtsverhältnis von (a) der Gesamtheit von Verbindungen der Formeln (I) und (*ent*-I) zu (b) der Gesamtheit von Verbindungen der Formeln (II), *(ent-*II), (III), (*ent*-III), (IV) und (*ent*-IV) mindestens 60:40, bevorzugt mindestens 90:10, insbesondere bevorzugt mindestens 95:5 ist.

Die folgenden bereits literaturbekannten Verbindungen sind kein bevorzugter Gegenstand der vorliegenden Erfindung: Das Dineomenthylharnstoffderivat - welches auch ohne definierte Stereochemie beschrieben wurde (Mikrochimica Acta 1989, 3 (1-2), 117-24) - und das Dineomenthylthioharnstoffderivat (ARKIVOK, 2007, 7, 157-166) sowie das Dineomenthylguanidinderivat (Chin. J. Chem. 2000, 18 (3) 411-413; Tetrahedron:Asymmetry 1999, 10 (4), 713-719)) sind bereits literaturbekannt, allerdings werden keine Aussagen über den Geschmack dieser Verbindungen getroffen:

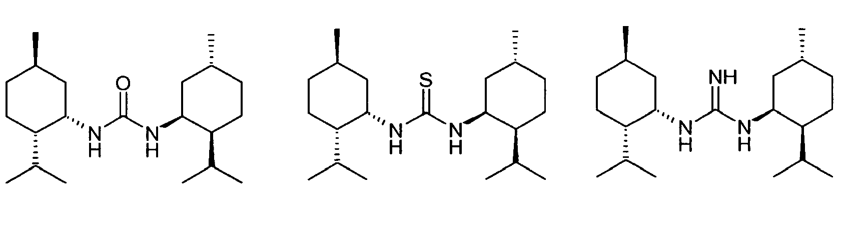

Ebenfalls literaturbekannt sind beide enantiomeren Isopropylcarbamatderivate (Angew. Chem. 1982, 94 (9), 709-10) sowie eine stereochemisch nicht weiter beschriebene Variante (20th Pept., Proc. Eur. Pept. Symp. 1989, 16-18). Auch hier sind die Geschmackseigenschaften nicht beschrieben worden:

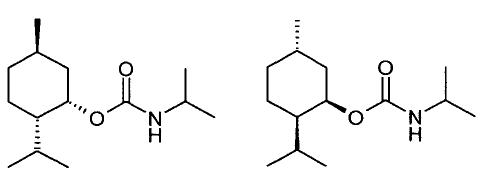

Das Phenyl-substituierte Harnstoffderivat ist bereits seit langem bekannt. Einige physikalische Eigenschaften wurden charakterisiert, über den Geschmack wird allerdings keine Aussage getroffen (J. Chem. Soc. 1951, 2968-2972):

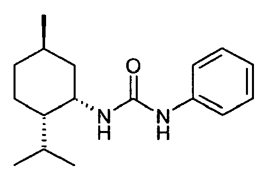

Diese Struktur - sowie ihr Thioanalogon - wird ebenfalls mehrfach in stereochemisch nicht eindeutiger Weise beschrieben (u.a. J. Chem. Soc. 1926, 2209-2223; J. Chem. Soc. 1926, 2223-2234, Chem. Ber. 1958, 91, 311-319). Über den Geschmack wird ebenfalls keine Aussage getroffen:

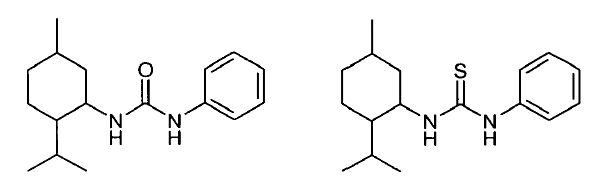

Schließlich ist das unter der CAS-Nummer [585829-94-5] bekannte Carbamat kommerziell erhältlich, welches zusätzlich bereits ohne genaue Angabe der Stereochemie literaturbekannt ist (J. Chem. Soc., Trans 1911, 1337-1340). Allerdings sind auch hier keine Geschmackseigenschaften bekannt:

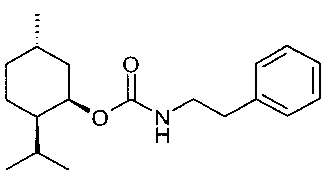

Des Weiteren sind die folgenden Verbindungen für die Zwecke der vorliegenden Erfindung nicht oder nur in Ausnahmefällen bevorzugt:
Verbindungen der Formeln (I) bzw. (ent-I), wobei Z eine α-Carbonyl-Gruppe darstellt.

Insbesondere nicht bevorzugt sind entsprechende Derivate mit α-Carboxyl-Gruppe, wie sie vielfach in der Literatur beschrieben sind.

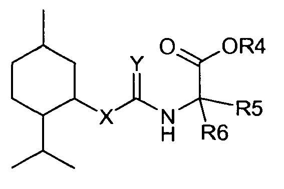

In diesem Fall bedeuten R4 H, Methyl, Ethyl, Propyl, iso-Propyl, Butyl, iso-Butyl oder tert.-Butyl, R5 und R6 unabhängig voneinander H oder ein organischer Rest und für X und Y gilt das für die Formeln (I) und (ent-I) Gesagte. Über den Geschmack solcher Derivate ist allerdings nichts bekannt. Insbesondere ausgenommen sind die bekannten diastereomeren Thioharnstoffderivate (Anal. Chim. Acta 1978, 101 (1), 111-116) mit (+)-*Neo*-Menthylgrundgerüst, die durch Umsetzung von Neo-Menthylisothiocyanat mit der freien Aminofunktion diverser Aminosäuren der D- und L-Reihe (AS = Alanin, Valin, Norvalin, Leucin, Norleucin, Isoleucin, Prolin, Phenylglycin, Phenylalanin, Serin, Threonin, Asparaginsäure und Glutaminsäure) erhalten wurden:

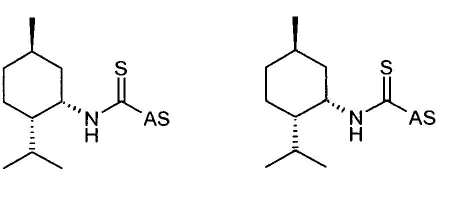

Das Ethyl-substituierte Carbamat wurde bereits beschrieben (Ann. Chim. 1954, 257-309) und findet bei Givaudan als (-)-Mentholderivat in WO 2004/000023 Verwendung als Insektenschutzmittel. Es werden keine Aussagen über die Geschmackseigenschaften getroffen:

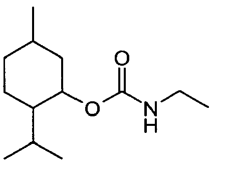

Ein Methylcyclohexyl-substituiertes Harnstoffderivat wurde ebenfalls ohne Untersuchungen zum Geschmack beschrieben (Sitzb. Ges. Beforderung gesamten Naturwissenschaften Marburg 62 (4), 113-35):

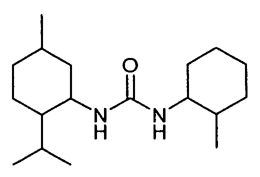

Ein Cyclohexyl-substituiertes Carbamatderivat wird in den Patenten US 6,531,506 und US 5,955,496 als Epoxidhydrolase-Inhibitor offenbart und wurde auch schon früher in Untersuchungen zur Reaktion von Cyclohexylisothiocyanat mit Lebensmittelbestandteilen beschrieben (Deutsche Lebensmittel-Rundschau 1984, 80 (6), 170-174). Eine Aussage zum Geschmack wird allerdings nicht getroffen.

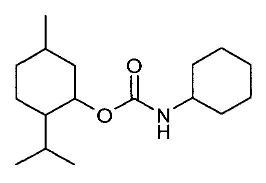

Ein weiteres Aryl-substituiertes Carbamatderivat wurde ebenfalls ohne weitere Untersuchungen zum Geschmack dieser Verbindung beschrieben (Organic Mass Spectrometry 1971, 5 (2), 157-169):

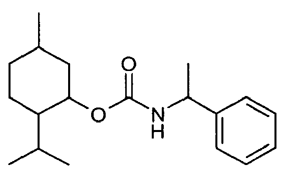

Das nachfolgende Hydroxyaryl-substituierte Carbamat wurde ebenfalls beschrieben (Annales Pharmaceutiques Francaises 1958, 16, 408-413), über den Geschmack dieser Verbindung wurde allerdings nichts berichtet:

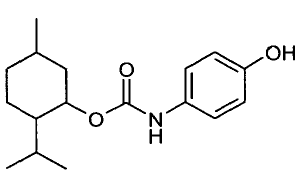

Die Verwendung des folgenden Carbamats in kosmetischen Anwendungen wird in WO 2002/002071 beschrieben:

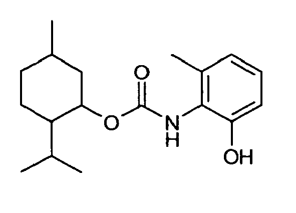

In DE4226043 werden ebenfalls zwei Carbamate mit unspezifischer Stereochemie als Kühlstoffe ohne störenden Eigengeruch oder Eigengeschmack offenbart, wobei die dort festgestellte Geschmacklosigkeit und der Drehwert die Verwendung von (-)-Menthol als Ausgangsmaterial zeigen:

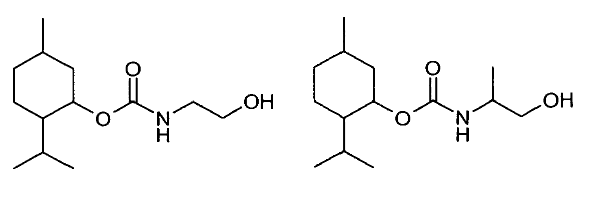

Auch das folgende Derivat basiert auf einer Carbamatstruktur (J. Chem. Soc. , Chem. Commun. 1992, 18, 1308-1310; Tetrahedron Lett. 1991, 32 (34), 4251-4254). Auch hier wurden über den Geschmack keine Untersuchungen angestellt.

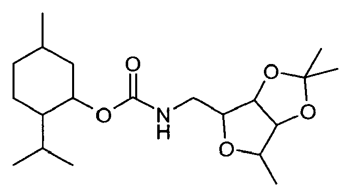

Schließlich ist folgendes Carbamatderivat unter der CAS-Nummer [200123-67-7] ohne Nennung von Daten und Eigenschaften registriert:

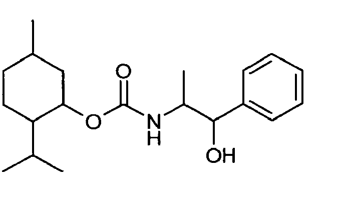

Alle vorstehend genannten literaturbekannten Verbindungen sind für die erfindungsgemäßen Zwecke nicht bevorzugt. Des Weiteren existiert noch eine Vielzahl von Derivaten, die auf einem diastereomeren Menthylgrundgerüst aufbauen, jedoch aufgrund der nicht passenden Stereochemie im vorliegenden Text keine Berücksichtigung finden.

Gemäß einem weiteren Aspekt betrifft die vorliegende Erfindung auch Zusammensetzungen und Zubereitungen, insbesondere zum Verzehr geeignete Zusammensetzungen und Zubereitungen, umfassend oder bestehend aus einer geschmacklich wirksamen Menge einer Verbindung der Formeln (I) und (*ent*-I) wie vorstehend definiert oder einer beliebigen Mischung wie vorstehend definiert oder einer Mischung wie oben definiert umfassend (a) eine oder mehrere Verbindungen der Formeln (I) und (*ent*-I) sowie (b) eine oder mehrere Verbindungen der Formeln (II), (*ent*-II), (III), (*ent*-III), (IV), (*ent*-IV) sowie einem oder mehreren weiteren zum Verzehr geeigneten Bestandteilen. Dabei gilt hinsichtlich bevorzugter Verbindungen und Mischungen das oben Gesagte entsprechend.

Die erfindungsgemäßen zum Verzehr geeigneten der Ernährung, der Mundpflege oder dem Genuss dienenden Zubereitungen oder Zusammensetzungen sind regelmäßig Produkte, die dazu bestimmt sind, in die menschliche Mundhöhle eingebracht zu werden, dort eine bestimmte Zeit zu verbleiben und anschließend entweder verzehrt (z.B. verzehrfertige Lebensmittel, siehe auch weiter unten) oder wieder aus der Mundhöhle entfernt zu werden (z.B. Kaugummis oder Zahnpasta). Zu diesen Produkten gehören dabei sämtliche Stoffe oder Erzeugnisse, die dazu bestimmt sind, in verarbeitetem, teilweise verarbeitetem oder unverarbeitetem Zustand vom Menschen aufgenommen zu werden. Hierzu zählen auch Stoffe, die Lebensmitteln bei ihrer Herstellung, Verarbeitung oder Bearbeitung zugesetzt werden und dazu vorgesehen sind, in die menschliche Mundhöhle eingebracht zu werden.

Im Rahmen des vorliegenden Textes werden unter einem "Lebensmittel" insbesondere Stoffe verstanden, die dazu bestimmt sind, in unverändertem, zubereitetem oder verarbeitetem Zustand vom Menschen geschluckt und dann verdaut zu werden; als Lebensmittel werden insoweit auch Umhüllungen, Überzüge oder sonstige Umschließungen verstanden, die dazu bestimmt sind, mitverschluckt zu werden, oder bei denen ein Verschlucken vorauszusehen ist. Auch bestimmte Produkte, die üblicherweise wieder aus der Mundhöhle entfernt werden (z.B. Kaugummis) sind im Rahmen des vorliegenden Textes als Lebensmittel zu verstehen, da bei ihnen nicht auszuschließen ist, dass sie zumindest teilweise geschluckt werden.

Unter einem verzehrfertigen Lebensmittel ist hierbei ein Lebensmittel zu verstehen, das hinsichtlich der für den Geschmack maßgeblichen Substanzen bereits vollständig zusammengesetzt ist. Unter den Begriff "verzehrfertiges Lebensmittel" fallen auch Getränke sowie feste oder halbfeste verzehrfertige Lebensmittel. Als Beispiele seien genannt Tiefkühlprodukte, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln.

Unter einer Halbfertigware ist im Zusammenhang des vorliegenden Textes ein Produkt zu verstehen, welches aufgrund eines sehr hohen Gehaltes an Aroma- und Geschmacksstoffen für die Verwendung als verzehrfertiges Lebensmittel ungeeignet ist. Erst durch Mischen mit mindestens einem weiteren Bestandteil (d.h. durch Verringern der Konzentration der betreffenden Aroma- und Geschmacksstoffe) und gegebenenfalls weitere Prozessschritte (z.B. Erwärmen, Gefrieren) wird die Halbfertigware in ein verzehrfertiges Lebensmittel überführt. Als Beispiel für Halbfertigwaren seien hier Tütensuppen, Backaromen und Puddingpulver genannt.

Unter einem Mundpflegeprodukt (auch Mundhygieneprodukt oder mundhygienische Zubereitung genannt) im Sinne der Erfindung wird eine der dem Fachmann geläufigen Formulierungen zur Reinigung und Pflege der Mundhöhle und des Rachenraumes sowie zur Erfrischung des Atems verstanden. Hierbei ist die Pflege der Zähne und des Zahnfleisches ausdrücklich eingeschlossen. Darreichungsformen gebräuchlicher mundhygienischer Formulierungen sind insbesondere Cremes, Gele, Pasten, Schäume, Emulsionen, Suspensionen, Areosole, Sprays, wie auch Kapseln, Granulate, Pastillen, Tabletten, Bonbons oder Kaugummis, ohne dass diese Aufzählung für die Zwecke dieser Erfindung limitierend verstanden werden soll.

Bevorzugte Mundpflegeprodukte (Mundhygieneprodukt) sind insbesondere solche in Form von Zahnpasta, Zahncreme, Zahngel, Zahnpulver, Zahnputzflüssigkeit, Zahnputzschaum, Mundwasser, Zahncreme und Mundwasser als 2-in-1 Produkt, Lutschbonbon, Mundspray, Zahnseide, oder Zahnpflegekaugummi.

Kaugummis umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkohole (insbesondere Sorbitol, Xylitol, Mannitol), Kühlwirkstoffe, Geschmackskorrigenzien für unangenehme Geschmackseindrücke, weitere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Stabilisatoren, Geruchskorrigentien und Aromen (z.B.: Eycalyptus-Menthol, Kirsche, Erdbeere, Grapefruit, Vanille, Banane, Citrus, Pfirsich, schwarze Johannisbeere, Tropenfrüchte, Ingwer, Kaffee, Zimt, Kombinationen (der genannten Aromen) mit Mintaromen sowie Spearmint und Pfefferminz alleine). Besonders interessant ist auch die Kombination der Aromen mit weiteren Stoffen, die kühlende, wärmende und/oder mundwässerndernde Eigenschaften aufweisen.

Im Stand der Technik sind zahlreiche unterschiedliche Kaugummibasen bekannt, wobei zwischen sogenannten "chewing gum" - oder "bubble gum" - Basen zu unterscheiden ist, wobei letztere weicher sind, damit sich damit auch Kaugummiblasen bilden lassen.

Gängige Kaugummibasen umfassen neben traditionell eingesetzten natürlichen Harzen oder dem Naturlatex Chicle heute zumeist Elastomere wie Polyvinylacetate (PVA), Polyethylene, (nieder- oder mittelmolekulare) Polyisobutene (PIB), Polybutadiene, Isobuten-Isopren Copolymere (Butyl Rubber), Polyvinylethylether (PVE), Polyvinylbutylether, Copolymere von Vinylestern und Vinylethern, Styrol-Butadien-Copolymere (Styrol-Butadien-Rubber, SBR) oder Vinyl-Elastomere, z.B. auf Basis Vinylacetat/Vinyllaurat, Vinylacetat/Vinylstaerat oder Ethylen/Vinylacetat, sowie Mischungen der genannten Elastomere, wie beispielsweise in EP 0 242 325, US 4,518,615, US 5,093,136, US 5,266,336 US 5,601,858 oder US 6,986,709 beschrieben. Daneben umfassen Kaugummibasen weitere Bestandteile wie beispielsweise (mineralische) Füllstoffe, Weichmacher, Emulgatoren, Antioxidantien, Wachse, Fette oder fette Öle, wie beispielsweise gehärtete (hydrierte) pflanzliche oder tierische Fette, Mono-, Di- oder Triglyceride. Geeignete (mineralische) Füllstoffe sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen. Geeignete Weichmacher bzw. Mittel zur Verhinderung des Verklebens (detackifier) sind beispielsweise Lanolin, Stearinsäure, Natriumstearat, Ethylacetat, Diacetin (Gylcerindiacetat), Triacetin (Gylcerintriacetat), Triethylcitrat. Geeignete Wachse sind beispielsweise Paraffin Wachse, Candelilla Wachs, Carnauba Wachs, mikrokristalline Wachse und Polyethylen Wachse. Geeignete Emulgatoren sind beispielsweise Phosphatide wie Lecithin, Mono- und Diglyceride von Fettsäuren, z.B. Glycerinmonostearat.

Eine Reihe von erfindungsgemäßen Zusammensetzungen sind bevorzugt. Dazu gehören Zusammensetzungen, insbesondere zum Verzehr geeignete Zusammensetzung, umfassend oder bestehend aus einer geschmacklich wirksamen Menge einer Verbindung oder einer Mischung wie vorstehend definiert und einem oder mehreren weiteren zum Verzehr geeigneten Bestandteilen. Besonders bevorzugt sind solche vorzugsweise sprühgetrockneten Zusammensetzungen, die als zum Verzehr geeignete Bestandteile feste Trägerstoffe und ggf. Aromakompositionen umfassen. Ebenfalls bevorzugt sind Zusammensetzungen wie vorstehend beschrieben, wobei die Zusammensetzungen sprühgetrocknet sind.

Insbesondere bevorzugt ist eine (vorzugsweise sprühgetrocknete) Zusammensetzung, die neben einer geschmacklich wirksamen Menge einer oder mehrerer Verbindungen der Formeln (I) und (*ent*-I) bzw. einer Mischung wie oben definiert umfassend (a) eine oder mehrere Verbindungen der Formeln (I) und (*ent*-I) sowie (b) eine oder mehrere Verbindungen der Formeln (II), (*ent*-II), (III), (*ent*-III), (IV), (*ent*-IV) einen oder mehrere zum Verzehr geeignete feste Trägerstoffe umfasst. Bevorzugte Zusammensetzungen bestehen aus der oder den erfindungsgemäß zu verwendenden Verbindungen der Formeln (I) und (*ent*-I) bzw. oben definierten Mischungen sowie dem oder den Trägerstoffen. Dabei gilt hinsichtlich bevorzugter Verbindungen und Mischungen das oben Gesagte entsprechend.

Vorteilhafte Trägerstoffe in diesen bevorzugten erfindungsgemäßen (vorzugsweise sprühgetrockneten) Zusammensetzungen sind Siliciumdioxid (Kieselsäure, Kieselgel), Kohlenhydrate und/oder Kohlenhydratpolymere (Polysaccharide), Cyclodextrine, Stärken, abgebaute Stärken (Stärkehydrolysate), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin oder Xanthan Gum zu nennen. Bevorzugte Stärkehydrolysate sind Maltodextrine und Dextrine.

Bevorzugte Trägerstoffe sind Siliciumdioxid, Gummi Arabicum und Maltodextrine, wobei hier wiederum Maltodextrine mit DE-Werten im Bereich 5 bis 20 bevorzugt sind. Es ist dabei unerheblich, welche Pflanze ursprünglich die Stärke zur Herstellung der Stärkehydrolysate geliefert hat. Geeignet und leicht verfügbar sind Mais-basierende Stärken sowie Stärken aus Tapioka, Reis, Weizen oder Kartoffeln. Die Trägerstoffe können dabei auch als Fließhilfsmittel fungieren, wie beispielsweise Siliciumdioxid.

Die erfindungsgemäßen Zusammensetzungen, die neben der oder den erfindungsgemäß zu verwendenden Verbindungen der Formeln (I) und (*ent*-I) bzw. oben definierten Mischungen noch einen oder mehrere feste Trägerstoffe umfassen, können beispielsweise durch mechanische Mischvorgänge, wobei gleichzeitig auch eine Zerkleinerung der Partikel erfolgen kann, oder mittels Sprühtrocknung hergestellt werden. Bevorzugt sind erfindungsgemäße Zusammensetzungen, die feste Trägerstoffe umfassen und mittels Sprühtrocknung hergestellt sind; hinsichtlich der Sprühtrocknung wird verwiesen auf US 3,159,585, US 3,971,852, US 4,532,145 oder US 5,124,162.

Bevorzugte erfindungsgemäße, Trägerstoffe umfassende Zusammensetzungen, die mittels Sprühtrocknung hergestellt wurden, besitzen eine mittlere Partikelgröße im Bereich von 30 bis 300 µm und eine Restfeuchte von kleiner oder gleich 5 Gew.-%.

Das Gewichtsverhältnis der Gesamtmasse der Verbindungen der Formeln (I) und (*ent*-I) bzw. -bei Verwendung der oben definierten Mischungen- das Gewichtsverhältnis der Gesamtmasse der Verbindungen der Formeln (I) und (*ent*-I)*,* (II), (*ent*-II), (III), (*ent*-III), (IV), (*ent*-IV) zu dem oder den zum Verzehr geeigneten festen Trägerstoffen liegt vorzugsweise im Bereich von 1 : 10 bis 1 : 100000, bevorzugt im Bereich von 1 : 50 (vorzugsweise von 1 : 100) bis 1 : 20000, besonders bevorzugt im Bereich von 1 : 100 (vorzugsweise von 1 : 1000) bis 1 : 5000, bezogen auf die Trockenmasse der Zusammensetzung

In der erfindungsgemäßen Zusammensetzung liegt die Summe der Bestandteile umfassend (i) Verbindungen der Formeln (I) und (*ent*-I) bzw. oben definierte Mischungen umfassend (a) eine oder mehrere Verbindungen der Formeln (I) und (*ent*-I) sowie (b) eine oder mehrere Verbindungen der Formeln (II), (*ent*-II), (III), (*ent*-III), (IV), (*ent*-IV) und (ii) Trägerstoffe vorzugsweise im Bereich von 70 bis 100 Gew.-%, bevorzugt im Bereich von 85 bis 100 Gew.-%.

Die Erfindung betrifft auch eine (vorzugsweise sprühgetrocknete) Zusammensetzung, die neben (i) einer geschmacklich wirksamen Menge einer oder mehrerer Verbindungen der Formeln (I) und (*ent*-I) bzw. einer Mischung wie oben definiert umfassend (a) eine oder mehrere Verbindungen der Formeln (I) und (*ent*-I) sowie (b) eine oder mehrere Verbindungen der Formeln (II), (*ent*-II), (III), (*ent*-III), (IV), (*ent*-IV) und (ii) festen Trägerstoffen zusätzlich (iii) eine oder mehrere Aromakompositionen umfasst bzw. aus den genannten Komponenten besteht. Dabei gilt hinsichtlich bevorzugter Verbindungen und Mischungen das oben Gesagte entsprechend.

Eine solche Aromakomposition im Sinne der vorliegenden Erfindung umfasst mindestens einen flüchtigen Aromastoff (nicht gemeint sind hierbei allerdings Verbindungen der Formeln (I) und (*ent*-I) oder oben definierte erfindungsgemäß zu verwendende Mischungen). Der flüchtige Aromastoff ist dabei vorzugsweise eine sensorisch wirksame Komponente mit einem Dampfdruck von größer oder gleich 0,01 Pa bei 25°C, vorzugsweise einem Dampfdruck von größer oder gleich 0,025 Pa bei 25°C. Ein Großteil der flüchtigen Aromastoffe weist einen Dampfdruck von größer oder gleich 1 Pa bei 25°C auf, diese Aromastoffe werden für die Verwendung in erfindungsgemäßen Zusammensetzungen als bevorzugt angesehen.

Beispiele für Aromastoffe, die Bestandteil der Aromakomposition sein können, finden sich z.B. in K. Bauer, D. Garbe und H. Surburg, Common Fragrance and Flavor Materials, 4th. Ed., Wiley-VCH, Weinheim 2001. Es seien beispielsweise genannt: organische Säuren (gesättigt und ungesättigt) wie z.B. Buttersäure, Essigsäure, Methylbuttersäure, Capronsäure; Alkohole (gesättigt und ungesättigt) wie z.B. Ethanol, Propylenglykol, Octenol, cis-3-Hexenol, Benzylalkohol; Sulfide und Disulfide wie z.B. Dimethylsulfid, Difurfuryldisulfid, Methylthiopropanal; Thiole wie z.B. Methylfuranthiol; Pyrazine und Pyrroline wie z.B. Methylpyrazin, Acetylpyrazin, 2-Propionylpyrrolin, 2-Acetylpyrrolin.

Die Aromakomposition können auch in Form von Reaktionsaromen (Maillard-Produkte) und/oder Extrakten bzw. ätherischen Ölen von Pflanzen oder Pflanzenteilen bzw. Fraktionen davon eingesetzt werden.

Eine weitere bevorzugte, zum Verzehr geeignete erfindungsgemäße Zusammensetzung, welche (a) eine oder mehrere erfindungsgemäß zu verwendende einzelne Verbindungen der Formeln (I) und (*ent*-I) bzw. (b) eine oben definierte Mischung umfasst, ist eine Wasser-in-Öl (W/O) - Emulsion. Neben der oder den erfindungsgemäß zu verwendenden Verbindungen der Formel (I) und (*ent*-I) bzw. oben definierten Mischungen, umfasst eine solche Emulsion Wasser, eine Ölphase, ein oder mehrere W/O-Emulgatoren, gegebenenfalls ein oder mehrere Antioxidantien und gegebenenfalls ein oder mehrere Stoffe zur Verstärkung einer antioxidativen Wirkung. Dabei gilt hinsichtlich bevorzugter Verbindungen und Mischungen das oben Gesagte entsprechend.

Besonders bevorzugt sind zum Verzehr geeignete Zusammensetzungen wie vorstehend definiert, worin die Zusammensetzung eine Wasser-in-Öl-Emulsion ist, umfassend Wasser, eine Ölphase, ein oder mehrere Wasser-in-Öl-Emulgatoren und gegebenenfalls ein oder mehrere Antioxidantien und gegebenenfalls ein oder mehrere Stoffe zur Verstärkung einer antioxidativen Wirkung.

Vorzugsweise umfasst eine solche erfindungsgemäße Zusammensetzung (W/O-Emulsion)
- insgesamt 0,01 bis 0,1 Gew.-% an Verbindungen der Formeln (I) und (*ent*-I) bzw. -bei Verwendung der oben definierten Mischungen- an Verbindungen der Formeln (I), (*ent*-I)*,* (II), (*ent*-II), (III), (*ent*-III), (IV), (*ent*-IV)
- 5 bis 30 Gew.-%, bevorzugt 8 bis 25 Gew.-% Wasser,
- 50 bis 90 Gew.-%, bevorzugt 60 bis 80 Gew.-% einer Ölphase,
- 0,1 bis 5 Gew.-% eines verzehrbaren W/O-Emulgators
- bezogen auf die Gesamtmasse der Zusammensetzung
- sowie gegebenenfalls ein oder mehrere Antioxidantien und gegebenenfalls einen oder mehrere Stoffe zur Verstärkung einer antioxidativen Wirkung

Besonders bevorzugt besteht eine solche erfindungsgemäße W/O-Emulsion aus den genannten Bestandteilen in den genannten Mengen.

Die Ölphase einer solchen erfindungsgemäßen W/O-Emulsion umfasst (oder besteht aus) vorzugsweise ein fettes Öl und/oder eine Aromakomposition. Bevorzugt sind Ölphasen umfassend oder bestehend aus einem fetten Öl und einer Aromakomposition.

Als fette Öle eignen sich beispielsweise Speiseöle, insbesondere Pflanzenöle. Geeignete fette Öle sind beispielsweise Borretschöl, Distelöl, Erdnussöl, Haselnussöl, Kokosöl, Kürbiskernöl, Leinöl, Maiskeimöl, Makadamianussöl, Mandelöl, Olivenöl, Palmkernöl, Pekannussöl, Pistazienkernöl, Rapsöl, Reiskeimöl, Sesamöl, Sojaöl, Sonnenblumenöl, Walnussöl oder Weizenkeimöl, oder daraus erhältliche Fraktionen. Es können auch flüssige Neutralester basierend auf mittelkettigen Fettsäuren und Glycerin verwendet werden, wie beispielsweise Miglyole (z.B. Miglyol 810, Miglyol 812). Bevorzugt sind Sonnenblumenöl, Palmkernöl und Rapsöl. Ferner bevorzugt werden fraktionierte Kokosöle verwendet, die hauptsächlich Fettsäurereste mit 6 bis 8 C-Atomen aufweisen. Diese zeichnen sich durch ihre Geschmacksneutralität sowie durch ihre gute Oxidationsstabilität aus.

Vorzugsweise wird der verzehrbare W/O-Emulgator gewählt aus der Gruppe bestehend aus Lecithin (E 322), Mono- und Diglyceride von Speisefettsäuren (E 471), Essigsäuremonoglyceride (E 472a), Milchsäuremonoglyceride (E 472b), Citronensäuremonoglyceride (E 472c), Weinsäuremonoglyceride (E 472d), Diacetylweinsäuremonoglyceride (E 472e), Sorbitanmonostearat (E 491).

Geeignete Antioxidantien und Stoffe, welche die antioxidative Wirkung verstärken können sind die natürlich vorkommenden Tocopherole und deren Derivate, Tocotrienole, Flavonoide, Ascorbinsäure und ihre Salze, alpha-Hydroxysäuren (z.B. Citronensäure, Milchsäure, Äpfelsäure, Weinsäure) und deren Na-, K- und Ca-Salze, aus Pflanzen isolierte Inhaltsstoffe, Extrakte bzw. Fraktionen davon z.B. aus Tee, Grüntee, Algen, Traubenkernen, Weizenkeimen, Rosmarin, Oregano, Flavonoide, Quercetin, phenolische Benzylamine. Weiterhin sind als Antioxidantien Propylgallat, Octylgallat, Dodecylgallat, Butylhydroxyanisol (BHA), Butylhydroxytoluol (BHT), Lecithine, Mono- und Diglyceride von Speisefettsäuren verestert mit Citronensäure, Orthophosphate und Na-, K- und Ca-Salze der Monophosphorsäure und Ascorbylpalmitat geeignet.

Die erfindungsgemäßen W/O-Emulsionen eignen sich besonders zum Aufbringen auf Lebensmitteloberflächen, wobei die Lebensmittel vorzugsweise einen Wassergehalt von höchstens 10 Gew.-%, bevorzugt von höchstens 5 Gew.-% aufweisen. In einer bevorzugten Ausführungsform weist die erfindungsgemäße W/O-Emulsion bei Aufbringungstemperatur eine ausreichend niedrige Viskosität auf, so dass ein Aufbringen der W/O-Emulsion mittels Sprühen möglich ist. Bevorzugte Lebensmittel, auf deren Oberflächen eine erfindungsgemäße W/O-Emulsion aufgebracht werden kann sind beispielsweise Cracker, Chips (z.B. auf Basis von Kartoffeln, Mais, Getreide oder Brot), extrudierte Knabberartikel (Snacks, z.B. Flips) oder Laugengebäck (z.B. Salzstangen). Erfindungsgemäße W/O-Emulsionen werden regelmäßig in einer Menge von 0,5 bis 6 Gew.-% auf die Lebensmitteloberflächen aufgebracht, bezogen auf das Gesamtgewicht des Lebensmittels.

Wie bereits erwähnt, betrifft ein Aspekt der vorliegenden Erfindung die Verwendung einer Verbindung der oben definierten Formeln (I) und (*ent*-I), insbesondere der oben als bevorzugt angegebenen Verbindungen (1) bis (6), bzw. der oben definierten Mischungen umfassend (a) eine oder mehrere Verbindungen der Formeln (I) und (*ent*-I) sowie (b) eine oder mehrere Verbindungen der Formeln (II), (*ent*-II), (III), (*ent*-III), (IV), (*ent*-IV) zum Erzeugen, Vermitteln, Modifizieren oder Verstärken eines Umami-Geschmacks.

Vorzugsweise werden die Verbindungen der Formeln (I) und (*ent*-I) (in geschmacklich wirksamer Menge), die oben definierten Mischungen umfassend (a) eine oder mehrere Verbindungen der Formeln (I) und (*ent*-I) sowie (b) eine oder mehrere Verbindungen der Formeln (II), (*ent*-II), (III), (*ent*-III), (IV), (*ent*-IV) oder die oben definierten erfindungsgemäßen Zusammensetzungen in der Ernährung oder dem Genuss dienenden (i) gebrauchs- oder verzehrfertigen Zubereitungen oder (ii) Halbfertigwaren eingesetzt, insbesondere in der Ernährung oder dem Genuss dienenden Natriumglutamat-reduzierten oder -freien Zubereitungen. Dabei gilt hinsichtlich bevorzugter Verbindungen und Mischungen das oben Gesagte entsprechend.

Besonders bevorzugt ist ebenfalls die Verwendung von Verbindungen oder Mischungen bestehend aus zwei oder mehreren Verbindungen oder enthaltend eine oder mehrere Verbindungen ausgewählt aus der Gruppe bestehend aus Verbindungen der Formeln (I) und (*ent*-I) wie vorstehend definiert oder von Zusammensetzungen wie vorstehend definiert in der Ernährung oder dem Genuss dienenden
(i) gebrauchs- oder verzehrfertigen Zubereitungen oder
(ii) Halbfertigwaren,

insbesondere in der Ernährung oder dem Genuss dienenden Natriumglutamat-reduzierten oder -freien Zubereitungen.

Der Begriff "Natriumglutamat-reduziert" bedeutet dabei, dass die erfindungsgemäße Zubereitung oder Halbfertigware deutlich weniger Natriumglutamat enthält, als in der üblichen Zubereitung oder Halbfertigware enthalten ist; der Natriumglutamatgehalt liegt dabei um 5 bis <100 Gew.-%, bevorzugt 10 bis 50 Gew.-%, besonders bevorzugt 20 bis 50 Gew.-% unter dem Natriumglutamatgehalt der üblichen Zubereitung. Sofern neben einer oder mehreren Verbindungen der Formeln (I) und (*ent*-I) bzw. einer oben definierten Mischung umfassend (a) eine oder mehrere Verbindungen der Formeln (I) und (*ent*-I) sowie (b) eine oder mehrere Verbindungen der Formeln (II), (*ent*-II), (III), (*ent*-III), (IV), (*ent*-IV) auch Natriumglutamat in einer erfindungsgemäßen Zubereitung oder Halbfertigware vorliegt, liegt das Gewichtsverhältnis der Gesamtmenge an Verbindungen der Formeln (I) und (*ent*-I) bzw. oben definierten Mischungen zu Natriumglutamat vorzugsweise im Bereich von 1 : 1 bis 1 : 200.

Die Erfindung betrifft auch Zubereitungen umfassend Verbindungen oder Mischungen bestehend aus zwei oder mehreren Verbindungen oder enthaltend eine oder mehrere Verbindungen ausgewählt aus der Gruppe bestehend aus Verbindungen der Formeln (I) und (*ent*-I) wie vorstehend definiert oder von Zusammensetzungen wie vorstehend definiert.

Bevorzugt sind erfindungsgemäße der Ernährung oder dem Genuss dienende gebrauchs- oder verzehrfertige Zubereitungen enthalten eine oder mehrere erfindungsgemäß zu verwendende Verbindungen der Formeln (I) und (*ent*-I) bzw. -bei Verwendung der oben definierten Mischungen- Verbindungen der Formeln (I), (*ent*-I), (II), (*ent*-II), (III), (*ent*-III), (IV), (*ent*-IV) vorzugsweise in einer Menge im Bereich von 0,01 ppm bis 100 ppm, bevorzugt im Bereich von 0,1 ppm bis 50 ppm, insbesondere bevorzugt im Bereich von 0,5 ppm (vorzugsweise von 1 ppm) bis 30 ppm bezogen auf das Gesamtgewicht der gebrauchs- oder verzehrfertigen Zubereitung.

Die Erfindung betrifft auch Halbfertigwaren umfassend Verbindungen oder Mischungen bestehend aus zwei oder mehreren Verbindungen oder enthaltend eine oder mehrere Verbindungen ausgewählt aus der Gruppe bestehend aus Verbindungen der Formeln (I) und (*ent*-I) wie vorstehend definiert oder von Zusammensetzungen wie vorstehend definiert.

Bevorzugt sind erfindungsgemäße Halbfertigwaren zur Herstellung von der Ernährung oder dem Genuss dienenden gebrauchs- oder verzehrfertigen Zubereitungen enthalten eine oder mehrere erfindungsgemäß zu verwendende Verbindungen der Formeln (I) und (*ent*-I) bzw. -bei Verwendung der oben definierten Mischungen- Verbindungen der Formeln (I), (*ent*-I), (II), (*ent*-II), (III), (*ent*-III), (IV), (*ent*-IV), vorzugsweise in einer Menge im Bereich von 10 ppm bis 100000 ppm (vorzugsweise bis 800 ppm), bevorzugt im Bereich von 25 ppm bis 5000 ppm (vorzugsweise bis 750 ppm), insbesondere bevorzugt im Bereich von 50 ppm bis 1200 ppm (vorzugsweise bis 700 ppm), bezogen auf das Gesamtgewicht der Halbfertigware.

Besonders relevant sind erfindungsgemäße Natriumglutamat-reduzierte Zubereitungen, die Natriumglutamat umfassen, wobei die Menge des Natriumglutamats nicht ausreicht, um in einer Vergleichszubereitung, die keine erfindungsgemäße Mischung umfasst, aber ansonsten identisch zusammengesetzt ist (normale Natriumglutamat-reduzierte Zubereitung), als befriedigender Umami-Geschmack wahrgenommen zu werden, und die Menge der erfindungsgemäßen Mischung ausreicht, um einen befriedigenden Umami-Geschmackseindruck zu erreichen.

Der Ernährung oder dem Genuss dienende Zubereitungen im Sinne der Erfindung sind insbesondere Backwaren (z.B. Brot, Trockenkekse, Kuchen, sonstiges Gebäck), Getränke (z.B. Gemüsesäfte, Gemüsesaftzubereitungen), Instantgetränke (z.B. Instant-Gemüsegetränke), Fleischprodukte (z.B. Schinken, Frischwurst- oder Rohwurstzubereitungen, gewürzte oder marinierte Frisch- oder Pökelfleischprodukte), gewürzte oder marinierte Fischprodukte (z.B. Surimi), Eier oder Eiprodukte (Trockenei, Eiweiß, Eigelb), Getreideprodukte (z.B. vorgegarte Fertigreis-Produkte, Reismehlprodukte, Hirse-und Sorghum-Produkte, rohe oder vorgegarte Nudeln und Pastaprodukte), Milchprodukte (z.B. Frischkäse, Weichkäse, Hartkäse, Milchgetränke, Molke, Butter, teilweise oder ganz hydrolysierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Gemüsezubereitungen (z.B. Ketchup, Soßen, Trockengemüse, Tiefkühlgemüse, vorgegarte Gemüse, in Essig eingelegte Gemüse, Gemüsekonzentrate oder -pasten, eingekochte Gemüse, Kartoffelzubereitungen), Knabberartikel (z.B. gebackene oder frittierte Kartoffelchips oder Kartoffelteigprodukte, Brotteigprodukte, Extrudate auf Mais-, Reis- oder Erdnussbasis), Produkte auf Fett- und Ölbasis oder Emulsionen derselben (z.B. Mayonnaise, Aufstrich, Remoulade, Dressings, Würzzubereitungen), sonstige Fertiggerichte und Suppen (z.B. Trockensuppen, Instant-Suppen, vorgegarte Suppen), Soßen (Instant-Soßen, Trockensoßen, Fertigsoßen), Gewürze oder Gewürzzubereitungen (z.B. Senfzubereitungen, Meerrettichzubereitungen), Würzmischungen sowie insbesondere Aufstreuwürzungen (englisch: Seasonings), die beispielsweise im Snackbereich Anwendung finden.

Besonders bevorzugt sind Halbfertigwaren oder der Ernährung oder dem Genuss dienende Zubereitungen (vorzugsweise mit reduziertem Gehalt an Natrium-Glutamat), z.B. Backwaren (z.B. Brot, Trockenkekse, Kuchen, sonstiges Gebäck), Gemüsesaftzubereitungen, Fleischprodukte (z.B. Schinken, Frischwurst- oder Rohwurstzubereitungen, gewürzte oder marinierte Frisch- oder Pökelfleischprodukte), gewürzte oder marinierte Fischprodukte (z.B. Surimi), Eier oder Eiprodukte (Trockenei, Eiweiß, Eigelb), Getreideprodukte (z.B. vorgegarte Fertigreis-Produkte, rohe oder vorgegarte Nudeln und Pastaprodukte), Milchprodukte (z.B. Frischkäse, Weichkäse, Hartkäse, Milchgetränke, Molke, Butter, teilweise oder ganz hydrolysierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fischsoßen wie z.B. Anchovis-Soßen, Austernsoßen, Gemüsezubereitungen (z.B. Ketchup, Soßen, Trockengemüse, Tiefkühlgemüse, vorgegarte Gemüse, in Essig eingelegte Gemüse, eingekochte Gemüse, Kartoffelzubereitungen), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Aufstrich, Remoulade, Dressings, Würzzubereitungen), Fertiggerichte, Suppen (z.B. Trockensuppen, Instant-Suppen, vorgegarte Suppen), Brühwürfel, Soßen (Instant-Soßen, Trockensoßen, Fertigsoßen), Gewürze, Würze, Würzmittel, Würzmischungen sowie insbesondere Aufstreuwürzungen (englisch: Seasonings), die beispielsweise im Snackbereich Anwendung finden.

Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen, z. B. als Nahrungsergänzungsmittel.

Die erfindungsgemäßen Halbfertigwaren dienen in der Regel zur Herstellung von der Ernährung oder dem Genuss dienenden gebrauchs- oder verzehrfertigen Zubereitungen.

Insbesondere können erfindungsgemäße Halbfertigwaren zur additiven Verstärkung des Umami-Geschmacks von Natriumglutamat-reduzierten Nahrungs- und Genussmitteln und auch direkt als Würzmittel für die industrielle oder nicht-industrielle Zubereitung von Nahrungs- und/oder Genussmitteln dienen.

Erfindungsgemäße Halbfertigwaren enthalten bevorzugt:
- eine Gesamtmenge von 10 ppm bis 100000 ppm (vorzugsweise bis 800 ppm), bevorzugt 25 ppm bis 5000 ppm (vorzugsweise bis 750 ppm), insbesondere 50 ppm bis 1200 ppm (vorzugsweise bis 700 ppm), an Verbindungen der Formeln (I) und (*ent*-I) bzw. -bei Verwendung der oben definierten Mischungen- an Verbindungen der Formeln (I), (*ent*-I), (II), (*ent*-II), (III), (*ent*-III), (IV), (*ent*-IV),
- kein Natriumglutamat oder einen Anteil von 0,00001 bis 10 Gew.-%, bevorzugt 0,0001 bis 5 Gew.-%, insbesondere 0,001 Gew.-% bis 2 Gew.-% an Natriumglutamat,
- und gegebenenfalls einen Anteil von 0,0001 Gew.-% bis 90 Gew.-%, bevorzugt 0,001 Gew.-% bis 30 Gew.-% einer Aromakomposition, jeweils bezogen auf das Gesamtgewicht der Halbfertigware.

Die erfindungsgemäßen Zusammensetzungen, Zubereitungen bzw. Halbfertigwaren werden vorzugsweise hergestellt, indem die Verbindungen der Formeln (I) und (*ent*-I) oder oben definierten Mischungen in Mischungen aus Ethanol und gegebenenfalls demineralisiertem und/oder gereinigtem Wasser gelöst und gemischt werden; anschließend werden die Lösungen durch einen Trocknungsprozess, vorzugsweise einen Sprühtrocknungs-, Vakuumgefriertrockungs-, Umkehrosmose-, Eindampf- oder anderen Konzentrationsprozess oder eine Kombination der genannten Prozesse, in eine (zumindest nahezu) feste Zubereitung überführt. Dabei kann die Trocknung unter Zuhilfenahme von Trägerstoffen (z.B. Stärke, Stärkederivate, Maltodextrin, Kieselgel, siehe oben) oder Hilfsstoffen (z.B. Pflanzengummen, Stabilisierungsmittel), erfolgen. Vorzugsweise erfolgt die Trocknung mittels Sprühtrocknung oder Vakuumgefriertrocknung.

Bevorzugte erfindungsgemäße Zusammensetzungen, Zubereitungen bzw. Halbfertigwaren sind Würze, Würzmischungen, Würzmittel, Brühwürfel, Instant-Suppen, Instant-Soßen, vegetarische Fertiggerichte, fleischhaltige Fertiggerichte, Fischsoßen wie z.B. Anchovis-Soßen, Austernsoßen und Sojasoßen.

Gemäß einer weiteren bevorzugten Ausführungsform werden zur Herstellung erfindungsgemäßer Zusammensetzungen, Zubereitungen bzw. Halbfertigwaren Verbindungen der Formeln (I) und (*ent*-I) bzw. oben definierte Mischungen sowie gegebenenfalls andere Bestandteile zunächst in Emulsionen, in Liposomen (z.B. ausgehend von Phosphatidylcholin) in Microsphären, in Nanosphären oder auch in Kapseln, Granulate oder Extrudate aus einer für Lebens- und Genussmittel geeigneten Matrix (z.B. aus Stärke, Stärkederivaten, Cellulose oder Cellulosederivaten wie Hydroxypropylcellulose, anderen Polysacchariden wie Alginat, natürlichen Fetten, natürlichen Wachsen wie Bienenwachs oder Carnaubawachs oder aus Proteinen wie Gelatine) eingearbeitet.

In einem weiteren bevorzugten Herstellungsverfahren werden Verbindungen der Formeln (I) und (*ent*-I) bzw. oben definierten Mischungen mit einem oder mehreren geeigneten Komplexbildnern, beispielsweise mit Cyclodextrinen oder Cyclodextrinderivaten, bevorzugt alpha- oder beta-Cyclodextrin, komplexiert und in dieser komplexierten Form eingesetzt.

Besonders bevorzugt sind erfindungsgemäße Zubereitungen, bei denen die Matrix so gewählt ist, dass die Verbindungen der Formeln (I) und (*ent*-I) bzw. oben definierten Mischungen verzögert von der Matrix freigegeben werden, so dass man eine langanhaltende Wirkung erhält. Als Matrix können hier z.B. natürliche Fette, natürliche Wachse (z.B. Bienenwachs, Carnaubawachs) oder auch natürliche Ballaststoffe (Weizenfasern, Apfelfasern, Haferfasern, Orangenfasern) verwendet werden.

Weitere Bestandteile einer erfindungsgemäßen, der Ernährung oder dem Genuss dienenden verzehrfertigen Zubereitung bzw. Halbfertigware können übliche Grund-, Hilfs-und Zusatzstoffe für Nahrungs- oder Genussmittel sein, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Kräuter, Nüsse, Gemüsesäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nucleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carrageenan, Alginat), Konservierungsstoffe (z.B. Benzoesäure und deren Salze, Sorbinsäure und deren Salze), Antioxidantien (z.B. Tocopherol, Ascorbinsäure), Chelatoren (z.B. Citronensäure), organische oder anorganische Säuerungsmittel (z.B. Essigsäure, Phosphorsäure), zusätzliche Bitterstoffe (z.B. Chinin, Coffein, Limonin, Amarogentin, Humolone, Lupolone, Catechine, Tannine), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigenzien.

Bevorzugt enthalten erfindungsgemäße Zusammensetzungen, Zubereitungen oder Halbfertigwaren eine Aromakomposition, um den Geschmack und/oder den Geruch abzurunden und zu verfeinern. Eine erfindungsgemäße Zusammensetzung, die als weitere Bestandteile einen festen Trägerstoff und eine Aromakomposition umfasst, wurde bereits weiter oben beschrieben. Geeignete Aromakompositionen enthalten z.B. synthetische, natürliche oder naturidentische Aroma-, Riech- und Geschmacksstoffe, Reaktionsaromen, Raucharomen oder andere aromagebende Zubereitungen (z.B. Protein[teil]hydrolysate, Grillaromen, Pflanzenextrakte, Gewürze, Gewürzzubereitungen, Gemüse und/oder Gemüsezubereitungen) sowie geeignete Hilfs- und Trägerstoffe. Insbesondere sind hier die nicht-erfindungsgemäßen Aromenkompositionen oder deren Bestandteile geeignet, die einen röstigen, fleischigen (insbesondere Huhn, Fisch, Meerestiere, Rind, Schwein, Lamm, Schaf, Ziege), gemüsigen (insbesondere Tomate, Zwiebel, Knoblauch, Sellerie, Lauch, Pilze, Auberginen, Seetang), einen würzigen (insbesondere schwarzer und weißer Pfeffer, Chili, Paprika, Kardamom, Muskat, Piment, Senf und Senf-Produkte), gebratenen, hefigen, gesottenen, fettigen, salzigen und/oder scharfen Aromaeindruck verursachen und somit den würzigen Eindruck verstärken können. In der Regel enthalten die Aromakompositionen mehr als einen der genannten Inhaltsstoffe.

In einer weiteren Ausgestaltung der vorliegenden Erfindung werden Verbindungen der Formeln (I) und (*ent*-I) bzw. oben definierte Mischungen in Zusammensetzungen, Zubereitungen und Halbfertigwaren in Kombination mit zumindest einer weiteren, nicht für sich erfindungsgemäßen Substanz zum Maskieren oder Vermindern eines unangenehmen (bitteren, metallischen, kalkigen, sauren, adstringierenden) Geschmackseindrucks oder zur Verstärkung oder Erzeugung eines angenehmen Geschmackseindrucks (süß, salzig, Umami) verwendet.

Auf diese Weise kann eine Verstärkung des Geschmacks, insbesondere des Umami-Geschmacks, erreicht werden. Diese weiteren Substanzen können aus der folgenden Liste ausgewählt werden, ohne die Erfindung damit einzuschränken: Mononatriumglutamat, Glutaminsäure, Nucleotide (z.B. Adenosin-5'-monophosphat, Cytidin-5'-monophosphat, Inosin-5'-monophosphat, Guanosin-5'-monophosphat) oder deren pharmazeutisch akzeptable Salze, Lactisole, Hydroxyflavanone (z.B. Eriodictyol, Homoeriodictyol oder deren Natriumsalze), insbesondere gemäß EP 1 258 200, Hydroxybenzoesäureamide (z. B. 2,4-Dihydroxybenzoesäurevanillylamid, 4-Hydroxybenzoesäurevanillylamid), Gemische von Molkeproteinen mit Lecithinen, Hefeextrakte, Pflanzenhydrolysate, pulverisierte Gemüse (z.B. Zwiebelpulver, Tomatenpulver), Pflanzenextrakte (z.B. von Liebstöckel oder von Pilzen wie Shiitake), Meeralgen und Mineralsalzmischungen.

In einer bevorzugten Ausgestaltung der vorliegenden Erfindung werden die erfindungsgemäß zu verwendenden Verbindungen der Formel (I) und/oder ent-(I) in den erfindungsgemäßen Zusammensetzungen, Zubereitungen und Halbfertigwaren in Kombination mit zumindest einem süßverstärkenden Stoff eingesetzt, insbesondere mit einer oder mehreren Verbindungen gemäß WO 2007/014879 A1 oder WO 2007/107596 A1, speziell zusammen mit Hesperetin und/oder Phloretin. Hierdurch wird eine Verstärkung und eine Vertiefung sowie Abrundung des Geschmacksprofils erzielt, inbesondere in Zusammensetzungen, Zubereitungen und Halbfertigwaren mit würzigem und/oder salzigem Geschmack. Der Gesamtanteil an Hesperetin und/oder Phloretin in solchen Zusammensetzungen oder Zubereitungen liegt vorzugsweise im Bereich von 1 bis 400 ppm, bevorzugt im Bereich von 5 - 200 ppm, bezogen auf das Gesamtgewicht der Zusammensetzung oder Zubereitung.

Zusätzlich zu einem oder mehreren süßverstärkenden Stoffen können in den erfindungsgemäßen Zusammensetzungen, Zubereitungen und Halbfertigwaren vorzugsweise Geschmackstoffe enthalten sein, die einen trigeminalen Reiz (tingling, kribbeln, scharf, kühlend etc.) bewirken. So wurde bei der Kombination der erfindungsgemäß zu verwendenden Verbindungen der Formel (I) und/oder ent-(I) mit Hesperetin und/oder Phloretin einerseits und cis- und/oder trans-Pellitorin (siehe WO 2004/000787 bzw. WO 2004/043906) andererseits ein weiter verbessertes und von Konsumenten bevorzugtes Geschmacksprofil erreicht. Der Gesamtanteil an cis-und/oder trans-Pellitorin in solchen Zusammensetzungen oder Zubereitungen liegt vorzugsweise im Bereich von 0,5 bis 500 ppm, bevorzugt im Bereich von 5 - 100 ppm, bezogen auf das Gesamtgewicht der Zusammensetzung oder Zubereitung.

Modulierende Aroma- und/oder Geschmackstoffe werden vorzugsweise ausgewählt aus der Gruppe bestehend aus Adenosin-5'-monophosphat, Cytidin-5'-monophosphat, Inosin-5'-monophosphat, und deren pharmazeutisch akzeptablen Salzen; Lactisolen; 2,4-Dihydroxybenzoesäure; 3-Hydroxybenzoesäure; Natriumsalzen, vorzugsweise Natriumchlorid, Natriumlactat, Natriumcitrat, Natriumacetat, Natriumgluconoat; Hydroxyflavanonen, wie zum Beispiel Eriodictyol, Homoeriodictyol, und deren Natriumsalzen; Hydroxybenzoesäureamiden, wie zum Beispiel 2,4-Dihydroxybenzoesäurevanillylamid, 2,4-Dihydroxybenzoesäure-*N*-(4-hydroxy-3-methoxybenzyl)amid, 2,4,6-Trihydroxybenzoesäure-*N*-(4-hydroxy-3-methoxybenzyl)amid, 2-Hydroxy-benzoesäure-N-4-(hydroxy-3-methoxybenzyl)amid, 4-Hydroxybenzoesäure-*N-*(4-hydroxy-3-methoxybenzyl)-amid, 2,4-Dihydroxybenzoesäure-*N*-(4-hydroxy-3-methoxybenzyl)amid-Mono-natriumsalz, 2,4-Dihydroxybenzoesäure-*N*-2-(4-hydroxy-3-methoxyphenyl)-ethyl-amid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid; 4-Hydroxybenzoesäurevanillylamid (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl) ethanon) (insbesondere solche wie beschrieben in WO 2006/106023, die bezüglich der darin offenbarten entsprechenden Verbindungen auf dem Wege der Verweisung Bestandteil dieser Anmeldung wird); Hydroxyphenlaalkandionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); γ-Aminobuttersäuren (insbesondere solche wie beschrieben in WO 2005/096841, die bezüglich der darin offenbarten entsprechenden Verbindungen auf dem Wege der Verweisung Bestandteil dieser Anmeldung wird) und Divanillinen (insbesondere Divanillin wie beschrieben in WO 2004/078302, die bezüglich der darin offenbarten entsprechenden Verbindungen auf dem Wege der Verweisung Bestandteil dieser Anmeldung wird); Bicyclo[4.1.0]heptan-7-carbonsäureamide, insbesondere solche wie beschrieben in PCT/EP2007/061171 sowie den darauf basierenden Dokumenten (Symrise), die bezüglich der darin offenbarten entsprechenden Verbindungen auf dem Wege der Verweisung Bestandteil dieser Anmeldung wird; Cyclopropancarbonsäure(3-methyl-cyclohexyl)amide, insbesondere solche wie beschrieben in US-Provisional 60/916,589 vom 08.05.2007 sowie den darauf basierenden Dokumenten (Symrise), die bezüglich der darin offenbarten entsprechenden Verbindungen auf dem Wege der Verweisung Bestandteil dieser Anmeldung wird; Aromatische Neo-Menthylamide, insbesondere solche wie beschrieben in US-Provisional Application 60/984,023 vom 31.10.2007 sowie den darauf basierenden Dokumenten (Symrise), die bezüglich der darin offenbarten entsprechenden Verbindungen auf dem Wege der Verweisung Bestandteil dieser Anmeldung wird.

Gemäß einer weiteren Ausgestaltung der vorliegenden Erfindung umfasst eine jeweils wie oben beschriebene erfindungsgemäße Zusammensetzung, gebrauchs- oder verzehrfertige Zubereitung oder Halbfertigware insbesondere zusätzlich einen oder mehrere süßverstärkende Stoffe. Insbesondere werden die erfindungsgemäßen Verbindungen der Formeln (I) oder (II) bzw. die erfindungsgemäßen Mischungen (wie oben beschrieben) hierbei in Kombination mit zumindest einem süßverstärkenden Stoff eingesetzt, insbesondere mit einer oder mehreren Verbindungen gemäß WO 2007/014879 A1 oder WO 2007/107596 A1, speziell zusammen mit Hesperetin und/oder Phloretin. Hierdurch wird vorteilhafterweise eine Verstärkung und eine Vertiefung sowie Abrundung des Geschmacksprofils, insbesondere des würzigen und/oder salzigen Geschmacks der Zusammensetzung, Zubereitung oder Halbfertigware erreicht. Für Halbfertigwaren liegt der Gesamtanteil an Hesperetin und/oder Phloretin hierbei vorzugsweise im Bereich von 10 bis 100000 ppm, bezogen auf das Gesamtgewicht der Halbfertigware, während im verzehrfertigen Lebensmittel der Gesamtanteil an Hesperetin und/oder Phloretin bezogen auf das Gesamtgewicht des Lebensmittels vorzugsweise im Bereich von 1 bis 400 ppm, bevorzugt im Bereich von 5 bis 200 ppm liegt.

Vorzugsweise sind in den erfindungsgemäßen Zusammensetzungen, Zubereitungen bzw. Halbfertigwaren zusätzlich ein oder mehrere süßverstärkende Stoffe und ein oder mehrere weitere Geschmacksstoffe, die einen trigeminalen Reiz (tingling, kribbeln, scharf, kühlend etc.) bewirken, enthalten. Insbesondere bei der Kombination der erfindungsgemäßen Verbindungen der Formeln (I) oder (II) bzw. der entsprechenden erfindungsgemäßen Mischungen mit Hesperetin und/oder Phloretin aber auch mit cis-und/oder trans-Pellitorin (siehe WO 2004/000787 bzw. WO 2004/043906) wird ein weiter verbessertes und vom Konsumenten bevorzugtes Geschmacksprofil erreicht. Dabei liegt der Gesamtanteil an cis- und/oder trans-Pellitorin in diesen Zusammensetzungen bzw. Zubereitungen oder Halbfertigwaren vorzugsweise im Bereich von 0,1 bis 500 ppm, bevorzugt im Bereich von 5 bis 100 ppm, bezogen auf das Gesamtgewicht der Zusammensetzung, Zubereitung oder Halbfertigware.

Aus dem vorstehenden Text ergibt sich, dass ein weiterer Aspekt der vorliegenden Erfindung auch ein Verfahren zum Erzeugen, Vermitteln, Modifizieren oder Verstärken eines Geschmacks, insbesondere eines Umami-Geschmacks, in einer der Ernährung, der Mundpflege oder dem Genuss dienenden (i) gebrauchs- oder verzehrfertigen Zubereitung oder (ii) Halbfertigware betrifft. Ein solches erfindungsgemäßes Verfahren umfasst den folgenden Schritt:
Vermischen einer geschmacklich wirksamen Menge einer oder mehrerer Verbindungen der Formeln (I) und (*ent*-I) oder oben definierter Mischungen oder einer erfindungsgemäßen Zusammensetzung mit einem oder mehreren weiteren Bestandteilen der (i) verzehr- oder gebrauchsfertigen Zubereitung oder der (ii) Halbfertigware, oder
Applizieren einer geschmacklich wirksamen Menge einer oder mehrerer Verbindungen der Formeln (I) und (*ent*-I) oder oben definierter Mischungen oder einer erfindungsgemäßen Zusammensetzung auf einen oder mehrere weitere Bestandteile der (i) verzehr- oder gebrauchsfertigen Zubereitung oder der (ii) Halbfertigware, oder
Einbetten einer geschmacklich wirksamen Menge einer oder mehrerer Verbindungen der Formeln (I) und (*ent*-I) oder oben definierter Mischungen oder einer erfindungsgemäßen Zusammensetzung in ein Hüll- oder Matrixmaterial.

Dabei gilt hinsichtlich bevorzugter Verbindungen und Mischungen das oben Gesagte entsprechend.

Weitere Aspekte der vorliegenden Erfindung ergeben sich aus den nachfolgenden Beispielen und den beigefügten Ansprüchen.

### Beispiele:

Die nachfolgenden Beispiele erläutern die Erfindung. Sofern nicht anders angegeben, beziehen sich alle Angaben auf das Gewicht.

Die als Ausgangsmaterial benötigten *Neo*-Menthylamine werden nach einer Vorschrift von Wallach et al. (Ann. Chem. 1893, 276, 296-313) aus den entsprechenden Menthonen in einer Reinheit ≥ 90%, bevorzugt ≥ 95%, dargestellt. Ein Gemisch aller möglichen isomeren Menthylamine kann nach oben genannter Vorschrift ohne entsprechende Kristallisation der Menthylformamide (ein Zwischenprodukt) in einer Reinheit von 99,3% erhalten werden (24,1% Menthylamin, 55,5% Neo-Menthylamin, 2,4% *Iso*-Menthylamin, 17,3% *Neo*-*Iso*-Menthylamin). Dabei können sowohl enantiomerenreine *D*- bzw. *L-*Menthone als auch eine racemische *D*/*L*-Menthonmischung verwandt werden. Alle eingesetzten Menthone können mit bis zu 25% der entsprechenden *Iso*-Menthone vermischt sein. Die diasteromerenangereicherten Neo-Menthylamine können ebenfalls in einer Reinheit von ≥ 60%, bevorzugt ≥ 90%, besonders bevorzugt ≥ 95% unter optimierten Bedingungen durch fraktionierte Kristallisation der entsprechenden Formamide gewonnen werden.

Die benötigten Neo-Menthole sind sowohl als Racemat als auch als reine Enantiomere kommerziell erhältlich.

Die Geschmacksprofile wurden von einem geschulten Panel aus mindestens 5 Testpersonen durch Verkostung auf einer 0,5%igen Salz und einer 0,5%igen Zuckerlösung bestimmt.
**Allgemeine Arbeitsvorschrift (AAV 1)**: Umsetzung von Neo-Menthol mit Triphosgen und primären Aminen zu den entsprechenden Carbamaten
   Zu einer auf 0 °C abgekühlten Lösung aus 1,0 g (6.4 mmol) des entsprechenden Neo-Menthols (D, L oder racemisch) und 0,7 g (8,4 mmol) Pyridin in 30 ml Dichlormethan wird langsam eine Lösung aus 0,8g (2,6 mmol) Triphosgen in 10 ml Dichlormethan zugetropft. Die Lösung wird für 1 h bei 0 °C gerührt, bevor erneut 1,0 ml (12,4 mmol) Pyridin und 10,2 mmol des entsprechenden Amins zugegeben werden. Nach weiteren 4 h Rühren bei 0 °C werden 35 ml 1 M Salzsäure zugegeben und die wässrige Phase dreimal mit je 40 ml Dichlormethan extrahiert. Die vereinigten organischen Phasen werden mit gesättigter Kochsalzlösung gewaschen, über Natriumsulfat getrocknet und am Rotationsverdampfer vom Lösungsmittel befreit. Die Aufreinigung erfolgt durch Chromatographie mit verschiedenen Mischungen aus Pentan und Diethylether an Kieselgel.
**Allgemeine Arbeitsvorschrift (AAV 2):** Umsetzung von Neo-Menthylamin mit Triphosgen zu den entsprechenden Isocyanaten
   Unter Schutzgas wird zu einer auf 0 °C abgekühlten Lösung aus 15,0 g (50,5 mmol) Triphosgen in 200 ml Dichlormethan eine Lösung aus 25,9 g (166,7 mmol) des entsprechenden Neo-Menthylamins (*D, L* oder racemisch) in 75 ml Dichlormethan so langsam zugetropft, dass die Innentemperatur 5 °C nicht überschreitet. Anschließend wird für drei Stunden bei 0 °C und weitere 14 Stunden bei Raumtemperatur gerührt. Die Reaktionsmischung wird mit 100 ml Dichlormethan verdünnt und zweimal mit 150 ml Eiswasser sowie zweimal mit 150 ml NaHCO₃-Lösung gewaschen. Die organische Phase wird am Rotationsverdampfer eingeengt und das gewünschte Produkt durch Chromatographie an Kieselgel mit Pentan / Diethylether (10:1) erhalten.
**Allgemeine Arbeitsvorschrift (AAV 3):** Umsetzung von Neo-Menthylamin mit Schwefelkohlenstoff zu den entsprechenden Isothiocyanaten
   Unter Schutzgas wird zu einer auf 0 °C abgekühlten Mischung aus 14,5 g (93,4 mmol) des entsprechenden Neo-Menthylamins (*D*, *L* oder racemisch) und 9,4 g (92,9 mmol) Triethylamin in 150 ml Cyclohexan werden 7,1 g (93,2 mmol) Schwefelkohlenstoff so langsam zugetropft, dass die Innentemperatur 5 °C nicht überschreitet. Anschließend wird für drei Stunden bei 0 °C gerührt und der entstandene Feststoff abfiltriert und viermal mit je 25 ml Diethylether gewaschen. Der Feststoff wird schließlich in 150 ml Dichlormethan gelöst, mit weiteren 9,4 g (92,9 mmol) Triethylamin versetzt, auf 0 °C abgekühlt und so langsam mit 10,1g (93,1 mmol) Chlorameisensäureethylester versetzt, dass die Innentemperatur 5 °C nicht überschreitet. Anschließend wird zuerst für 30 min bei 0 °C nachgerührt, bevor das Reaktionsgemisch unter CO₂ Entwicklung für 2 Stunden refluxiert wird. Nach Beendigung der Gasentwicklung wird die organische Phase nacheinander mit je 50 ml Wasser, 10%iger Salzsäure, Wasser sowie gesättigter NaHCO₃-Lösung gewaschen. Die organische Phase wird über Natriumsulfat getrocknet und nach Entfernen des Lösungsmittels wird der Rückstand über 40 g Kieselgel filtriert und mit 350 ml Pentan nachgewaschen. Nach erneutem Entfernen des Lösungsmittels wird das gewünschte Thioisocyanat erhalten welches mit bis zu 25% des entsprechenden Isocyanats verunreinigt sein kann.
**Allgemeine Arbeitsvorschrift (AAV 4):** Umsetzung von Neo-Menthylaminisocyanat bzw. Neo-Menthylaminisothiocyanat mit primären Aminen zu den korrespondierenden Harnstoffen bzw. Thioharnstoffen
   Zu einer Lösung des entsprechenden nach **AAV 2** oder **AAV 3** dargestellten Neo-Menthylisocyanates oder Neo-Menthylisothiocyanates in Heptan oder Toluol werden 2.5 Äquivalente des entsprechenden Amins zugegeben und anschließend wird die Reaktionslösung für sechs Stunden refluxiert. Nach Abkühlen auf Raumtemperatur wird mit Dichlormethan oder Toluol verdünnt und die organische Phase mit 10%iger Salzsäure, Wasser sowie gesättigter NaHCO₃-Lösung gewaschen. Dann wird die organische Phase über Natriumsulfat getrocknet, das Lösungsmittel am Rotationsverdampfer entfernt und das Rohprodukt durch Chromatographie an Kieselgel und/oder Kristallisation aufgereinigt.

### Synthesebeispiel 1: 1-((1S,2S,5R)-2-lsopropyl-5-methyl-cyclohexyl)-3-(3-methoxypropyl)-harnstoff (1) / 1-((1R,2R,5S)-2-Isopropyl-5-methyl-cyclohexyl)-3-(3-methoxypropyl)-harnstoff (2)

Analog zu **AAV 4** werden das nach **AAV 2** dargestellte racemische *Neo-*Menthylisocyanat mit 3-Methoxypropylamin zu den gewünschten Produkten umgesetzt.

### Analysendaten:

**¹H-NMR (400 MHz, CDCl₃):** 0.87 (d, *J* = 6.5 Hz, 3H); 0.87 - 0.95 (m, 1H); 0.89 (d, *J* = 6.6 Hz, 3H); 0.91 (d, *J* = 6.6 Hz, 3H); 0.97 - 1.06 (m, 1 H); 1.39 (m, 1 H); 1.48 (m, 1 H); 1.28-1.41 (m, 3H); 1.71 (m, 1H); 1.77 (m, 2H); 1.86 (m, 2H); 3.28 (dt, *J* = 5.6 Hz /*J* = 6.3 Hz, 2H); 3.34 (s, 3H); 3.48 (m, 2H); 4.02 (bs, 1 H); 4.43 (bd, J = 8.9 Hz, 1 H); 4.73 (bs, 1 H) ppm.

**¹³C-NMR (100 MHz, CDCl₃):** 20.8 (CH₃); 21.0 (CH₃); 22.3 (CH₃); 25.3 (CH₂); 26.7 (CH); 29.5 (CH); 29.8 (CH₂); 34.8 (CH₂); 38.9 (CH₂); 40.7 (CH₂); 46.7 (CH); 47.1 (CH); 58.7 (CH₃); 71.4 (CH₂); 157.8 (C=O) ppm.

**Massenspektrum (EI):** m/z (%) = 270 (M^{·+}, 11); 227 (14); 133 (16); 112 (25); 90 (26); 70 (100); 56 (14); 43 (16); 41 (16); 30 (19).

**Geschmacksprofil:** süß, salzig, metallisch, Umami.

### Synthesebeispiel 1a: 1-((1S,2S,5R)-2-Isopropyl-5-methyl-cyclohexyl)-3-(3-methoxypropyl)-harnstoff (1)

Analog zu **AAV 4** werden das nach **AAV 2** dargestellte (1S,2S,5R)-Neo-Menthylisocyanat mit 3-Methoxypropylamin zum gewünschten enantiomerenreinen Produkt umgesetzt.

### Analysendaten:

NMR und MS: vgl. Synthesebeispiel 1

**Geschmacksprofil:** salzig, haftfest, Umami, keine Beinoten

**Synthesebeispiel 2:** 1-(3-Hydroxy-propyl)-3-((1S,2S,5R)-2-isopropyl-5-methylcyclohexyl)-harnstoff (**3**) / 1-(3-Hydroxy-propyl)-3-((1R,2R,5S)-2-isopropyl-5-methylcyclohexyl)-harnstoff (**4**)

Analog zu **AAV 4** wird das nach **AAV 2** dargestellte racemische *Neo*-Menthylisocyanat mit 3-Aminopropan-1-ol zu den gewünschten Produkten umgesetzt.

### Analysendaten:

**¹H-NMR (400 MHz, CDCl₃):** 0.87 (d, *J* = 6.5 Hz, 3H); 0.89 (d, *J* = 6.7 Hz, 3H); 0.91 (d, *J* = 6.7Hz, 3H); 1.00 (m, 3H); 1.38 (m, 1H); 1.47 (m, 1H); 1.55 (m, 1H); 1.64 (m, 2H); 1.71 (m, 1H); 1.82 (m, 2H); 3.32 (m, 1H); 3.38 (m, 1H); 3.64 (qart, *J* = 5.8 Hz, 2H); 3.96 (bt, *J* = 6.3Hz, 1H); 4.07 (bd, *J* = 7.3Hz, 1H); 4.85 (bd, *J* = 9.1 Hz, 1H); 5.05 (bt, *J* = 6.2Hz, 1 H) ppm.

**¹³C-NMR (100 MHz, CDCl₃):** 20.8 (CH₃); 21.0 (CH₃); 22.3 (CH₃); 25.3 (CH₂); 26.7 (CH); 29.6 (CH); 33.3 (CH₂); 34.8 (CH₂); 36.4 (CH₂); 40.8 (CH₂); 46.6 (CH); 47.0 (CH); 58.9 (CH₂); 159.1 (C=O) ppm.

**Massenspektrum** (LC-MS: *Luna C18; 150x2 mm; 0.2 ml*/*min; H₂O*/*CH₃CN 95*/*5* → *0*/*100):* m/z (%) = 514 (18); 513 (100); 413 (4); 412 (4); 259 (3) 258 (2).

**Geschmacksprofil:** fischig, salzig, schwach umami.

### Synthesebeispiel 3: 1-((1S,2S,5R)-2-lsopropyl-5-methyl-cyclohexyl)-3-(2-methoxy-1-methyl-ethyl)-harnstoff (5) / 1-((1R,2R,5S)-2-Isopropyl-5-methyl-cyclohexyl)-3-(2-methoxy-1-methyl-ethyl)-harnstoff (6)

Analog zu **AAV 4** wird das nach **AAV 2** dargestellte racemische *Neo*-Menthylisocyanat mit 2-Methoxy-1-methyl-ethylamin zu den gewünschten Produkten umgesetzt.

### Analysendaten (2 Diastereomere):

**¹³C-NMR (100 MHz, CDCl₃):** 18.20, 18.29 (CH₃); 20.91, 20.94 (CH₃); 21.0 (CH₃); 22.37, 22.40 (CH₃); 25.25, 25.32 (CH₂); 26.66, 26.68 (CH); 29.34, 29.52 (CH); 35.0 (CH₂); 40.79, 40.94 (CH₂); 46.31, 46.54 (CH); 47.72, 46.76 (CH); 46.8 (CH); 58.96, 59.00 (CH₃); 77.56, 77.90 (CH₂); 158.07, 158.28 (C=O) ppm.

**Massenspektrum (EI):** m/z (%) = 270 (M^{·+}, 14); 225 (13); 112 (8); 90 (9); 70 (22); 69 (9); 57 (8); 55 (7); 44 (100); 43 (28).

**Geschmacksprofil:** haftfest, salzig, umami.

### Synthesebeispiel 4: 3-[3-((1S,2S,5R)-2-Isopropyl-5-methyl-cyclohexyl)-ureido]-buttersäureethylester (7) / 3-[3-((1R,2R,5S)-2-Isopropyl-5-methyl-cyclohexyl)-ureido]-buttersäureethylester (8)

Analog zu **AAV 4** wird das nach **AAV 2** dargestellte racemische *Neo*-Menthylisocyanat mit 3-Aminobuttersäureethylester zu den gewünschten Produkten umgesetzt.

### Analysendaten (2 Diastereomere):

**¹³C-NMR (100 MHz, CDCl₃):** 14.2 (CH₃); 20.82, 20.83 (CH₃); 20.85, 20.93 (CH₃); 21.0 (CH₃); 22.3 (CH₃); 25.26, 25.28 (CH₂); 26.65, 26.69 (CH); 29.45, 29.52 (CH); 34.84, 34.86 (CH₂); 40.73, 40.74 (CH₂); 40.95, 41.05 (CH₂); 43.16, 43.27 (CH); 46.67, 46.68 (CH); 47.0 (CH); 60.53, 60.55 (CH₂); 156.96, 157.03 (C=O); 172.32, 172.35 (C=O) ppm.

**Massenspektrum (EI):** m/z (%) = 312 (M^{·+}, 9); 267 (10); 132 (44); 130 (11); 112 (22); 70 (100); 55 (11); 44 (38); 43 (17); 41 (13).

**Geschmacksprofil:** bitter, salzig, metallisch, Umami.

### Synthesebeispiel 5: (3-Methoxy-propyl)-carbaminsäure (1S,2S,5R)-2-isopropyl-5-methyl-cyclohexylester (9) und (3-Methoxy-propyl)-carbaminsäure (1R,2R,5S)-2-isopropyl-5-methyl-cyclohexylester (10)

Analog zu **AAV 1** wird racemisches *Neo*-Menthol mit 3-Methoxypropylamin zu den gewünschten Produkten umgesetzt.

### Analysendaten:

**¹H-NMR (400 MHz, CDCl₃):** 0.84 - 1.07 (m, 3H); 0.85 (d, *J* = 6.4 Hz, 3H); 0.89 (d, *J* = 6.6Hz, 3H); 0.90 (d, *J* = 6.5 Hz, 3H); 1.25 (m, 1H); 1.46 (m, 1H); 1.56 - 1.81 (m, 5H); 1.97 (bd, *J* = 13.6 Hz, 1 H); 3.28 (m, 2H); 3.34 (s, 3H); 3.43 (t, *J* = 6.0 Hz, 2H); 4.90 (bs, 1H); 5.07 (bs, 1 H) ppm.

**¹³C-NMR (100 MHz, CDCl₃):** 20.87 (CH₃); 20.92 (CH₃); 22.3 (CH₃); 25.0 (CH₂); 26.5 (CH); 29.3 (CH); 29.7 (CH₂); 34.9 (CH₂); 38.9 (CH₂); 39.6 (CH₂); 46.9 (CH); 58.7 (CH₃); 71.1 (CH₂); 71.3 (CH); 156.5 (C=O) ppm.

**Massenspektrum (EI)**: m/z (%) = 271 (M^{·+}, 1); 133 (51); 95 (97); 90 (90); 83 (100); 81 (65); 71 (58); 69 (54); 55 (61); 41 (53).

**Geschmacksprofil:** süß, salzig, umami, leicht bitter.

### Synthesebeispiel 5A: (3-Methoxy-propyl)-carbaminsäure (1S,2S,5R)-2-isopropyl-5-methyl-cyclohexylester (9)

Analog zu **AAV 1** wird (1S,2S,5R)-*Neo*-Menthol mit 3-Methoxypropylamin zu den gewünschten Produkten umgesetzt.

### Analysendaten:

NMR und MS: vgl. Synthesebeispiel 5

**Geschmacksprofil:** Umami, salzig, metallisch, bitter

**Synthesebeispiel 5B:** (3-Methoxy-propyl)-carbaminsäure (1R,2R,5S)-2-isopropyl-5-methyl-cyclohexylester (**10**)

Analog zu **AAV 1** wird (1R,2R,5S)-*Neo*-Menthol mit 3-Methoxypropylamin zu den gewünschten Produkten umgesetzt.

### Analysendaten:

NMR und MS: vgl. Synthesebeispiel 5

**Geschmacksprofil:** Umami, salzig, metallisch, nicht bitter

### Synthesebeispiel 6: 4-((1S,2S,5R)-2-Isopropyl-5-methyl-cyclohexyloxycarbonylamino)-buttersäureethylester (11) / 4-((1R,2R,5S)-2-Isopropyl-5-methyl-cyclohexyloxycarbonyl-amino)-buttersäureethylester (12)

Analog zu **AAV 1** wird racemisches Neo-Menthol mit 3-Methoxypropylamin zu den gewünschten Produkten umgesetzt.

### Analysendaten:

**¹H-NMR (400 MHz, CDCl₃):** 0.84 - 1.07 (m, 3H); 0.85 (d, *J* = 6.6 Hz, 3H); 0.88 (d, *J* = 6.6 Hz, 3H); 0.89 (d, *J* = 6.6 Hz, 3H); 1.23 - 1.33 (m, 1H); 1.26 (t, *J* = 7.2 Hz, 3H); 1.45 (m, 1H); 1.50-1.77 (m, 3H); 1.84 (quint, *J* = 7.1 Hz, 2H); 1.97 (m, 1H); 2.35 (t, *J* = 7.3 Hz, 2H); 3.22 (m, 2H); 4.13 (quart, *J* = 7.2 Hz, 2H); 4.78 (bs, 1H); 5.07 (bs, 1H) ppm.

**¹³C-NMR (100 MHz, CDCl₃):** 14.2 (CH₃); 20.85 (CH₃); 20.92 (CH₃); 22.2 (CH₃); 25.0 (CH₂); 25.3 (CH₂); 26.5 (CH); 29.3 (CH); 31.6 (CH₂); 34.9 (CH₂); 40.3 (CH₂); 46.9 (CH); 60.5 (CH₂); 71.4 (CH); 156.5 (C=O); 173.3 (C=O) ppm.

**Massenspektrum (EI):** m/z (%) = 175 (76); 130 (84); 112 (56); 95 (90); 86 (59); 83 (100); 81 (62); 69 (66); 55 (59); 44 (74).

**Geschmacksprofil:** bitter, salzig, umami.

### Synthesebeispiel 6A: 4-((1S,2S,5R)-2-lsopropyl-5-methyl-cyclohexyloxycarbonylamino)-buttersäureethylester (11)

Analog zu **AAV 1** wird (1*S*,2*S*,5*R*)-*N*eo-Menthol mit 3-Methoxypropylamin zu den gewünschten Produkten umgesetzt.

### Analysendaten:

NMR und MS: vgl. Synthesebeispiel 6

**Geschmacksprofil:** salzig, Umami, relativ schwach im Vergleich zum Racemat

### Synthesebeispiel 6B: 4-((1R,2R,5S)-2-lsopropyl-5-methyl-cyclohexyloxycarbonylamino)-buttersäureethylester (12)

Analog zu **AAV 1** wird (1*R*,2*R*,5*S*)-*Neo*-Menthol mit 3-Methoxypropylamin zu den gewünschten Produkten umgesetzt.

### Analysendaten:

NMR und MS: vgl. Synthesebeispiel 6

**Geschmacksprofil:** salzig, Umami, verzögert und schwächer im Vergleich zum Racemat

Die folgenden Strukturen sind weitere Beispiele aus dieser Substanzklasse, die entsprechend **AAV 1 - AAV 4** - ausgehend von racemischem Neo-Menthol bzw. Neo-Menthylamin - dargestellt und verkostet wurden.

| **Struktur** | **MS-Daten** | **Geschmacksprofil** |
|---|---|---|
| 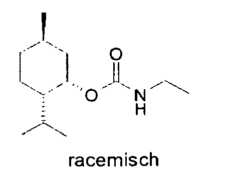 | **m/z (%)** = 227 (M˙⁺, 0.2); 123 (26); 113 (24); 95 (100); 83 (37); 81 (56); 71 (31); 69 (29); 55 (34); 41 (32). | kühlend, Umami, Mentholnote |
| 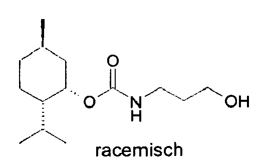 | **m/z (%)** = 257 (M^{·+}, 0.1); 138 (56); 120 (49); 95 (100); 83 (72); 81 (69); 76 (84); 69 (42); 55 (59); 41 (46). | seifig, Gemüse, Spargel, Kartoffel, Fülle, leicht Umami |
| 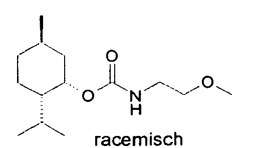 | **m/z (%)** = 257 (M^{·+}, 0.4); 119 (41); 95 (46); 87 (31); 83 (100); 81 (38); 69 (37); 57 (31); 55 (43); 30 (30). | süß, Umami |
| 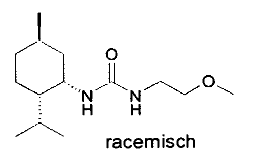 | m/z (%) = 256 (M^{·+}, 13); 171 (15); 112 (16); 76 (36); 70 (100); 55 (16); 44 (21); 43 (36); 41 (15); 30 (74). | fischig, salzig, Umami |
| 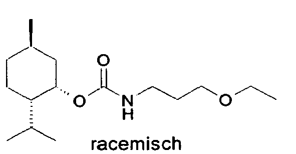 | **m/z (%)** = 285 (M^{·+}, 0.4); 104 (65); 103 (43); 102 (82); 101 (49); 95 (74); 83 (100); 81 (50); 57 (53); 55 (49). | ranzig, muffig, bitter, Umami |
| 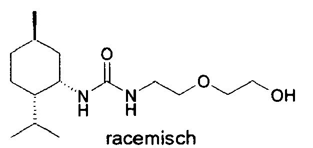 | m/z (%) (LC-MS) = 573 (100); 444 (4); 445 (5); 290 (12); 289 (19). | fettig, ölig (Fisch), Umami |
| 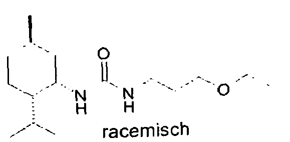 | **m/z (%)** = 284 (M^{·+}, 12); 112 (17); 104 (22); 74 (12); 70 (100); 56 (15); 44 (11); 43 (14); 41 (16); 30 (33). | fettig, Talg, trockenstaubig, haftfest, Umami |
| 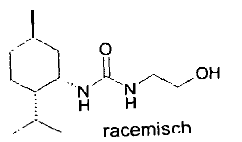 | **m/z (%)** (LC-MS) = 486 (2); 485 (100); 400 (2); 399 (3); 398 (2); 397 (2); 245 (2). | Alge, Röstnote, Umami, süß |
| 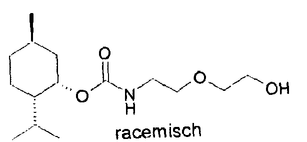 | **m/z (%)** = 119 (33); 95 (38); 88 (46); 83 (100); 81 (33); 69 (38); 57 (29); 55 (39); 44 (33); 43 (34). | fettig, Talg, ölig, salzig, metallisch |
| 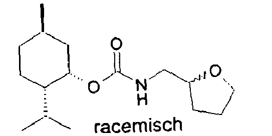 | **m/z (%)** = 139 (21); 102 (16); 95 (12); 83 (40); 81 (13); 71 (100); 57 (14); 55 (19); 43 (20); 41 (14). | bitter, haftfest, Umami |
| 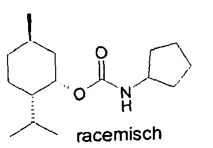 | **m/z (%)** = 267 (M^{·+}, 0.3); 138 (63); 130 (100); 95 (59); 83 (85); 81 (46); 69 (50); 56 (30); 55 (45); 41 (33). | bitter, salzig |
| 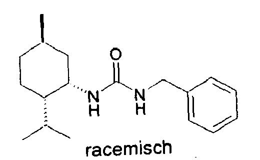 | **m/z (%)** = 288 (M^{·+}, 34); 245 (15); 151 (47); 112 (41); 106 (19); 91 (47); 70 (100); 43 (18); 41 (15); 30 (14). | haftfest, Umami |
| 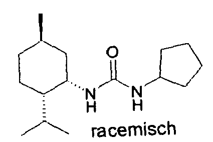 | **m/z (%)** = 266 (M^{·+}, 13); 223 (12); 129 (47); 112 (31); 84 (11); 70 (100); 56 (28); 55 (13); 43 (16); 41 (21). | bitter, adstringierend, haftfest, salzig, metallisch |
| 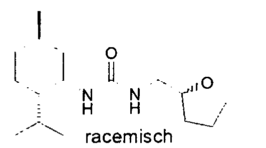 | **m/z (%)** = 282 (M^{·+}, 11); 239 (39); 169 (56); 112 (46); 111 (31); 102 (41); 71 (47); 70 (100); 69 (47); 30 (48). | salzig, schwach Umami |
| 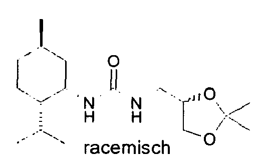 | **m/z (%)** = 312 (M^{·+}, 36); 297 (47); 254 (97); 211 (47); 169 (100); 117 (63); 112 (42); 73 (80); 70 (72); 30 (47). | süß, Umami |
| 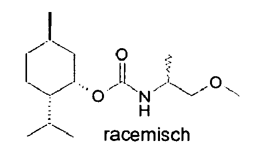 | **m/z (%)** = 226 (37); 139 (42); 95 (35); 83 (97); 69 (44); 57 (36); 55 (42); 45 (30); 44 (100); 41 (34). | bitter, metallisch, Umami |
| 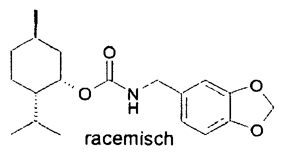 | **m/z (%)** = 333 (M^{·+}, 10); 196 (9); 195 (78); 194 (100); 150 (16); 135 (26); 83 (20); 69 (12); 55 (17); 41 (9). | bitter, Umami |
| 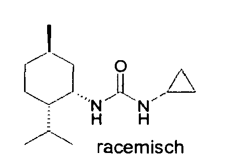 | **m/z (%)** = 238 (M^{·+}, 2); 83 (4); 69 (4); 58 (5); 57 (100); 56 (20); 55 (8); 43 (5); 41 (9); 32 (6). | bitter, haftfest, salzig, Umami |
| 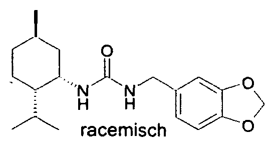 | **m/z (%)** = 332 (M^{·+}, 46); 194 (26); 150 (32); 134 (95); 112 (30); 70 (100); 55 (19); 43 (14); 41 (16); 30 (18). | salzig, schwach Umami |
| 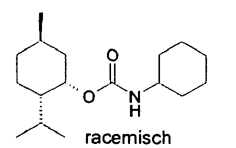 | **m/z (%)** = 281 (M^{·+}, 0.4); 144 (100); 138 (66); 95 (57); 83 (100); 81 (46); 69 (42); 56 (37); 55 (60); 41 (38). | schwach süß und schwach Umami |
| 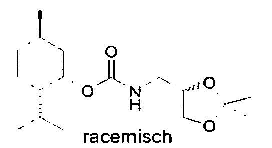 | **m/z (%)** = 160 (24); 139 (11); 138 (18); 118 (21); 101 (100); 83 (27); 73 (15); 59 (11); 55 (15); 43 (29). | süß, bitter, Karamell, salzig |
| 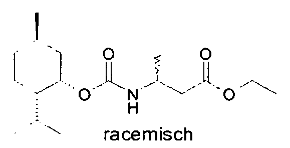 | **m/z (%)** = 313 (M^{·+}, 0.3); 132 (95); 130 (84); 116 (62); 95 (79); 83 (90); 81 (58); 69 (52); 55 (54); 44 (100). | bitter, trockenstaubig, Tingling, leicht Umami |
| 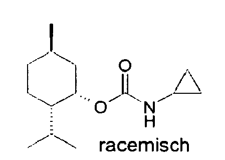 | **m/z (%)** = 239 (M^{·+}, 0.5); 139 (13); 97 (18); 83 (100); 81 (18); 69 (41); 57 (72); 55 (43); 43 (17); 41 (24). | bitter, salzig, Umami |
| 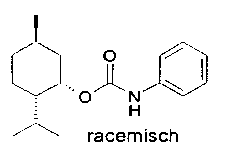 | **m/z (%)** = 275 (M^{·+}, 15); 138 (23); 93 (82); 83 (100); 81 (21); 69 (44); 57 (28); 55 (46); 43 (21); 41 (26). | bitter, trocken, staubig, Umami, stumpf |
| 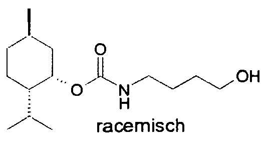 | **m/z (%)** = 138 (49); 95 (92); 90 (38); 83 (100); 81 (57); 71 (58); 69 (50); 55 (89); 43 (48); 41 (44). | leicht bitter, salzig, Umami |
| 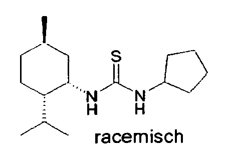 | **m/z (%)** = 282 (M^{·+}, 16); 145 (100); 95 (26); 83 (18); 77 (19); 69 (28); 55 (27); 43 (18); 41 (11); 28 (20). | bitter, haftfest, Umami, metallisch |
| 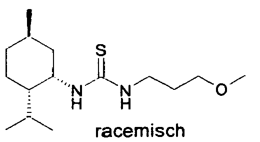 | **m/z (%)** = 286 (M^{·+}, 31); 149 (100); 117 (36); 112 (29); 95 (28); 90 (30); 83 (26); 55 (36); 45 (32); 41 (38). | sehr bitter, Umami |
| 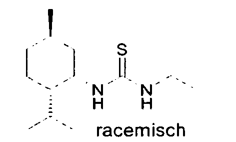 | **m/z (%)** = 242 (M^{·+}, 17); 193 (19); 112 (15); 105 (100); 95 (18); 69 (12); 55 (19); 44 (12); 43 (15); 41 (18). | bitter, trockenstaubig, Umami |
| 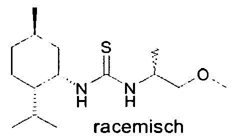 | **m/z (%)** = 286 (M^{·+}, 11); 254 (63); 149 (43); 139 (54); 90 (45); 83 (100); 69 (51); 58 (63); 55 (59); 41 (59). | bitter, leicht Umami |
| 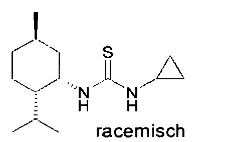 | **m/z (%)** = 254 (M^{·+}, 64); 117 (71); 83 (40); 69 (29); 58 (100); 57 (77); 56 (45); 55 (43); 43 (31); 41 (67). | bitter, Fülle, Umami |

### Beispiele

Die Beispiele dienen zur Verdeutlichung der Erfindung, ohne diese damit einzuschränken. Sofern nicht anders angegeben, beziehen sich alle Angaben auf das Gewicht.

### Anwendungsbeispiel 1: Sprühgetrocknete Zusammensetzungen

| **1.1** | **Bestandteil** | **Anteil** |
|---|---|---|
| | 4-((1S,2S,5R)-2-Isopropyl-5-methyl-cyclohexyloxycarbonylamino)-buttersäureethylester (**11**) | 3 g |
| | Maltodextrin | 97 g |
| | | |

| **1.2** | **Bestandteil** | **Anteil** |
|---|---|---|
| | 1:1 Mischung aus 3-[3-((1S,2S,5R)-2-Isopropyl-5-methyl-cyclohexyl)-ureido]-buttersäureethylester (**7**) und 3-[3-((1R,2R,5S)-2-Isopropyl-5-methyl-cyclohexyl)-ureido]-buttersäureethylester (**8**) Synthesebeispiel 4 | 4 g |
| | Maltodextrin | 96 g |

Die Bestandteile werden in einer Mischung aus Ethanol und demineralisiertem Wasser gelöst und anschließend sprühgetrocknet.

### Anwendungsbeispiel 2: Aromakomposition, nicht erfindungsgemäß

| **Inhaltsstoff** | **Anteil** |
|---|---|
| 10 Gew.-% Pellitorin in 1,2-Propylenglycol/Diethylmalonat | 0,25 g |
| Hesperetin | 2,50 g |
| Phloretin | 1,50 g |
| Propylenglycol | 95,75 g |

Die Aromakomposition wurde in nachfolgend beschriebenen Anwendungsbeispielen eingesetzt.

### Anwendungsbeispiel 3: Würzmittel

| **Teil** | **Bestandteil** | **Anteil** |
|---|---|---|
| A | 1:1 Mischung von Verbindungen der Formeln **(7)** + **(8)** | 0,02 g |
| | Natriumchlorid | 15 g |
| B | Senfsamenmehl | 5g |
| | Senfaroma | 0,1g |

Teil A wurde eingewogen. Es wurden 290 ml Wasser vorgelegt und unter Rühren Teil A zugegeben und gelöst. Die Lösung wird mit Wasser auf 1,84 kg verdünnt (pH 6,0) und anschließend gefriergetrocknet (Eutektischer Punkt: -15°C; Arbeitsvakuum: 0,52 mbar; Stellflächentemperatur: -5°C bis +25°C). Das Produkt wird mit Senfsamenmehl und dem Senfaroma aus Teil B gemischt und zu einem Würzmittel konfektioniert.

### Anwendungsbeispiel 4: Reaktionsaroma

| **Inhaltsstoff** | **Einsatz [g]** |
|---|---|
| L-Alanin | 41,0 |
| L-Asparaginsäure | 123,0 |
| Bernsteinsäure | 4,7 |
| Calciumchlorid Dihydrat | 7,0 |
| L-Cystein•HCl Monohydrat | 11,0 |
| Dikaliumphosphat | 6,0 |
| Fructose gemahlen | 1,0 |
| L-Isoleucin | 1,6 |
| Kaliumchlorid | 228,0 |
| L-Leucin | 1,6 |
| L-Lysin•HCl | 3,6 |
| Magnesiumchlorid Hexahydrat | 19,0 |
| Maltodextrin | 49,0 |
| L-Phenylalanin | 2,0 |
| L-Prolin | 74,0 |
| L-Serin | 6,5 |
| L-Threonin | 3,0 |
| L-Valin | 9,0 |
| Wasser | 404,0 |
| 1:1 Mischung der Verbindungen der Formeln **(7)** + **(8)**, 20 Gew.-% in EtOH | 5,0 |

Alle Komponenten werden bei 40°C gemischt und anschließend bei 85°C für 10 Minuten erhitzt (Rückflussreaktion). Nach dem Abkühlen auf 40°C wird mit Kalilauge auf pH 5 eingestellt. Dieses Umami-Reaktionsaroma kann anstelle der reinen Verbindung ((7)+(8)) in die Bouillon - Zubereitungen C bzw. D des Anwendungsbeispiels 9 eingearbeitet werden, wobei in Zubereitung C 5 g und in Zubereitung D 13 g des Umami-Reaktionsaromas verwendet wurden.

### Anwendungsbeispiel 5: Instantsuppe, Typ Lauch-Creme

A = Vergleichszubereitung
B, C, D = erfindungsgemäße Zubereitungen (Natriumglutamatfrei)
E = erfindungsgemäße Zubereitungen (Salzreduziert und Natriumglutamatfrei)

| **Bestandteil** | **A** | **B** | **C** | **D** | **E** |
|---|---|---|---|---|---|
| Kartoffelstärke | 20,0 g | 20,0 g | 20,0 g | 20,0 g | 20,0 g |
| Fettpulver | 25,0 g | 25,0 g | 25,0 g | 25,0 g | 25,0 g |
| Lactose | 20,0 g | 20,0 g | 20,0 g | 20,0 g | 20,0 g |
| Maltodextrin | 11,730 g | 14,729 g | 14,725 g | 14,710 g | 15,723 g |
| Kochsalz | 8,0 g | 8,0 g | 8,0 g | 8,0 g | 7,0 g |
| Natriumglutamat | 3,0 g | - | - | - | - |
| Spinatpulver | 2,0 g | 2,0 g | 2,0 g | 2,0 g | 2,0 g |
| Grünes Lauchpulver | 2,0 g | 2,0 g | 2,0 g | 2,0 g | 2,0 g |
| Citronensäure, als Pulver | 0,3 g | 0,3 g | 0,3 g | 0,3 g | 0,3 g |
| Gehärtetes Pflanzenfett | 3,0 g | 3,0 g | 3,0 g | 3,0 g | 3,0 g |
| Gefriergetrockneter Lauch | 1,0 g | 1,0 g | 1,0 g | 1,0 g | 1,0 g |
| Huhnaroma | 1,0 g | 1,0 g | 1,0 g | 1,0 g | 1,0 g |
| Würzmischung Typ "grüner Lauch", Pulver | 2,0 g | 2,0 g | 2,0 g | 2,0 g | 2,0 g |
| Würzmischung, Typ "gekochte Zwiebel" | 0,6 g | 0,6 g | 0,6 g | 0,6 g | 0,6 g |
| Hefe-Würzmischung, Typ "Gemüsebrühe", Pulver | 0,3 g | 0,3 g | 0,3 g | 0,3 g | 0,3 g |
| Curcuma-Extrakt | 0,07 g | 0,07 g | 0,07 g | 0,07 g | 0,07 g |
| 1:1 Mischung von Verbindungen der Formeln **(1)** + **(2)** | - | 0,001 g | 0,005 g | 0,020 g | 0,007 g |

5 g der jeweiligen Pulvermischung wurden mit je 100 ml heißem Wasser aufgegossen, um eine verzehrfertige Suppe zu erhalten. Bei der Verkostung durch ein Panel ausgebildeter Testpersonen wurden die Vergleichszubereitung A und die erfindungsgemäße Zubereitung E hinsichtlich ihres Umami-Charakters sowie ihrer Salzigkeit als gleich bewertet. Bei der erfindungsgemäßen Zubereitung B wurde der Umami-Geschmack (und Mundfülle) als wahrnehmbar, jedoch schwächer gegenüber den Zubereitungen A und E beschrieben. Die erfindungsgemäße Zubereitung C wird hinsichtlich ihres Umami-Charakters gleich den Zubereitungen A und E bewertet, allerdings wird hier auch eine gesteigerte Salzigkeit beschrieben. Die erfindungsgemäße Zubereitung D wurde hinsichtlich Umami-Geschmack (und Mundfülle) als sehr ausgeprägt und deutlich stärker als die Zubereitungen A und E bewertet, was durch eine einhergehende Steigerung der Salzigkeit weiter unterstrichen wird.

### Anwendungsbeispiel 6: Instantsuppe, Typ Hühnersuppe mit Nudeln

A = Vergleichszubereitung
B, C, D = erfindungsgemäße Zubereitungen (Natriumglutamat-frei)
E = erfindungsgemäße Zubereitungen (Salz-reduziert und Natriumglutamat-frei)

| **Bestandteil** | **A** | **B** | **C** | **D** | **E** |
|---|---|---|---|---|---|
| Stärke | 16,0 g | 16,0 g | 16,0 g | 16,0 g | 16,0 g |
| Kochsalz | 7 g | 7 g | 7 g | 7 g | 5 g |
| Saccharose, raffiniert | 3,2 g | 3,2 g | 3,2 g | 3,2 g | 3,2 g |
| Natriumglutamat | 3,2 g | - | - | - | - |
| Natriuminosinat / Natriumguanylat im Verhältnis 1:1 | 0,8 g | 0,8 g | 0,8 g | 0,8 g | 0,8 g |
| Säurehydrolysiertes Pflanzenprotein | 8,0 g | 8,0 g | 8,0 g | 8,0 g | 8,0 g |
| Fettpulver | 2,0 g | 2,0 g | 2,0 g | 2,0 g | 2,0 g |
| Gemüsefett, sprühgetrocknet | 1,0 g | 1,0 g | 1,0 g | 1,0 g | 1,0 g |
| Gefriergetrocknetes Hühnerfleisch, in Stückchen | 2,0 g | 2,0 g | 2,0 g | 2,0 g | 2,0 g |
| Suppen-Nudeln | 32,0 g | 32,0 g | 32,0 g | 32,0 g | 32,0 g |
| Maltodextrin | 12,160 g | 15,359 g | 14,155 g | 14,140 g | 15,152 g |
| Chinesisches Gemüse, gefriergetrocknet | 4,6 g | 4,6 g | 4,6 g | 4,6 g | 4,6 g |
| Huhnaroma | 8,0 g | 8,0 g | 8,0 g | 8,0 g | 8,0 g |
| Lebensmittelfarbstoff Riboflavin | 0,04 g | 0,04 g | 0,04 g | 0,04 g | 0,04 g |
| 1:1 Mischung von Verbindungen der Formeln **(7)** + **(8)** | - | 0,001 g | 0,005 g | 0,02 g | 0,008 g |
| Aromenkomposition nach Anwendungsbeispiel 2.1 | - | - | 1,2 g | 1,2 g | 1,2 g |

4,6 g der jeweiligen Pulvermischung wurden 10 Minuten in je 100 ml Wasser gekocht, um eine verzehrfertige Suppe zu erhalten. Bei der Verkostung durch ein Panel ausgebildeter Testpersonen wurden die Vergleichszubereitung A und die erfindungsgemäße Zubereitung E hinsichtlich ihres Umami-Charakters sowie ihrer Salzigkeit als gleich bewertet. Bei der erfindungsgemäßen Zubereitung B wurde der Umami-Geschmack (und Mundfülle) als wahrnehmbar, jedoch schwächer gegenüber den Zubereitungen A und E beschrieben. Die erfindungsgemäße Zubereitung C wird hinsichtlich ihres Umami-Charakters gleich den Zubereitungen A und E bewertet, allerdings wird hier auch eine gesteigerte Salzigkeit beschrieben. Die erfindungsgemäße Zubereitung D wurde hinsichtlich Umami-Geschmack (und Mundfülle) als sehr ausgeprägt und deutlich stärker als die Zubereitungen A und E bewertet, was durch eine einhergehende Steigerung der Salzigkeit weiter unterstrichen wird.

### Anwendungsbeispiel 7: Würzmischung, Typ "Pfeffer":

A = Vergleichszubereitung
B, C, D = erfindungsgemäße Zubereitungen (Natriumglutamat-frei)
E = erfindungsgemäße Zubereitungen (Salz-reduziert und Natriumglutamat-frei)

| **Bestandteil** | **A** | **B** | **C** | **D** | **E** |
|---|---|---|---|---|---|
| Milchprotein | 0,8 g | 0,8 g | 0,8 g | 0,8 g | 0,8 g |
| Johannisbrotkernmehl | 2,0 g | 2,0 g | 2,0 g | 2,0 g | 2,0 g |
| Maisstärke | 22,0 g | 27,998 g | 27,985 g | 27,940 g | 29,970 g |
| Kochsalz | 14,0 g | 14,0 g | 14,0 g | 14,0 g | 12,0 g |
| Paprikapulver | 13,0 g | 13,0 g | 13,0 g | 13,0 g | 13,0 g |
| Tomatenpulver | 13,0 g | 13,0 g | 13,0 g | 13,0 g | 13,0 g |
| Saccharose | 4,0 g | 4,0 g | 4,0 g | 4,0 g | 4,0 g |
| Knoblauchpulver | 0,5 g | 0,5 g | 0,5 g | 0,5 g | 0,5 g |
| Gehärtetes Pflanzenfett | 8,0 g | 8,0 g | 8,0 g | 8,0 g | 8,0 g |
| Fettpulver | 11,0 g | 11,0 g | 11,0 g | 11,0 g | 11,0 g |
| Natriumglutamat | 6,0 g | - | - | - | - |
| Lebensmittelfarbstoff Rote Bete und Paprika | 2,0 g | 2,0 g | 2,0 g | 2,0 g | 2,0 g |
| Aroma Typ "Pfeffer" | 2,0 g | 2,0 g | 2,0 g | 2,0 g | 2,0 g |
| Aroma Typ "Pizza" | 1,2 g | 1,2 g | 1,2 g | 1,2 g | 1,2 g |
| Aroma Typ "Tomate" | 0,4 g | 0,4 g | 0,4 g | 0,4 g | 0,4 g |
| Extrakt aus schwarzem Pfeffer | 0,1 g | 0,1 g | 0,1 g | 0,1 g | 0,1 g |
| Verbindung der Formel **(1)** | - | 0,002 g | 0,015 g | 0,060 g | 0,03 g |

Jeweils 100 g Nackensteak vom Schwein wurde mit jeweils 1,7 g der Zubereitungen A, B, C und D gleichmäßig bestreut und gebraten. Bei der Verkostung durch ein Panel ausgebildeter Testpersonen wurden die Vergleichszubereitung A und die erfindungsgemäße Zubereitung E hinsichtlich ihres Umami-Charakters sowie ihrer Salzigkeit als gleich bewertet. Bei der erfindungsgemäßen Zubereitung B wurde der Umami-Geschmack (und Mundfülle) als wahrnehmbar, jedoch schwächer gegenüber den Zubereitungen A und E beschrieben. Die erfindungsgemäße Zubereitung C wird hinsichtlich ihres Umami-Charakters gleich den Zubereitungen A und E bewertet, allerdings wird hier auch eine gesteigerte Salzigkeit beschrieben. Die erfindungsgemäße Zubereitung D wurde hinsichtlich Umami-Geschmack (und Mundfülle) als sehr ausgeprägt und deutlich stärker als die Zubereitungen A und E bewertet, was durch eine einhergehende Steigerung der Salzigkeit weiter unterstrichen wird.

### Anwendungsbeispiel 8: Tomatenketchup

A = Vergleichszubereitung
B = Vergleichszubereitung (Salz- und Zucker-reduziert, Natriumglutamat-frei)
C = erfindungsgemäße Zubereitungen (Natriumglutamatfrei, Zucker-reduziert)
D = erfindungsgemäße Zubereitungen (Salz- und Zucker-reduziert, Natriumglutamatfrei)

| **Bestandteil** | **A** | **B** | **C** | **D** |
|---|---|---|---|---|
| Natriumglutamat | 0,40 g | - | - | - |
| Kochsalz | 2 g | 1 g | 2 g | 1 g |
| Stärke, Farinex WM 55 | 1 g | 1 g | 1 g | 1 g |
| Sucrose | 12 g | 9,2 g | 9,2 g | 9,2 g |
| Tomaten-Konzentrat 2-fach | 40 g | 40 g | 40 g | 40 g |
| Glucosesirup 80 Brix | 18 g | 18 g | 18 g | 18 g |
| Branntweinessig 10% | 7 g | 7 g | 7 g | 7 g |
| Wasser | 19,60 g | 23,80 g | 22,30 g | 23.25 g |
| Aromenkomposition nach Anwendungsbeispiel **2.1** | - | - | 0,4 g | 0,4 g |
| 1 %ige Lösung einer 1:1 Mischung der Verbindungen der Formeln **(11)** + **(12)** in Propylenglycol | - | - | 0,10 g | 0,15 g |

Die Inhaltsstoffe werden in der angegebenen Reihenfolge gemischt und das fertige Ketchup mit Hilfe eines Rührwerkes homogenisiert, in Flaschen abgefüllt und sterilisiert.

### Anwendungsbeispiel 9: Bouillon

A = Vergleichszubereitung
B = Vergleichszubereitung (Natriumglutamat-reduziert)
C = erfindungsgemäße Zubereitungen
D = erfindungsgemäße Zubereitungen (Natriumglutamat-frei)
E = erfindungsgemäße Zubereitungen (Salz-reduziert und Natriumglutamat-frei)

| **Bestandteil** | **A** | **B** | **C** | **D** | **E** |
|---|---|---|---|---|---|
| Fettpulver | 8,77 g | 8,77 g | 8,77 g | 8,77 g | 8,77 g |
| Natriumglutamat | 8,77 g | 5 g | 5 g | - | - |
| Hefeextrakt Pulver | 12,28 g | 12,28 g | 12,28 g | 12,28 g | 12,28 g |
| Kochsalz | 29,83 g | 29,83 g | 29,83 g | 29,83 g | 26,83 g |
| Maltodextrin | 37,28 g | 41,050 g | 41,047 g | 46,020 g | 49,020 g |
| Natürlicher Gemüseextrakt | 3,07 g | 3,07 g | 3,07 g | 3,07 g | 3,07 g |
| 1:1 Mischung von Verbindungen der Formeln **(7)** + **(8)** | - | - | 0,003 g | 0,030 g | 0,030 g |

15 g der jeweiligen Pulvermischung wurden mit je 1000 ml heißem Wasser aufgegossen. Bei der Verkostung durch ein Panel ausgebildeter Testpersonen wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet. Bei beiden wurde eine verstärkte Salzigkeit im Vergleich zur Zubereitung A festgestellt. Die Natriumglutamat-freie, Salz-reduzierte Zubereitung E wurde hinsichtlich ihres Umami-Geschmacks (und Mundfülle) und ihrer Salzigkeit als sehr ähnlich zur Vergleichszubereitung A bezeichnet.

### Anwendungsbeispiel 10: Würzmischung für Kartoffelchips

A = Vergleichszubereitung
B = Vergleichszubereitung (Natriumglutamat-reduziert)
C = erfindungsgemäße Zubereitungen (Natriumglutamat-reduziert)
D = erfindungsgemäße Zubereitungen (Natriumglutamat-frei)
E = erfindungsgemäße Zubereitungen (Natriumglutamat- und Salz-reduziert)

| **Bestandteil** | **A** | **B** | **C** | **D** | **E** |
|---|---|---|---|---|---|
| Natriumglutamat | 3,50 g | 2,00 g | 2,00 g | - | 1,00g |
| Käsepulver | 10,00 g | 10,00 g | 10,00 g | 10,00 g | 10,00 g |
| Knoblauchpulver | 2,00 g | 2,00 g | 2,00 g | 2,00 g | 2,00 g |
| Molkenpulver | 38,86 g | 40,36 g | 40,06 g | 41,91 g | 44,76 g |
| Würzextraktöl | 0,20 g | 0,20 g | 0,20 g | 0,20 g | 0,20 g |
| Paprikapulver | 9,80 g | 9,80 g | 9,80 g | 9,80 g | 9,80 g |
| Kochsalz | 21,00 g | 21,00 g | 21,00 g | 21,00 g | 17,00 g |
| Tomatenpulver | 9,00 g | 9,00 g | 9,00 g | 9,00 g | 9,00 g |
| Trockenaroma | 2,50 g | 2,50 g | 2,50 g | 2,50 g | 2,50 g |
| Siliciumdioxid | 0,02 g | 0,02 g | 0,02 g | 0,02 g | 0,02 g |
| Pflanzenöl | 0,02 g | 0,02 g | 0,02 g | 0,02 g | 0,02 g |
| Zwiebelpulver | 3,00 g | 3,00 g | 3,00 g | 3,00 g | 3,00 g |
| Sahne Aromakonzentrat | 0,03 g | 0,03 g | 0,03 g | 0,03 g | 0,03 g |
| Käse Aroma | 0,03 g | 0,03 g | 0,03 g | 0,03 g | 0,03 g |
| Tomaten Aromakonzentrat | 0,04 g | 0,04 g | 0,04 g | 0,04 g | 0,04 g |
| **Sprühgetrockne-te Zusammensetzung gemäß Beispiel 1.1** | - | - | 0,30 g | 0,45 g | 0,60 g |

6 g der Würzmischung wurden auf 94 g Kartoffelchips aufgezogen. Bei der Verkostung durch ein Panel ausgebildeter Testpersonen wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet. Bei beiden wurde eine verstärkte Salzigkeit im Vergleich zur Zubereitung A festgestellt. Die Natriumglutamat-reduzierte, Salz-reduzierte Zubereitung E wurde hinsichtlich ihres Umami-Geschmacks (und Mundfülle) und ihrer Salzigkeit als abgerundeter und intensiver zur Vergleichszubereitung A bezeichnet.

### Anwendungsbeispiel 11: Weiße Soße

A = Vergleichszubereitung
B = Vergleichszubereitung (Natriumglutamat-reduziert)
C = erfindungsgemäße Zubereitungen (Natriumglutamat-reduziert)
D = erfindungsgemäße Zubereitungen (Natriumglutamat-frei)
E = erfindungsgemäße Zubereitungen (Natriumglutamat- und Salz-reduziert)

| **Bestandteil** | **A** | **B** | **C** | **D** | **E** |
|---|---|---|---|---|---|
| Maltodextrin | 25,98 g | 27,18 g | 27,08 g | 27,58 g | 28,43 g |
| Kochsalz | 7,50 g | 7,50 g | 7,50 g | 7,50 g | 6,00 g |
| Natriumglutamat | 2,00 g | 0,80 g | 0,80 g | - | 0,80 g |
| Pflanzenfett | 5,00 g | 5,00 g | 5,00 g | 5,00 g | 5,00 g |
| Pfeffer, weiß | 0,02 g | 0,02 g | 0,02 g | 0,02 g | 0,02 g |
| Zwiebelpulver | 1,50 g | 1,50 g | 1,50 g | 1,50 g | 1,50 g |
| vorverkleisterte Maisstärke | 30,00 g | 30,00 g | 30,00 g | 30,00 g | 30,00 g |
| Fettpulver | 28,00 g | 28,00 g | 28,00 g | 28,00 g | 28,00 g |
| **Sprühgetrocknete Zusammensetzung gemäß Beispiel 1.2** | - | - | 0,10 g | 0,40 g | 0,25 g |

90 g der Soßenmischung wurden mit 1000 ml heißem Wasser aufgegossen und mit dem Schneebesen kräftig verrührt. Bei der Verkostung durch ein Panel ausgebildeter Testpersonen wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet. Bei beiden wurde eine verstärkte Salzigkeit im Vergleich zur Zubereitung A festgestellt. Die Natriumglutamat-reduzierte, Salz-reduzierte Zubereitung E wurde hinsichtlich ihres Umami-Geschmacks (und Mundfülle) und ihrer Salzigkeit als deutlich abgerundeter und intensiver zur Vergleichszubereitung A bezeichnet.

### Anwendungsbeispiel 12: Braune Soße

A = Vergleichszubereitung
B = Vergleichszubereitung (Natriumglutamat-reduziert)
C = erfindungsgemäße Zubereitungen (Natriumglutamat-reduziert)
D = erfindungsgemäße Zubereitungen (Natriumglutamat-frei)
E = erfindungsgemäße Zubereitungen (Natriumglutamat- und Salz-reduziert)

| **Bestandteil** | **A** | **B** | **C** | **D** | **E** |
|---|---|---|---|---|---|
| Stärke | 40,00 g | 40,00 g | 40,00 g | 40,00 g | 40,00 g |
| Maltodextrin | 33,10 g | 33,80 g | 33,66 g | 34,70 g | 35,07 g |
| Kochsalz | 6,00 g | 6,00 g | 6,00 g | 6,00 g | 4,50 g |
| Zuckerkulör, sprühgetrocknet | 5,00 g | 5,00 g | 5,00 g | 5,00 g | 5,00 g |
| Hefeextraktpulver | 3,00 g | 3,00 g | 3,00 g | 3,00 g | 3,00 g |
| Natriumglutamat | 2,00 g | 1,30 g | 1,30 g | - | 1,30 g |
| Zucker | 0,50 g | 0,50 g | 0,50 g | 0,50 g | 0,50 g |
| Fettpulver | 5,00 g | 5,00 g | 5,00 g | 5,00 g | 5,00 g |
| Tomatenpulver | 3,00 g | 3,00 g | 3,00 g | 3,00 g | 3,00 g |
| Natürlicher Gemüseextrakt | 1,00 g | 1,00 g | 1,00 g | 1,00 g | 1,00 g |
| Zwiebelextrakt | 0,30 g | 0,30 g | 0,30 g | 0,30 g | 0,30 g |
| Pfefferextrakt | 0,10 g | 0,10 g | 0,10 g | 0,10 g | 0,10 g |
| Trockenaroma | 1,00 g | 1,00 g | 1,00 g | 1,00 g | 1,00 g |
| **Sprühgetrocknete Zusammensetzung gemäß Beispiel 1.2** | - | - | 0,70 g | 2,00 g | 0,70 g |

90 g der Soßenmischung wurden mit 1000 ml heißem Wasser aufgegossen und mit dem Schneebesen kräftig verrührt. Bei der Verkostung durch ein Panel ausgebildeter Testpersonen wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet. Bei beiden wurde eine verstärkte Salzigkeit im Vergleich zur Zubereitung A festgestellt. Die Natriumglutamat-reduzierte, Salz-reduzierte Zubereitung E wurde hinsichtlich ihres Umami-Geschmacks (und Mundfülle) und ihrer Salzigkeit als deutlich abgerundeter und intensiver zur Vergleichszubereitung A bezeichnet.

### Anwendungsbeispiel 13: Tomatensuppe

A = Vergleichszubereitung
B = Vergleichszubereitung (Natriumglutamat-reduziert)
C = erfindungsgemäße Zubereitungen (Natriumglutamat-reduziert)
D = erfindungsgemäße Zubereitungen (Natriumglutamat-frei)
E = erfindungsgemäße Zubereitungen (Natriumglutamat- und Salz-reduziert)

| **Bestandteil** | **A** | **B** | **C** | **D** | **E** |
|---|---|---|---|---|---|
| Wasser | 50,65 g | 50,80 g | 50,799 g | 51,035 g | 51,29 g |
| Pflanzenöl | 5,50 g | 5,50 g | 5,50 g | 5,50 g | 5,50 g |
| Tomatenpaste | 24,00 g | 24,00 g | 24,00 g | 24,00 g | 24,00 g |
| Sahne | 1,05 g | 1,05 g | 1,05 g | 1,05 g | 1,05 g |
| Zucker | 2,00 g | 2,00 g | 2,00 g | 2,00 g | 2,00 g |
| Kochsalz | 1,70 g | 1,70 g | 1,70 g | 1,70 g | 1,20 g |
| Natriumglutamat | 0,40 g | 0,25 g | 0,25 g | - | 0,25 g |
| Weizenmehl | 5,50 g | 5,50 g | 5,50 g | 5,50 g | 5,50 g |
| Stärke | 1,20 g | 1,20 g | 1,20 g | 1,20 g | 1,20 g |
| gewürfelte Tomaten | 8,00 g | 8,00 g | 8,00 g | 8,00 g | 8,00 g |
| **Sprühgetrocknete Zusammensetzung gemäß Beispiel 1.2** | - | - | 0,001 g | 0,015 g | 0,010 g |

Die festen Bestandteile wurden eingewogen, gemischt und dem Wasser hinzugefügt. Das Pflanzenöl wurde zudosiert und die Tomatenpaste hinzugegeben. Die Mischung wurde unter Rühren aufgekocht. Bei der Verkostung durch ein Panel ausgebildeter Testpersonen wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet. Bei beiden wurde eine verstärkte Salzigkeit im Vergleich zur Zubereitung A festgestellt. Die Natriumglutamat-reduzierte, Salz-reduzierte Zubereitung E wurde hinsichtlich ihres Umami-Geschmacks (und Mundfülle) und ihrer Salzigkeit als deutlich abgerundeter und intensiver zur Vergleichszubereitung A bezeichnet.

### Anwendungsbeispiel 14: Anwendung in einem zuckerfreien Kaugummi

| **Teil** | **Inhaltsstoff** | **Einsatz in Gew.-%** |
|---|---|---|
| A | Kaugummibase, Company "Jagum T" | 29,995 |
| B | Sorbit, pulverisiert | 39,00 |
| | Isomalt® (Palatinit GmbH) | 9,50 |
| | Xylit | 2,00 |
| | Mannit | 3,00 |
| | Aspartam® | 0,10 |
| | Acesulfam® K | 0,10 |
| | Emulgum® (Colloides Naturels, Inc.) | 0,30 |
| C | Sorbitol, 70 % | 14,00 |
| | Glycerin | 1,00 |
| D | Aromakomposition, entsprechend Anwendungsbeispiel **2.1** | 1,00 |
| | 1 : 1 Mischung der Verbindungen **(11)** + **(12)** | 0,005 |

Teile A bis D werden gemischt und intensiv geknetet. Die Rohmasse kann z.B. in Form von dünnen Streifen zu verzehrfertigen Kaugummis verarbeitet werden.

### Anwendungsbeispiel 15: Anwendung in einem Grünteegetränk

| **Inhaltsstoff** | **Einsatz in Gew.-%** |
|---|---|
| Grünteekonzentrat | 18,00 |
| 1% ige Lösung einer equimolaren Mischung von Verbindungen der Formeln **(7)** und **(8)** in Propylenglycol | 0,002 |
| entmineralisiertes Wasser | 81,998 |

Das Grünteekonzentrat wird mit der 1 %igen Lösung einer equimolaren Mischung der Formeln **(7)** und **(8)** in Propylenglycol vermischt. Anschließend wird mit entmineralisiertem Wasser aufgefüllt und erneut gründlich durchmischt. Dann wird das Produkt gefiltert, verbrauchsfertig verpackt und bei 118°C sterilisiert. Der Geschmack wird durch ein Panel ausgebildeter Testpersonen als deutlich bevorzugt gegenüber dem nicht aromatisierten Grünteekonzentrat bewertet.

### Anwendungsbeispiel 16: Rindfleischwürzmischung für (Fertig)-Nudeln

| **Inhaltsstoff** | **Gew.-%** |
|---|---|
| Rindsfettaroma | 5 |
| Zuckercouleur | 3,00 |
| Zitronensäure (wasserfrei) | 0,40 |
| Schnittlauch (entwässert) | 2,00 |
| Maltodextrin (ex Tapoica) | 10,30 |
| Mononatriumglutamat | 15,00 |
| Zwiebelpulver | 5,00 |
| Ribotide | 0,80 |
| Natriumchlorid | 45,65 |
| Zucker | 2,80 |
| Süßmolkepulver | 6,50 |
| 1% ige Lösung einer equimolaren Mischung von Verbindungen der Formeln Verbindungen der Formeln **(7)** + **(8)** in Propylenglycol | 0,05 |

Alle Inhaltsstoffe werden vermischt, bis sich eine homogene Mischung ergibt.

### Anwendungsbeispiel 17: (Fertig-)Nudeln

| **Teil** | **Inhaltsstoff** | **Gew.-%** |
|---|---|---|
| A | Weizenmehl | 62,00 |
| | Kartoffelstärke | 10,90 |
| B | Salz | 1,10 |
| | Guarkernmehl | 0,06 |
| | Natriumcarbonat | 0,07 |
| | Kaliumcarbonat | 0,25 |
| | Na₂H₂P₂O₇ | 0,07 |
| | 1% ige Lösung einer equimolaren Mischung von Verbindungen der Formeln Verbindungen der Formeln **(11)** + **(12)** in Propylenglycol | 0,05 |
| C | Wasser | 25,45 |

Eine Suspension der Zutaten B in Wasser wird zu einer Mischung der Zutaten A gegeben und zu einem Teig geknetet. Nachdem der Teig für ca. 5 Minuten geruht hat, wird dieser mit Hilfe einer Nudelmaschine zu Platten verarbeitet, die in einem letzten Arbeitsschritt in eine übliche Form zurechtgeschnitten werden. Die Nudeln sind nach einer Kochzeit von 3 Minuten verzehrfertig und werden mit 8 g der Rindfleischwürzmischung (Anwendungsbeispiel 16) angerichtet.

### Figurenbeschreibung:

**Fig. 1****:** Geschmacklicher Vergleich von einer Mischung aus 1-((1S,2S,5R)-2-Isopropyl-5-methyl-cyclohexyl)-3-(3-methoxy-propyl)-harnstoff (**1**) und 1-((1R,2R,5S)-2-Isopropyl-5-methyl-cyclohexyl)-3-(3-methoxy-propyl)-harnstoff (**2**) mit Natriumglutamat.
   Der Geschmack eines 0,5% amerikanischen Rindfleischextrakts als Base (durchgezogene, dunkle Linie) wurde anhand einer Verkostung durch ein Panel ausgebildeter Testpersonen verglichen mit dem Geschmack erstens einer solchen Base, der 1 ppm einer Mischung aus 1-((1S,2S,5R)-2-Isopropyl-5-methyl-cyclohexyl)-3-(3-methoxy-propyl)-harnstoff (**1**) und 1-((1R,2R,5S)-2-Isopropyl-5-methyl-cyclohexyl)-3-(3-methoxy-propyl)-harnstoff (**2**) zugesetzt wurde (durchgezogene, helle Linie), und zweitens einer solchen Base, der 0,05 Gew.-% MSG zugesetzt wurde (punktierte Linie).
   Die Testpersonen bewerteten die Stärke der angegebenen Geschmacksrichtungen jeweils durch Vergeben von Punktzahlen auf einer Skala von 0 (kein entsprechender Geschmack) bis 8 (sehr starker entsprechender Geschmack). Dargestellt sind die Mittelwerte der jeweiligen Punktzahlen.
**Fig. 2****:** Geschmacklicher Vergleich von 1-((1S,2S,5R)-2-Isopropyl-5-methyl-cyclohexyl)-3-(3-methoxy-propyl)-harnstoff (**1**) mit einem Natriumglutamat-haltigen Standardhuhnaroma.
   Der Geschmack der Hühnerbase (durchgezogene, dunkle Linie) wurde anhand einer Verkostung durch ein Panel ausgebildeter Testpersonen verglichen mit dem Geschmack erstens einer solchen Base, der 1 ppm 1-((1S,2S,5R)-2-Isopropyl-5-methyl-cyclohexyl)-3-(3-methoxy-propyl)-harnstoff (**1**) zugesetzt wurde (durchgezogene, helle Linie), und zweitens einer solchen Base, der 0,2 Gew.-% eines Natriumglutamat-haltigen Standardhuhnaromas zugesetzt wurde (punktierte Linie).
   Die Testpersonen bewerteten die Stärke der angegebenen Geschmacksrichtungen jeweils durch Vergeben von Punktzahlen auf einer Skala von 0 (kein entsprechender Geschmack) bis 8 (sehr starker entsprechender Geschmack). Dargestellt sind die Mittelwerte der jeweiligen Punktzahlen.
**Fig. 3****:** Geschmacklicher Vergleich von einer 1:1 Mischung aus 3-[3-((1S,2S,5R)-2-Isopropyl-5-methyl-cyclohexyl)-ureido]-buttersäureethylester (**7**) und 3-[3-((1R,2R,5S)-2-Isopropyl-5-methyl-cyclohexyl)-ureido]-buttersäureethylester (**8**) mit Natriumglutamat.
   Der Geschmack eines 0,5% amerikanischen Rindfleischextrakts als Base (durchgezogene, dunkle Linie) wurde anhand einer Verkostung durch ein Panel ausgebildeter Testpersonen verglichen mit dem Geschmack erstens einer solchen Base, der 5 ppm einer 1:1 Mischung aus 3-[3-((1S,2S,5R)-2-Isopropyl-5-methyl-cyclohexyl)-ureido]-buttersäureethylester (**7**) und 3-[3-((1R,2R,5S)-2-Isopropyl-5-methyl-cyclohexyl)-ureido]-buttersäureethylester (**8**) zugesetzt wurde (durchgezogene, helle Linie), und zweitens einer solchen Base, der 0,05 Gew.-% MSG zugesetzt wurde (punktierte Linie).
   Die Testpersonen bewerteten die Stärke der angegebenen Geschmacksrichtungen jeweils durch Vergeben von Punktzahlen auf einer Skala von 0 (kein entsprechender Geschmack) bis 8 (sehr starker entsprechender Geschmack). Dargestellt sind die Mittelwerte der jeweiligen Punktzahlen.

## Patentansprüche

1. Verwendung einer Verbindung oder einer Mischung bestehend aus zwei oder mehreren Verbindungen oder enthaltend eine oder mehrere Verbindungen ausgewählt aus der Gruppe bestehend aus Verbindungen der Formeln (I) und (*ent*-I) 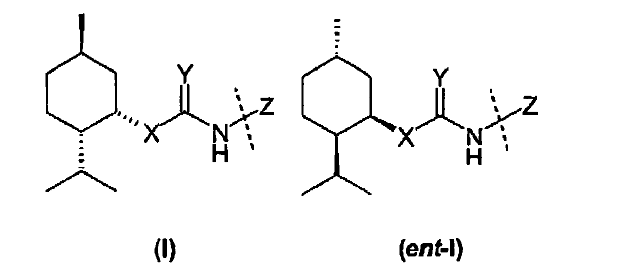 wobei die gestrichelte Linie jeweils die Bindung markiert, welche den organischen Rest Z mit dem in Formel (I) oder (*ent*-I) benachbarten Stickstoffatom verknüpft
und wobei in den Formeln (I) und (*ent*-I) unabhängig voneinander gilt:
X = NH oder O;
Y = O, S oder NR1, wobei R1 Wasserstoff, Methyl, Ethyl, Propyl oder Isopropyl bedeutet; und
Z = organischer Rest mit maximal 15 C-Atomen,
als Geschmacksstoff bzw. Geschmacksstoffmischung.

2. Verwendung einer Verbindung oder einer Mischung wie in Anspruch 1 definiert, wobei (i) die Verbindung der Formel (I) bzw. (*ent*-I) bzw. (ii) eine, zwei, mehr als zwei oder sämtliche Verbindungen der Formeln (I) und (*ent*-I) in der Mischung ausgewählt sind aus der Gruppe bestehend aus:
(1) 1-((1S,2S,5R)-2-Isopropyl-5-methyl-cyclohexyl)-3-(3-methoxy-propyl)-harnstoff,
(2) 1-((1R,2R,5S)-2-Isopropyl-5-methyl-cyclohexyl)-3-(3-methoxy-propyl)-harnstoff,
(3) 1-(3-Hydroxy-propyl)-3-((1S,2S,5R)-2-isopropyl-5-methyl-cyclohexyl)-harnstoff,
(4) 1-(3-Hydroxy-propyl)-3-((1R,2R,5Sr2-isopropyl-5-methyl-cyclohexyl)-harnstoff,
(5) 1-((1S,2S,5R)-2-Isopropyl-5-methyl-cyclohexyl)-3-(2-methoxy-1-methylethyl)-harnstoff,
(6) 1-((1R,2R,5S)-2-Isopropyl-5-methyl-cyclohexyl)-3-(2-methoxy-1-methylethyl)-harnstoff,
(7) 3-[3-((1S,2S,5R)-2-Isopropyl-5-methyl-cyclohexyl)-ureido]-buttersäureethylester,
(8) 3-[3-((1R,2R,5S)-2-Isopropyl-5-methyl-cyclohexyl)-ureido]-buttersäureethylester,
(9) (3-Methoxy-propyl)-carbaminsäure (1S,2S,5R)-2-isopropyl-5-methylcyclohexyl ester,
(10) (3-Methoxy-propyl)-carbaminsäure (1R,2R,5S)-2-isopropyl-5-methylcyclohexyl ester,
(11) 4-((1S,2S,5R)-2-isopropyl-5-methyl-cyclohexyloxycarbonylamino)-buttersäureethylester
und
(12) 4-((1R,2R,5S)-2-Isopropyl-5-methyl-cyclohexyloxycarbonylamino)-buttersäureethylester.

3. Verwendung einer Mischung wie in einem der vorhergehenden Ansprüche definiert, wobei die Mischung ein oder mehrere Paare von Verbindungen der Formeln (I) und (*ent-*I) enthält oder aus ein oder mehreren solcher Paare besteht, wobei jedes Paar aus einer Verbindung der Formel (I) und einer Verbindung der Formel (*ent-*I) wie in einem der vorangehenden Ansprüche definiert besteht, wobei
die Bedeutung von X und Y in der Verbindung der Formel (I) jeweils identisch ist mit der Bedeutung von X und Y in der Verbindung der Formel (*ent-*I) und
wobei
(a) die Bedeutung von Z in der Verbindung der Formel (I) identisch ist mit der Bedeutung von Z in der Verbindung der Formel (*ent*-I) oder
(b) Z in der Verbindung der Formel (I) sowie Z in der Verbindung der Formel (*ent-*I) ein oder mehrere chirale Zentren enthalten und sich ausschließlich durch die absolute Konfiguration an einem, mehreren oder sämtlichen dieser chiralen Zentren unterscheiden,
wobei vorzugsweise jedes Paar ein Enantiomeren- oder Epimerenpaar ist.

4. Verwendung einer Mischung als Geschmacksstoffmischung, welche aus folgenden Komponenten besteht oder diese enthält:
(a) eine Verbindung ausgewählt aus der Gruppe bestehend aus Verbindungen der Formeln (I) und (*ent*-I) wie in einem der vorhergehenden Ansprüche definiert oder eine Mischung bestehend aus zwei oder mehreren Verbindungen oder enthaltend eine oder mehrere Verbindungen ausgewählt aus der Gruppe bestehend aus Verbindungen der Formeln (I) und (*ent*-I) wie in einem der vorhergehenden Ansprüche definiert;
sowie
(b) eine Verbindung ausgewählt aus der Gruppe bestehend aus Verbindungen der Formeln (II), (*ent*-II), (III), (ent-III), (IV), (*ent*-IV) oder eine Mischung bestehend aus zwei oder mehreren Verbindungen oder enthaltend eine oder mehrere Verbindungen ausgewählt aus der Gruppe bestehend aus Verbindungen der Formeln (II), (*ent*-II), (III), (*ent*-III), (IV), (*ent*-IV): 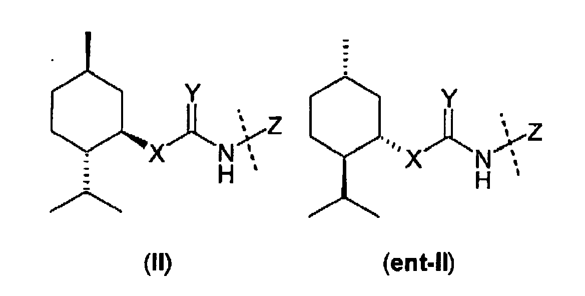 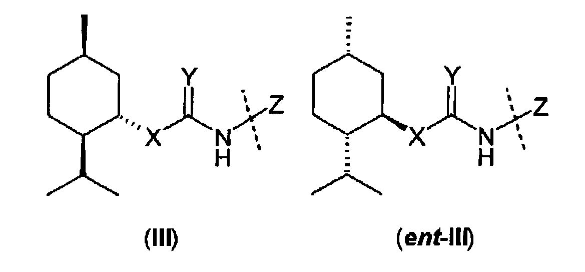 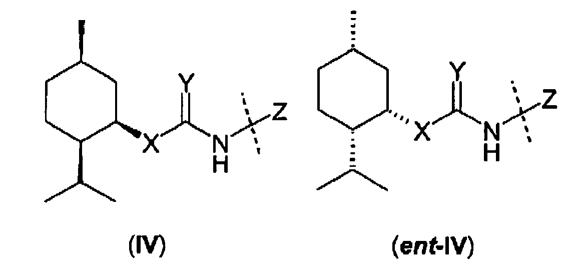 wobei in den Formeln (II), (*ent*-II), (III), (*ent*-III), (IV), (*ent-*IV) X, Y und Z jeweils unabhängig voneinander eine der in einem der vorhergehenden Ansprüche für die Formeln (I) und (*ent*-I) angegebenen Bedeutungen besitzt
und
wobei in der Geschmacksstoffmischung das Gewichtsverhältnis von (a) der Gesamtheit von Verbindungen der Formeln (I) und (*ent*-I) zu (b) der Gesamtheit von Verbindungen der Formeln (11), (*ent*-II), (III), (*ent*-III), (IV) und (*ent*-IV) mindestens 60:40, bevorzugt mindestens 90:10, insbesondere bevorzugt mindestens 95:5 ist.

5. Verwendung einer Verbindung wie in Anspruch 1 oder 2 definiert oder einer Mischung wie in einem der vorhergehenden Ansprüche definiert, zum Erzeugen, Vermitteln, Modifizieren und/oder Verstärken eines Umami-Geschmacks und/oder der Salzigkeit.

6. Einzelne Verbindung oder Mischung bestehend aus zwei oder mehreren Verbindungen oder enthaltend eine oder mehrere Verbindungen ausgewählt aus der Gruppe bestehend aus Verbindungen der Formen (I) und (ent-I) 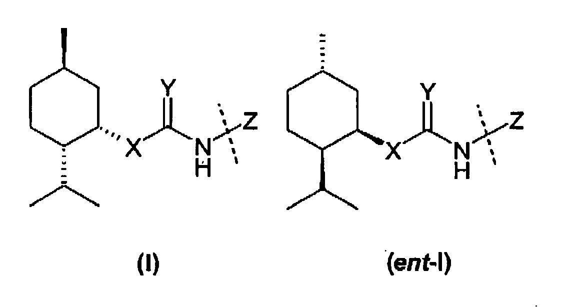 wobei die gestrichelte Linie jeweils die Bindung markiert, welche den organischen Rest Z mit dem in Formel (I) oder (ent-I) benachbarten Stickstoffatom verknüpft
und wobei in den Formeln (I) und (ent-I) unabhängig voneinander gilt:
X = NH oder O;
Y = O, S oder NR1, wobei R1 Wasserstoff, Methyl, Ethyl, Propyl oder Isopropyl bedeutet: und
Z = organischer Rest bestehend aus Kohlenstoff- und Wasserstoffatomen sowie gegebenenfalls Sauerstoffatomen, wobei
die Summe der Anzahl von Kohlenstoff- und Sauerstoffatomen maximal 15 ist und die Zahl der Sauerstoffatome maximal 4, und
das Atom, mit dem der organische Rest Z an das in Formel (I) oder (*ent*-I) benachbarte Stickstoffatom gebunden ist, Kohlenstoff ist,
mit der Maßgabe dass
der organische Rest Z keine C-C-Doppel- oder -Dreifachbindung enthält, höchstens einen aromatischen Ring enthält und keine Gruppe -C(=Y²)-R2 ist, in der Y² eine beliebige der oben für Y angegebenen Bedeutungen hat und R2 ein beliebiger organischer Rest ist,
Z nicht -C(R5)(R6)-C(=O)OR4 ist, wobei R4 H, Methyl, Ethyl, Propyl, iso-Propyl, Butyl, iso-Butyl oder tert.-Butyl bedeutet und R5 und R6 unabhängig voneinander H oder einen organischen Rest bedeuten,
Z nicht 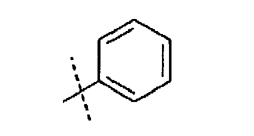
ist, wobei die gestrichelte Linie die Bindung markiert, welche den organischen Rest Z mit dem in Formel (I) oder (ent-I) benachbarten Stickstoffatom verknüpft,
wenn X = NH und Y = O ist, Z nicht Neomenthyl oder 2-Methylcyclohexyl ist, und
wenn X = NH und Y = S ist, Z nicht Neomenthyl ist, und
wenn X = O und Y = O ist, Z nicht Ethyl, Hydroxyathyl, 1-Phenylethyl, 2-Phsnylethyl, 2-Hydroxy-1-methyl-2-phenylethyl, Isopropyl, 2-Hydroxy-1-methylethyl, Cyclohexyl, 4-Hydroxyphenyl, 2-Hydroxy-5-methylphenyl oder 2,4,7-Trioxa-3,3,8 trimethylbicyclo[3.3.0]octan-6-ylmethyl bedeutet
und
wobei die Verbindungen der Formel (I) die folgende Verbindung nicht umfassen: 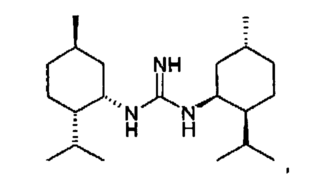 und wobei weiterhin ausgenommen sind
die einzelnen Verbindungen der Formeln (I) und (*ent*-I) in denen X = O, Y =O und Z = (-)-Menthyl bedeuten,
eine Mischung bestehend aus 50,2% der Verbindung der Formel (I), in der X = O, Y =O und Z = (-)-Menthyl bedeuten und 49,8% der Verbindung der Formel (*ent*-I) in der X = O, Y =O und Z = (-)-Menthyl bedeuten und
eine Mischung bestehend aus 30,7% der Verbindung der Formeln (I) in denen X = O, Y =O und Z = (-)-Menthyl bedeuten und 69,3% N-(-)-Menthytcarbaminsäure (-)-Menthylester.

7. Einzelne Verbindung oder Mischung wie in Anspruch 6 definiert, wobei (i) für die Verbindung der Formeln (I) bzw. (*ent*-I) bzw. (ii) für eine oder mehrere oder sämtliche Verbindungen der Formeln (I) und (*ent*-I) in der Mischung entweder A:
der organische Rest Z ausgewählt ist aus der Gruppe bestehend aus: 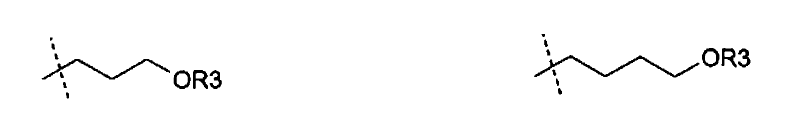 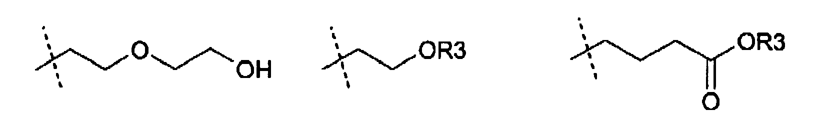 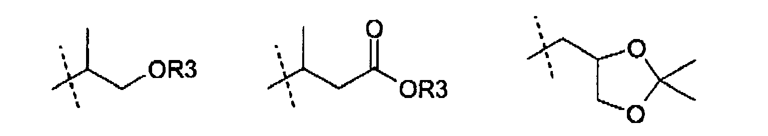 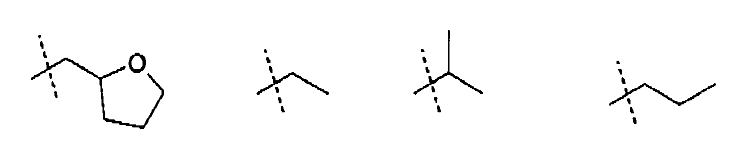 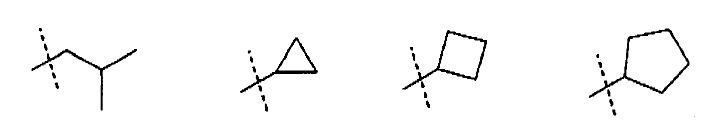 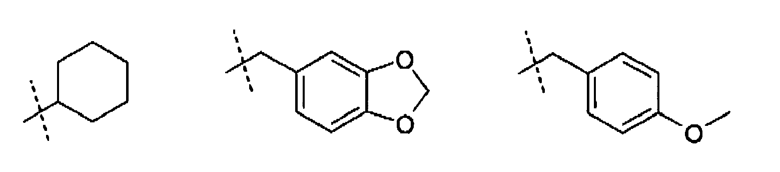 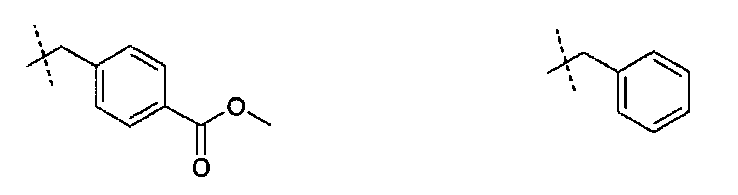 wobei gegebenenfalls vorhandene Reste R3 unabhängig voneinander Wasserstoff. Methyl, Ethyl, *n*-Propyl, *iso*-Propyl, *n*-Butyl oder *iso*-Butyl bedeuten und die gestrichelte Linie die Bindung markiert, welche den organischen Rest Z mit dem in Formel (I) oder (*ent*-I) benachbarten Stickstoffatom verknüpft
oder
B:
Y O: und
Z = organischer Rest in dem die Summe der Anzahl von Kohlenstoff und Sauerstoffatomen maximal 11 ist und die Zahl der Sauerstoffatome maximal 2, wobei Z ein Rest ausgewählt aus der Gruppe bestehend aus gegebenenfalls ein- oder mehrfach substituiertem Alkyl, C₃-Cycloalkyl, C₇-Cycloalkyl und Phenylalkyl ist, wobei ein oder mehrere gegebenenfalls im Rest Z vorhandene Substituenten vorzugsweise ausgewählt sind aus der Gruppe bestehend aus OH, C₁-C₄-Alkoxy, C₁-C₄-Alkoxycarbonyl und cyclische Ether,
wobei cyclische Gruppen im organischen Rest Z gegebenenfalls durch C₁-C₄-Alkylgruppen substituiert sind.

8. Einzelne Verbindung oder Mischung wie in einem der Ansprüche 6 oder 7 definiert, wobei (i) die Verbindung der Formel (I) bzw. (ent-I) bzw. (ii) eine, zwei, mehr als zwei oder sämtliche Verbindungen der Formeln (I) und (ent-I) in der Mischung ausgewählt sind aus der Gruppe bestehend aus:
(1) 1-((1S,25,5R)-2-Isopropyl-5-methyl-cyclohexyl)-3-(3-methoxy-propyl)-harnstoff,
(2) 1-((1R,2R,5S)-2-Isopropyl-5-methyl-cyclohexyl)-3-(3-mathoxy-propyl)-harnstoff,
(3) 1-(3-Hydroxy-propyl)-3-((1S,25,5R)-2-isopropyl-5-methyl-cyclohexyl)-harnstoff,
(4) 1-(3-Hydroxy-propyl)-3-((1R,2R,5S)-2-isopropyl-5-methyl-cyclohexyl)-harnstoff,
(5) 1-((1S,2S,5R)-2-Isopropyl-5-methl-cydohexyl)-3-(2-methoxy-1-methylethyl)-harnstoff,
(6) 1-((1R,2R,5S)-2-lsopropyl-5-methyl-cyclohexyl)-3-(2-methoxy-1-methylethyl)-harnstoff,
(7) 3-[3-((1S,2S,5R)-2-Isopropyl-5-methyl-cyclohexyl)-ureido]-buttersäureethylester,
(8) 3-[3-((1R,2R,5S)-2-Isopropyl-5-methyl-cyclohexyl)-ureido]-buttersäureethylester,
(9) (3-Methoxy-propyl)-carbaminsäure (1S,2S,5R)-2-isopropyl-5-methylcyclohexyl ester,
(10) (3-Methoxy-propyl)-carbaminsäure (1R,2R,5S)-2-isopropyl-5-methylcyclohexyl ester,
(11) 4-((1S,25,5R)-2-Isopropyl-5-methyl-cyclohexyloxycarbonylamino)-buttersäureethylester
und
(12) 4-((1R,2R,5S)-2-Isopropyl-5-methyl-cyclohexyloxycarbonylamino)-buttersäureethylester.

9. Mischung wie in einem der Ansprüche 6 bis 8 definiert, wobei die Mischung ein oder mehrere Paare von Verbindungen der Formeln (I) und (*ent*-I) enthält oder aus ein oder mehreren solcher Paare besteht, wobei jedes Paar aus einer Verbindung der Formel (I) und einer Verbindung der Formel (*ent*-I) wie in einem der vorangehenden Ansprüche definiert besteht,
wobei
die Bedeutung von X und Y in der Verbindung der Formel (I) jeweils identisch ist mit der Bedeutung von X und Y in der Verbindung der Formel (*ent*-I) und
wobei
(a) die Bedeutung von Z in der Verbindung der Formel (I) identisch ist mit der Bedeutung von Z in der Verbindung der Formel (*ent*-I) oder
(b) Z in der Verbindung der Formel (I) sowie Z in der Verbindung der Formel (*ent-*I) ein oder mehrere chirale Zentren enthalten und sich ausschließlich durch die absolute Konfiguration an einem oder sämtlichen dieser chirale Zentren unterscheiden,
wobei vorzugsweise jedes Paar ein Enantiomeren- oder Epimerenpaar ist.

10. Mischung, welche aus folgenden Komponenten besteht oder diese enthält:
(a) eine Verbindung ausgewählt aus der Gruppe bestehend aus Verbindungen der Formeln (I) und (*ent*-I) wie in einem der Ansprüche 6 bis 8 definiert oder eine Mischung bestehend aus zwei oder mehreren Verbindungen oder enthaltend eine oder mehrere Verbindungen ausgewählt aus der Gruppe bestehend aus Verbindungen der Formeln (I) und *(ent-I)* wie in einem der Ansprüche 6 bis 8 definiert oder eine Mischung bestehend aus oder enthaltend eine Mischung wie in Anspruch 9 definiert;
sowie
(b) eine Verbindung ausgewählt aus der Gruppe bestehend aus Verbindungen der Formeln (II), *(ent*-II), (III), (ent-III), (IV), *(ent-IV)* oder eine Mischung bestehend aus zwei oder mehreren Verbindungen oder enthaltend eine oder mehrere Verbindungen ausgewählt aus der Gruppe bestehend aus Verbindungen der Formeln (II), (*engt*-II), (III), (*ent*-III), (IV), (*ent*-IV): 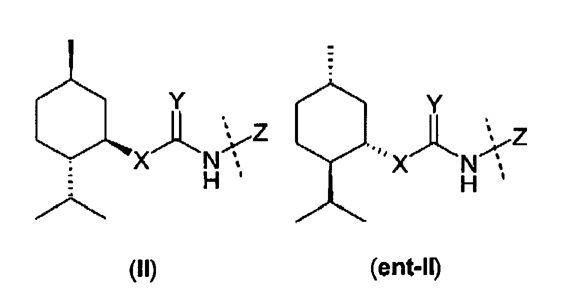 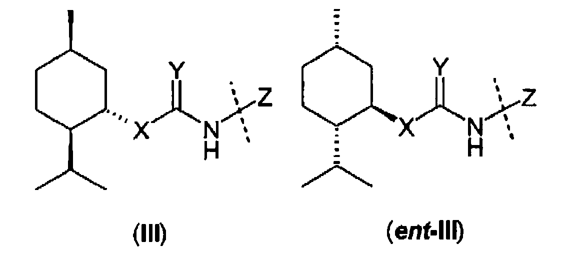 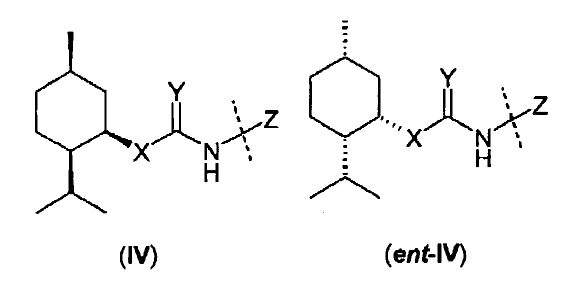 wobei in den Formeln (II), (*ent*-II), (III), (*ent*-III), (IV), (*ent*-IV) X, Y und Z jeweils unabhängig voneinander eine der in einem der vorhergehenden Ansprüche für die Formeln (I) und (*ent*-I) angegebenen Bedeutungen besitzt
und
wobei in der Mischung das Gewichtsverhältnis von (a) der Gesamtheit von Verbindungen der Formeln (I) und (*ent*-I) zu (b) der Gesamtheit von Verbindungen der Formeln (II), (*ent-*II), (III), (*ent*-III), (IV) und (*ent*-IV) mindestens 60:40, bevorzugt mindestens 90:10, insbesondere bevorzugt mindestens 95:5 ist.

11. Zusammensetzung, insbesondere zum Verzehr geeignete Zusammensetzung, umfassend oder bestehend aus
einer geschmacklich wirksamen Menge einer Verbindung oder einer Mischung wie in einem der vorstehenden Ansprüche definiert
und einem oder mehreren weiteren zum Verzehr geeigneten Bestandteilen, die als zum Verzehr geeignete Bestandteile feste Trägerstoffe und ggf. Aromakompositionen umfasst.

12. Verwendung von Verbindungen wie in einem der Ansprüche 1, 2 oder 6 bis 8 definiert oder Mischungen wie in einem der Ansprüche 1 bis 10 definiert oder von Zusammensetzungen insbesondere zum Verzehr geeignete Zusammensetzungen, umfassend oder bestehend aus einer geschmacklich wirksamen Menge einer Verbindung oder einer Mischung wie in einem der Ansprüche 1 bis 9 definiert und einem oder mehreren weiteren zum Verzehr geeigneten Bestandteilen,
in der Ernährung oder dem Genuss dienenden
(i) gebrauchs- oder verzehrfertigen Zubereitungen oder
(ii) Halbfertigwaren,
insbesondere in der Ernährung oder dem Genuss dienenden Natriumglutamatreduzierten oder -freien Zubereitungen.

13. Der Ernährung oder dem Genuss dienende gebrauchs- oder verzehrfertige Zubereitung umfassend Verbindungen wie in einem der Ansprüche 1, 2 oder 6 bis 8 definiert oder Mischungen wie in einem der Ansprüche 1 bis 10 definiert oder Zusammensetzungen gemäß Anspruch11 in einer Menge im Bereich von 0,01 ppm bis 100 ppm.

14. Halbfertigwaren umfassend Verbindungen wie in einem der Ansprüche 1, 2 oder 6 bis 8 definiert oder Mischungen wie in Ansprüchen 1 bis 9 definiert oder Zusammensetzungen gemäß Anspruch 11 und Natriumglutamat.

15. Verfahren zum Erzeugen, Vermitteln, Modifizieren und/oder Verstärken eines Geschmackseindrucks, worin einer Substanz oder Zusammensetzung eine Verbindung wie in einem der Ansprüche 1, 2 oder 6 bis 8 definiert oder eine Mischung wie in einem der Ansprüche 1 bis 10 definiert oder eine Zusammensetzung wie in Anspruch 11 definiert oder eine Zubereitung gemäß Anspruch 13 oder eine Halbfertigware gemäß Anspruch 14 in einer geschmacklich wirksamen Menge zugesetzt wird.

## Claims

1. Use of a compound or of a mixture consisting of two or more compounds or containing one or more compounds selected from the group consisting of compounds of the formulae (I) and (*ent*-I) 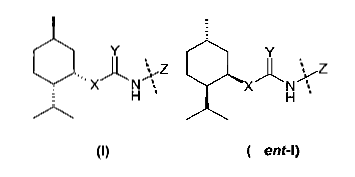 wherein the dashed line respectively marks the bond which links the organic residue Z to the adjacent nitrogen atom in the formula (I) or (*ent*-I),
and wherein the following apply independently of one another in the formulae (I) and (*ent*-I):
X = NH or 0;
Y = 0, S or NR1, where R1 denotes hydrogen, methyl, ethyl, propyl or isopropyl; and
Z = organic residue having at most 15 C atoms,
as a flavour material or flavour material mixture.

2. The use of a compound or of a mixture as defined in claim 1, wherein (i) the compound of the formula (I) or (*ent*-I) or (ii) one, two, more than two or all of the compounds of the formulae (I) and (*ent*-I) in the mixture are selected from the group consisting of:
(1) 1-((1S,2S,5R)-2-isopropyl-5-methylcyclohexyl)-3-(3-methoxypropyl)urea,
(2) 1-((1R,2R,5S)-2-isopropyl-5-methylcyclohexyl)-3-(3-methoxypropyl)urea,
(3) 1-(3-hydroxypropyl)-3-((1S,2S,5R)-2-isopropyl-5-methylcyclohexyl) urea,
(4) 1-(3-hydroxypropyl)-3-((1R,2R,5S)-2-isopropyl-5-methylcyclohexyl)urea,
(5) 1-((1S,2S,5R)-2-isopropyl-5-methylcyclohexyl)-3-(2-methoxy-1-methylethyl)urea,
(6) 1-((1R,2R,5S)-2-isopropyl-5-methylcyclohexyl)-3-(2-methoxy-1-methylethyl)urea,
(7) 3-[3-((1S,2S,5R)-2-isopropyl-5-methylcyclohexyl)ureido]butyric acid ethyl ester,
(8) 3-[3-((1R,2R,5S)-2-isopropyl-5-methylcyclohexyl)ureido]butyrie acid ethyl ester,
(9) (3-methoxypropyl)carbamic acid (1S,2S,5R)-2-isopropyl-5-methylcyclohexyl ester,
(10) (3-methoxypropyl)carbamic acid (1R,2R,5S)-2-isopropyl-5-methylcyclohexyl ester,
(11) 4-((1S,2S,5R)-2-isopropyl-5-methylcyclohexyloxycarbonylamino)butyric acid ethyl ester
and
(12) 4-((1R,2R,5S)-2-isopropyl-5-methylcyclohexyloxycarbonylamino)butyric acid ethyl ester.

3. The use of a mixture as defined in one of the preceding claims, wherein the mixture contains one or more pairs of compounds of the formulae (I) and (ent-I) or consists of one or more such pairs, each pair consisting of a compound of the formula (I) and a compound of the formula (ent-I) as defined in one of the preceding claims, wherein
the meaning of X and Y in the compound of the formula (I) is identical in each case to the meaning of X and Y in the compound of the formula (ent-I) and
wherein
(a) the meaning of Z in the compound of the formula (I) is identical to the meaning of Z in the compound of the formula (*ent*-I) or
(b) Z in the compound of the formula (I) and Z in the compound of the formula (*ent*-I) contain one or more chiral centres and differ only by the absolute configuration at one, several or all of these chiral centres,
wherein each pair is preferably an enantiomer or epimer pair.

4. Use of a mixture as a flavour material mixture, which consists of the following components or contains them:
(a) a compound selected from the group consisting of compounds of the formulae (I) and (*ent*-I) as defined in one of the preceding claims or a mixture consisting of two or more compounds or containing one or more compounds selected from the group consisting of compounds of the formulae (I) and (*ent*-I) as defined in one of the preceding claims;
and
(b) a compound selected from the group consisting of compounds of the formulae (II), (*ent*-II), (III), (ent-III), (IV), (*ent*-IV) or a mixture consisting of two or more compounds or containing one or more compounds selected from the group consisting of compounds of the formulae (II), (*ent*-II), (III), (*ent*-III), (IV), (*ent*-IV): 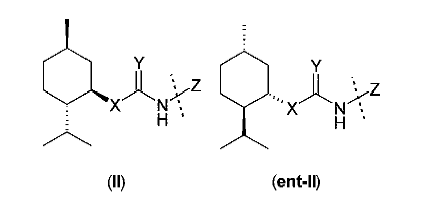 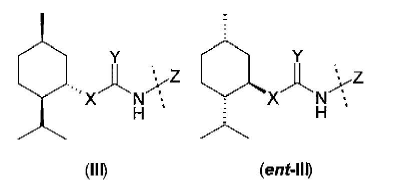 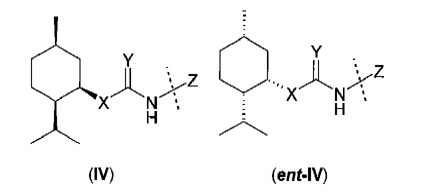 wherein X, Y and Z in the formulae (II), (*ent*-II), (III), (*ent*-III), (IV), (*ent*-IV) respectively have, independently of one another, one of the meanings specified in one of the preceding claims for the formulae (I) and (*ent*-I),
and
wherein the weight ratio of (a) the total quantity of compounds of the formulae (I) and (*ent*-I) to (b) the total quantity of compounds of the formulae (II), (*ent*-II), (III), (*ent*-III), (IV) and (*ent*-IV) in the flavour material mixture is at least 60:40, preferably at least 90:10 and particularly preferably at least 95:5.

5. The use of a compound as defined in claim 1 or 2 or of a mixture as defined in one of the preceding claims for producing, imparting, modifying and/or enhancing an umami flavour and/or saltiness.

6. A single compound or mixture consisting of two or more compounds or containing one or more compounds selected from the group consisting of compounds of the formulae (I) and (*ent*-I) 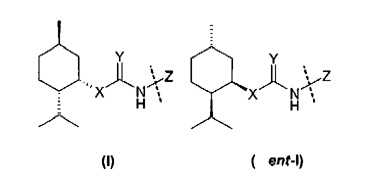 wherein the dashed line respectively marks the bond which links the organic residue Z to the adjacent nitrogen atom in the formula (I) or (*ent*-I),
and wherein the following apply independently of one another in the formulae (I) and (*ent*-I);
X = NH or 0;
Y = O, S or NR1, where R1 denotes hydrogen, methyl, ethyl, propyl or isopropyl; and
Z = organic residue consisting of carbon and hydrogen atoms and optionally oxygen atoms, wherein
the sum of the number of carbon and oxygen atoms is at most 15 and the number of oxygen atoms is at most 4, and
the atom by which the organic residue Z is bound to the adjacent nitrogen atom in the formula (I) or (*ent*-I) is carbon,
with the proviso that
the organic residue Z does not contain a C-C double or triple bond, contains at most one aromatic ring, and is not a group -C(=Y²)-R2 in which Y² has any of the meanings specified above for Y and R2 is any organic residue,
Z is not -C(R5)(R6)-C(=O)OR4, where R4 denotes H, methyl, ethyl, propyl, isopropyl, butyl, isobutyl or tert.-butyl and R5 and R6 independently of one another denote H or an organic residue,
Z is not 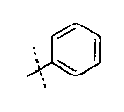 wherein the dashed line marks the bond which links the organic residue Z to the adjacent nitrogen atom in the formula (I) or (*ent*-I),
if X = NH and Y = O, then Z is not neomenthyl or 2-methylcyclohexyl, and
if X = NH and Y = S, then Z is not neomenthyl, and
if X = O and Y = O, then Z does not denote ethyl, hydroxyethyl, 1-phenylethyl, 2-phenylethyl, 2-hydroxy-1-methyl-2-phenylethyl, isopropyl, 2-hydroxy-1-methylethyl, cyclohexyl, 4-hydroxyphenyl, 2-hydroxy-5-methylphenyl or 2,4,7-trioxa-3,3,8-trimethylbicyclo[3.3.0]octan-6-ylmethyl
and
wherein the compounds of the formula (I) do not include the following compound: 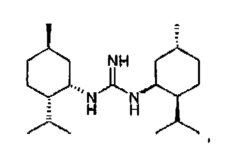 and wherein the following are also excluded:
the individual compounds of the formulae (I) and
(*ent*-I) in which X = O, Y = O and Z = (-)-menthyl,
a mixture consisting of 50.2% of the compound of the formula (I) in which X = O, Y = O and Z = (-)-menthyl and 49.8% of the compound of the formula (ent-I) in which X = O, Y = O and Z = (-)-menthyl and
a mixture consisting of 30.7% of the compound of the formulae (I) in which X = O, Y = O and Z = (-)-menthyl and 69.3% N-(-)menthylcarbamic acid (-)-menthyl ester.

7. The single compound or mixture as defined in claim 6, wherein (i) for the compound of the formulae (I) or (*ent*-I) or (ii) for one or several or all of the compounds of the formulae (I) and (*ent*-I) in the mixture, either
A:
the organic residue Z is selected from the group consisting of: 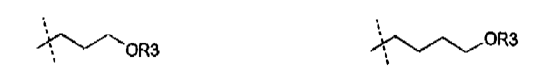 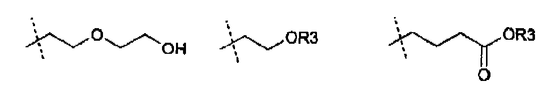 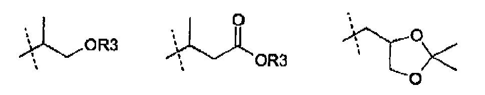 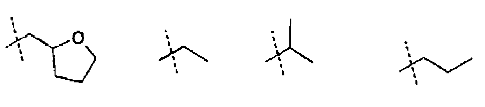 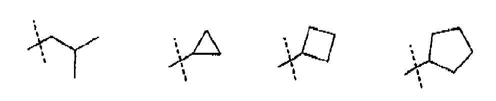 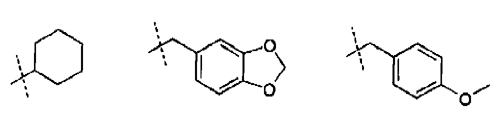 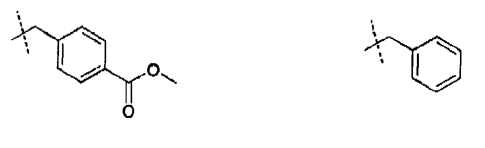
wherein optionally present residues R3 independently of one another denote hydrogen, methyl, ethyl, n-propyl, isopropyl, n-butyl or isobutyl and the dashed line marks the bond which links the organic residue Z to the adjacent nitrogen atom in the formula (I) or (*ent*-I)
or
B:
Y = O; and
Z = organic residue in which the sum of the number of carbon and oxygen atoms is at most 11 and the number of oxygen atoms is at most 2, wherein Z is a residue selected from the group consisting of optionally mono- or polysubstituted alkyl, C₃ cycloalkyl, C₇ cycloalkyl and phenylalkyl, wherein
one or more substituents optionally present in the residue Z are preferably selected from the group consisting of OH, C₁-C₄ alkoxy, C₁-C₄ alkoxycarbonyl and cyclic ethers, wherein cyclic groups in the organic residue Z are optionally substituted by C₁-C₄ alkyl groups.

8. The single compound or mixture as defined in one of claims 6 or 7, wherein (i) the compound of the formulae (I) or (*ent*-I) or (ii) one, two, more than two or all of the compounds of the formulae (I) and (*ent*-I) in the mixture are selected from the group consisting of:
(1) 1-((1S,2S,5R)-2-isopropyl-5-methylcyclohexyl)-3-(3-methoxypropyl)urea,
(2) 1-((1R,2R,5S)-2-isopropyl-5-methylcyclohexyl)-3-(3-methoxypropyl)urea,
(3) 1-(3-hydroxypropyl)-3-((1S,2S,5R)-2-isopropyl-5-methylcyclohexyl)urea,
(4) 1-(3-hydroxypropyl)-3-((1R,2R,5S)-2-isopropyl-5-methylcyclohexyl)urea,
(5) 1-((1S,2S,5R)-2-isopropyl-5-methylcyclohexyl)-3-(2-methoxy-1-methylethyl)urea
(6) 1-((1R,2R,5S)-2-isopropyl-5-methylcyclohexyl)-3-(2-methoxy-1-methylethyl)urea,
(7) 3-[3-((1S,2S,5R)-2-isopropyl-5-methylcyclohexyl)ureido]butyric acid ethyl ester,
(8) 3-[3-((1R,2R,5S)-2-isopropyl-5-methylcyclohexyl)ureido]butyric acid ethyl ester,
(9) (3-methoxypropyl)carbamic acid (1S,2S,5R)-2-isopropyl-5-methylcyclohexyl ester,
(10) (3-methoxypropyl)carbamic acid (1R,2R,5S)-2-isopropyl-5-methylcyclohexyl ester,
(11) 4-((1S,2S,5R)-2-isopropyl-5-methylcyclohexyloxycarbonylamino)butyric acid ethyl ester
and
(12) 4-((1R,2R,5S)-2-isopropyl-5-methylcyclohexyloxycarbonylamino)butyric acid ethyl ester.

9. The mixture as defined in one of claims 6 to 8, wherein the mixture contains one or more pairs of compounds of the formulae (I) and (*ent*-I) or consists of one or more such pairs, each pair consisting of a compound of the formula (I) and a compound of the formula (*ent*-I) as defined in one of the preceding claims,
wherein
the meanings of X and Y in the compound of the formula (I) are respectively identical to the meanings of X and Y in the compound of the formula (*ent*-I), and
wherein
(a) the meaning of Z in the compound of the formula (I) is identical to the meaning of Z in the compound of the formula (*ent*-I) or
(b) Z in the compound of the formula (I) and Z in the compound of the formula (*ent*-I) contain one or more chiral centres and differ only by the absolute configuration at one or all of these chiral centres Z,
wherein each pair is preferably an enantiomer or epimer pair.

10. A mixture, which consists of the following components or contains them:
(a) a compound selected from the group consisting of compounds of the formulae (I) and (*ent*-I) as defined in one of claims 6 to 8 or a mixture consisting of two or more compounds or containing one or more compounds selected from the group consisting of compounds of the formulae (I) and (*ent*-I) as defined in one of claims 6 to 8 or a mixture consisting of or containing a mixture as defined in claim 9;
and
(b) a compound selected from the group consisting of compounds of the formulae (II), (*ent*-II), (III), (*ent*-III), (IV), (*ent*-IV) or a mixture consisting of two or more compounds or containing one or more compounds selected from the group consisting of compounds of the formulae (II), (*ent*-II), (III), (*ent*-III), (IV), (*ent*-IV): 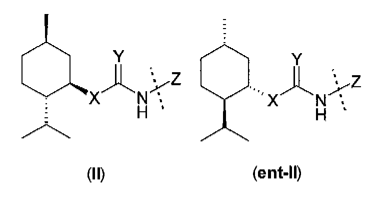 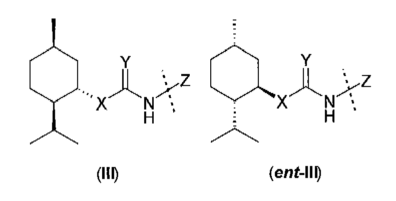 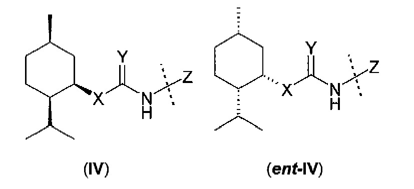 wherein X, Y and Z in the formulae (II), (*ent*-II), (III), (*ent*-III), (IV), (*ent*-IV) respectively have, independently of one another, one of the meanings specified in one of the preceding claims for the formulae (I) and (*ent*-I),
and
wherein the weight ratio of (a) the total amount of compounds of the formulae (I) and (*ent*-I) to (b) the total amount of compounds of the formulae (II), (*ent-*II), (III), (*ent*-III), (IV) and (*ent*-IV) in the mixture is at least 60:40, preferably at least 90:10 and particularly preferably at least 95:5.

11. A composition, in particular a composition suitable for consumption, comprising or consisting of an effective flavouring amount of a compound or mixture as defined in one of the preceding claims and one or more further constituents suitable for consumption, which comprises solid carriers and optionally aroma compositions as constituents suitable for consumption.

12. The use of compounds as defined in one of claims 1, 2 or 6 to 8 or mixtures as defined in one of claims 1 to 10 or of compositions in particular compositions suitable for consumption comprising or consisting of an effective flavouring amount of a compound or mixture as defined in one of claims 1 to 9 and one or more further constituents suitable for consumption, in
(i) preparations ready for use or consumption or
(ii) semi-finished products used for staple or luxury foods,
in particular in sodium glutamate-reduced or -free preparations used for staple or luxury foods.

13. A preparation ready for use or consumption used for staple or luxury foods comprising compounds as defined in one of claims 1, 2 or 6 to 8 or mixtures as defined in one of claims 1 to 10 or compositions according to claim 11 in a quantity in the range of 0.01 ppm to 100 ppm.

14. Semi-finished products comprising compounds as defined in one of claims 1, 2 or 6 to 8 or mixtures as defined in claims 1 to 9 or compositions according to claim 11 and sodium glutamate.

15. A method for producing, imparting, modifying and/or enhancing a flavour impression, wherein a compound as defined in one of claims 1, 2 or 6 to 8 or a mixture as defined in one of claims 1 to 10 or a composition as defined in claim 11 or a preparation according to claim 13 or a semi-finished product according to claim 14 is added to a substance or composition in an effective flavouring amount.

## Revendications

1. Utilisation d'un composé ou d'un mélange constitué de deux composés ou plus ou contenant un ou plusieurs composés choisis dans le groupe comprenant des composés de formules (I) et (ent-I) 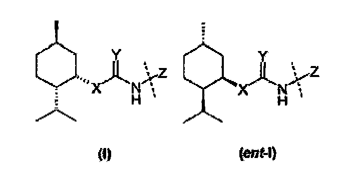 dans lesquelles la ligne en pointillé marque respectivement la liaison qui relie le radical organique Z à l'atome d'azote voisin dans les formules (I) ou (ent-I),
et où, dans les formules (I) et (ent-I), indépendamment les uns des autres :
X = NH ou 0 ;
Y = O, S ou NR1, où R1 est un atome d'hydrogène, un groupe méthyle, éthyle, propyle ou isopropyle ;
et
Z = un radical organique comportant au maximum 15 atomes de carbone,
comme agent de sapidité ou mélange d'agents de sapidité.

2. Utilisation d'un composé ou d'un mélange tel que défini dans la revendication 1, dans laquelle (i) le composé de formules (I) ou (ent-I) ou (ii), un, deux, plus de deux ou tous les composés de formules (I) et (ent-I) dans le mélange sont choisis dans le groupe comprenant :
(1) la 1-((1S,2S,5R)-2-isopropyl-5-méthylcyclohexyl)-3-(3-méthoxy-propyl)-urée,
(2) la 1-((1R,2R,5S)-2-isopropyl-5-méthylcyclohexyl)-3-(3-méthoxy-propyl)-urée
(3) la 1-(3-hydroxy-propyl)-3-((1S,2S,5R)-2-isopropyl-5-méthyl-cyclohexyl)urée
(4) la 1-(3-hydroxy-propyl)-3-((1R,2R,5S)-2-isopropyl-5-méthyl-cyclohexyl)urée
(5) la 1-((1S,2S,5R)-2-isopropyl-5-méthylcyclohexyl)-3-(2-méthoxy-1-méthyl-éthyl)-urée
(6) la 1-((1R,2R,5S)-2-isopropyl-5-méthylcyclohexyl)-3-(2-méthoxy-1-méthyl-éthyl)-urée
(7) l'acide 3-[3-((1S,2S,5R)-2-isopropyl-5-méthylcyclohexyl)-uréido]-butyrique éthylester
(8) l'acide 3-[3-((1R,2R,5S)-2-isopropyl-5-méthylcyclohexyl)-uréido]-butyrique éthylester
(9) l'acide (3-méthoxypropyl)-carbamique (1S,2S,SR)-2-isopropyl-5-méthyl-cyclohexyl-ester
(10) l'acide (3-méthoxypropyl)-carbamique (1R,2R,5S)-2-isopropyl-5-méthyl-cyclohexyl-ester
(11) l'acide 4-((1S,2S,5R)-2-isopropyl-5-méthylcyclohexyloxycarbonylamino)-butyrique éthylester et
(12) l'acide 4-((1R,2R,5S)-2-isopropyl-5-méthylcyclohexyloxycarbonylamino)-butyrique éthylester

3. Utilisation d'un mélange tel que défini dans l'une des revendications précédentes, dans laquelle le mélange contient une ou plusieurs paires de composés de formules (I) et (ent-I) ou consiste en une ou plusieurs de ces paires, chaque paire consistant en un composé de formule (I) et en un composé de formule (ent-I) tel que défini dans l'une des revendications précédentes,
dans laquelle
- la signification de X et Y dans le composé de formule (I) est respectivement identique à la signification de X et de Y dans le composé de formule (ent-I) et
dans laquelle
- (a) la signification de Z dans le composé de formule (I) est identique à la signification de Z dans le composé de formule (ent-I) ou
- (b) Z dans le composé de formule (I) et Z dans le composé de formule (ent-I) contiennent un ou plusieurs centres chiraux et se distinguent exclusivement par la configuration absolue sur un, plusieurs ou tous ces centres chiraux,
dans laquelle de préférence, chaque paire est une paire d'énantiomères ou d'épimères.

4. Utilisation d'un mélange en tant que mélange d'agents de sapidité qui consiste en les composants suivants ou contient ceux-ci :
(a) un composé choisi dans le groupe constitué des composés de formules (I) et (ent-I) tel que défini dans l'une des revendications précédentes ou un mélange constitué de deux composés ou plus ou contenant un ou plusieurs composés choisis dans le groupe comprenant des composés de formules (I) et (ent-I) tels que définis dans l'une des revendications précédentes ;
et
(b) un composé choisi dans le groupe comprenant des composés de formules (II), (ent-II), (III), (ent-III), (IV), (ent-IV) ou un mélange constitué de deux composés ou plus ou contenant un ou plusieurs composés choisis dans le groupe comprenant des composés de formules (II), (ent-II), (III), (ent-III), (IV), (ent-IV) ; 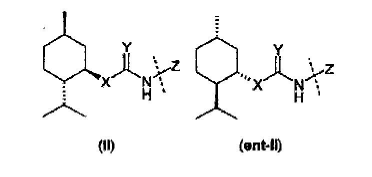 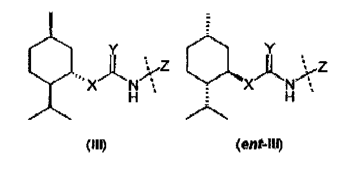 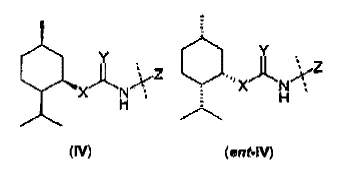 où, dans les formules (II), (ent-II), (III), (ent-III), (IV), (ent-IV), X, Y et Z ont respectivement, indépendamment les uns des autres, l'une des significations indiquées dans l'une des revendications précédentes pour les formules (I) et (ent-I)
et
où, dans le mélange d'agents de sapidité, le rapport en poids de (a) la totalité des composés de formules (I) et (ent-I) à (b) la totalité des composés de formules (II), (ent-II), (III), (ent-III), (IV) et (ent-IV) est d'au moins 60:40, de préférence d'au moins 90:10, de manière particulièrement préférée, d'au moins 95:5.

5. Utilisation d'un composé selon la revendication 1 ou 2, ou d'un mélange tel que défini dans l'une des revendications précédentes, pour produire, conférer, modifier et/ou renforcer un goût d'umami et/ou un caractère salé.

6. Composé individuel ou mélange consistant en deux composés ou plus ou contenant un ou plusieurs composés choisis dans le groupe comprenant des composés de formule (I) et (ent-I) 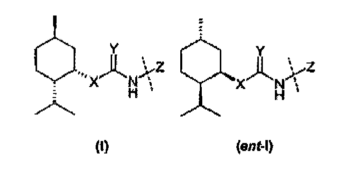 dans lesquelles la ligne en pointillé marque respectivement la liaison qui relie le radical organique Z à l'atome d'azote voisin dans les formules (I) ou (ent-I),
et où, dans les formules (I) et (ent-I), indépendamment les uns des autres :
X = NH ou O;
Y = O, S ou NR1, où R1 est un atome d'hydrogène, un groupe méthyle, éthyle, propyle ou isopropyle ;
et
Z = un radical organique consistant en atome de carbone ou d'hydrogène et éventuellement, en atomes d'oxygène, où
- le total du nombre d'atomes de carbone et d'oxygène est au maximum de 15 et le nombre d'atomes d'oxygène est au maximum de 4, et
- l'atome, avec lequel le radical organique Z est lié à l'atome d'azote voisin dans la formule (I) ou (ent-I), est le carbone,
à condition que
- le radical organique Z ne contienne pas de liaison C-C double ou triple, contienne au plus un cycle aromatique et ne soit pas un groupe -C(=Y²)-R² dans lequel Y² a l'une quelconque des significations indiquées ci-dessus pour Y et R2 est un radical organique quelconque,
Z ne soit pas -C(R5) (R6)-C(=O)OR4, où R4 est H, un groupe méthyle, éthyle, propyle, iso-propyle, butyle, iso-butyle ou tert-butyle et R5 et R6 désignent indépendamment l'un de l'autre, H ou un radical organique,
Z ne soit pas 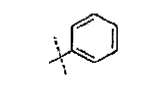
où la ligne en pointillé marque la liaison qui relie le radical organique Z à l'atome d'azote voisin dans les formules (I) ou (ent-I),
si X = NH et Y = 0, Z n'est pas un groupe néomenthyle ou 2-méthylcyclohexyle, et
si X = NH et Y = S, Z n'est pas un groupe néomenthyle, et
si X = O et Y =O, Z n'est pas un groupe éthyle, hydroxyéthyle, 1-phényléthyle, 2-phényléthyle, 2-hydroxy-1-méthyl-2-phényléthyle, isopropyle, 2-hydroxy-1-méthyléthyle, cyclohexyle, 4-hydroxyphényle, 2-hydroxy-5-méthylphényle ou 2,4,7-trioxa-3,3,8-triméthylbicyclo[3.3.0]octan-6-ylméthyle
et
où les composés de formule (I) ne comprennent pas le composé suivant : 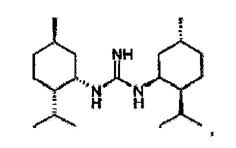
et où sont en outre exclus :
les composés individuels de formules (I) et (ent-I) dans lesquels X = O, Y = O et Z = (-)-mentyle,
un mélange consistant en 50,2 % du composé de formule (I) dans laquelle X = O, Y = O et Z = (-menthyle et 49,8 % du composé de formule (ent-I) dans laquelle X=O, Y = O et Z = (-)-menthyle et
un mélange consistant en 30,7 % du composé de formule (I) dans laquelle X = O, Y = O et Z = (-)-menthyle et 69,3 % d'acide N-(-)-menthylcarbamique (-)-menthylester

7. Composé individuel ou mélange tel que défini dans la revendication 6, dans lequel (i) pour le composé de formules (I) ou (ent-I) ou (II), pour un ou plusieurs ou tous les composés de formules (I) et (ent-I) dans le mélange, soit
A :
est le radical organique Z choisi dans le groupe comprenant : 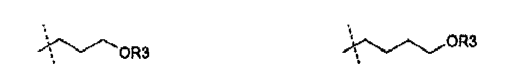 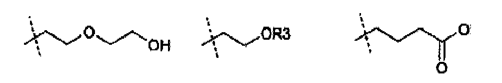 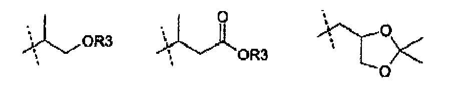 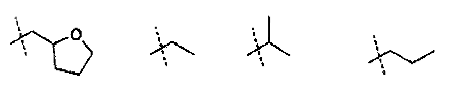 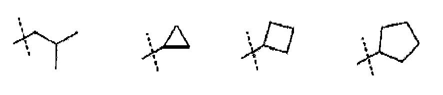 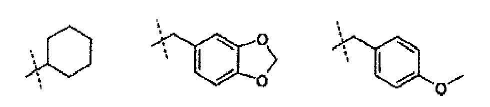 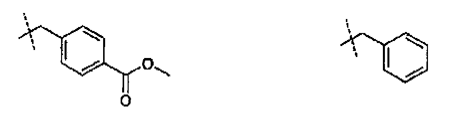
dans lequel des radicaux R3 éventuellement présents sont indépendamment les uns des autres un atome d'hydrogène, un groupe méthyle, éthyle, n-propyle, iso-propyle, n-butyle ou iso-butyle et la ligne en pointillé marque la liaison qui relie le radical organique Z avec l'atome d'azote voisin dans la formule (I) ou (ent-I)
soit
B
Y = O ; et
Z = un radical organique dans lequel le total du nombre d'atomes de carbone et d'oxygène est au maximum de 11 et le nombre d'atomes d'oxygène est au maximum de 2, où Z est un radical choisi dans le groupe comprenant éventuellement un groupe alkyle mono- ou multisubstitué, cycloalkyle en C₃, cycloalkyle en C₇ et phénylalkyle, où un ou plusieurs substituants éventuellement présents dans le radical sont choisis de préférence dans le groupe comprenant OH, un groupe alcoxy en C₁ à C₄, alcoxycarbonyle en C₁ à C₄ et éther cyclique, des groupes cycliques dans le radical organique Z étant éventuellement substitués par des groupes alkyle en C₁ à C₄.

8. Composé individuel ou mélange selon l'une des revendications 6 ou 7, dans lequel (i) le composé de formule (I) ou (ent-I) ou (II) représente un, deux, plus de deux ou tous les composés de formules (I) et (ent-I) dans le mélange, choisi dans le groupe comprenant :
(1) la 1-((1S,2S,5R)-2-isopropyl-5-méthylcyclohexyl)-3-(3-méthoxy-propyl)-urée,
(2) la 1-((1R,2R,5S)-2-isopropyl-5-méthylcyclohexyl)-3-(3-méthoxy-propyl)-urée
(3) la 1-(3-hydroxy-propyl)-3-((1S,2S,5R)-2-isopropyl-5-méthyl-cyclohexyl)urée
(4) la 1-(3-hydroxy-propyl)-3-((1R,2R,5S)-2-isopropyl-5-méthyl-cyclohexyl)urée
(5) la 1-((1S,2S,5R)-2-isopropyl-5-méthylcyclohexyl)-3-(2-méthomy-1-méthyl-éthyl)-urée
(6) la 1-((1R,2R,5S)-2-isopropyl-5-méthylcyclohexyl)-3-(2-méthoxy-1-méthyl-éthyl)-urée
(7) l'acide 3-[3-((1S,2S,5R)-2-isopropyl-5-méthylcyclohexyl)-uréido]-butyrique éthylester
(8) l'acide 3-[3-((1R,2R,5S)-2-isopropyl-5-méthylcyclohexyl)-uréido]-butyrique éthylester
(9) l'acide (3-méthoxypropyl)-carbamique (1S,2S,5R)-2-isopropyl-5-méthyl-cyclohexyl-ester
(10) l'acide (3-méthoxypropyl)-carbamique (1R,2R,5S)-2-isopropyl-5-méthyl-cyclohexyl-ester
(11) l'acide 4-((1S,2S,5R)-2-isopropyl-5-méthylcyclohexyloxycarbonylamino)-butyrique éthylester et
(12) l'acide 4-((1R,2R,5S)-2-isopropyl-5-méthylcyclohexyloxycarbonylamino)-butyrique éthylester.

9. Mélange selon l'une des revendications 6 à 8, le mélange contenant une ou plusieurs paires de composés de formules (I) et (ent-I) ou consistant en une ou plusieurs de ces paires, chaque paire consistant en un composé d'un composé de formule (I) et en un composé de formule (ent-I) tel que défini dans l'une des revendications précédentes,
dans lequel
- la signification de X et Y dans le composé de formule (I) est respectivement identique à la signification de X et Y dans le composé de formule (ent-I) et
dans lequel
- (a) la signification de Z dans le composé de formule (I) est identique à la signification de Z dans le composé de formule (ent-I)
- ou
- (b) Z dans le composé de formule (I) et Z dans le composé de formule (ent-I) contiennent un ou plusieurs centres chiraux et se distinguent exclusivement par la configuration absolue sur un ou tous ces centres chiraux,
dans lequel de préférence, chaque paire est une paire d'énantiomères ou d'épimères.

10. Mélange qui consiste en les composants suivants ou contient ceux-ci :
(a) un composé choisi dans le groupe constitué des composés de formules (I) et (ent-I) tel que défini dans l'une des revendications 6 à 8 ou un mélange constitué de deux composés ou plus ou contenant un ou plusieurs composés choisis dans le groupe comprenant des composés de formules (I) et (ent-I) tels que définis dans l'une des revendications 6 à 8 ou un mélange consistant en, ou contenant un mélange tel que défini dans la revendication 9 ;
et
(b) un composé choisi dans le groupe comprenant des composés de formules (II), (ent-II), (III), (ent-III), (IV), (ent-IV) ou un mélange constitué de deux composés ou plus ou contenant un ou plusieurs composés choisis dans le groupe comprenant des composés de formules (II), (ent-II), (III), (ent-III), (IV), (ent-IV) ; 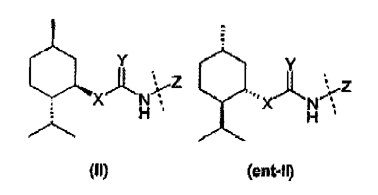 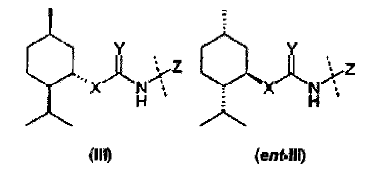 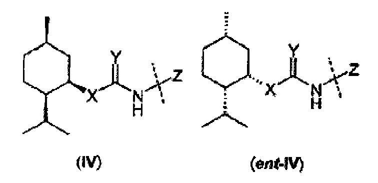 où dans les formules (II), (ent-II), (III), (ent-III), (IV), (ent-IV), X, Y et Z ont respectivement, indépendamment les uns des autres, l'une des significations indiquées dans l'une des revendications précédentes pour les formules (I) et (ent-I)
et
où, dans le mélange, le rapport en poids de (a) la totalité des composés de formules (I) et (ent-I) à (b) la totalité des composés de formules (II), (ent-II), (III), (ent-III), (IV) et (ent-IV) est d'au moins 60:40, de préférence d'au moins 90:10, de manière particulièrement préférée, d'au moins 95:5.

11. Composition, en particulier composition appropriée pour la consommation, comprenant ou consistant en
une quantité efficace d'un point de vue gustatif d'un composé ou d'un mélange tel que défini dans l'une des revendications précédentes
et un ou plusieurs autres composants appropriés pour la consommation qui comprend comme composants appropriés pour la consommation, des véhicules solides et éventuellement des compositions aromatiques.

12. Utilisation de composés selon l'une des revendications 1, 2 ou 6 à 8 ou mélanges selon l'une des revendications 1 à 10 ou compositions, en particulier compositions appropriées pour la consommation, comprenant ou consistant en une quantité efficace d'un point de vue gustatif d'un composé ou d'un mélange selon l'une des revendications 1 à 9 et un ou plusieurs autres composants appropriés pour la consommation,
(i) préparations prêtes à l'emploi ou prêtes à la consommation ou
(ii) produit semi-finis
servant dans l'alimentation ou la consommation
en particulier, préparations à teneur réduite en glutamate de sodium ou sans glutamate de sodium servant dans l'alimentation ou la consommation.

13. Préparation prête à l'emploi ou à la consommation servant dans l'alimentation ou la consommation selon l'une des revendications 1, 2 ou 6 à 8 ou mélanges selon l'une des revendications 1 à 10 ou compositions selon la revendication 11, en une quantité dans une plage de 0,01 ppm à 100 ppm.

14. Composés comprenant des produits semi-finis selon l'une des revendications 1, 2 ou 6 à 8 ou mélanges selon les revendications 1 à 9 ou compositions selon la revendication 11 et du glutamate de sodium.

15. Procédé pour produire, conférer, modifier et/ou renforcer une sensation gustative, dans lequel on ajoute à une substance ou composition, un composé selon l'une des revendications 1, 2 ou 6 à 8 ou un mélange selon l'une des revendications 1 à 10 ou une composition selon la revendication 11 ou une préparation selon la revendication 13 ou un produit semi-fini selon la revendication 14 en une quantité efficace d'un point de vue gustatif.
